# EUROPEAN PATENT SPECIFICATION

(11) **EP 1 440 079 B1**
(45) Date of publication and mention of the grant of the patent: **13.12.2006**
(21) Application number: 02769804.2
(22) Date of filing: 29.10.2002
(51) Int. Cl.: C07H 21/00, A61K 48/00, C12N 15/00

(54) **ACYCLIC LINKER-CONTAINING OLIGONUCLEOTIDES AND USES THEREOF**
AZYKLISCHE LINKER ENTHALTENDE OLIGONUKLEOTIDE UND DEREN VERWENDUNGEN
OLIGONUCLEOTIDES CONTENANT UN LIEUR ACYCLIQUE ET UTILISATIONS ASSOCIEES

(30) Priority: 29.10.2001 US 330719 P
(43) Date of publication of application: 28.07.2004
(73) Proprietor: McGill University, Montreal, Québec H3A 2T5 (CA)
(72) Inventor: DAMHA, Masad, J., St. Hubert, Quebec J3Y 8N9 (CA); VIAZOVKINA, Ekaterina, Montreal, Quebec H2L 3M8 (CA); MANGOS, Maria, M., Montreal, Quebec H3H 2P1 (CA); PARNIAK, Michael, A., Pittsburgh, PA 15217 (US); MIN, Kyung-Lyum, Montreal, Quebec H3V 1B4 (CA)
(74) Representative: Nargolwalla, Cyra
(86) International application number: PCT/CA2002/001628
(87) International publication number: WO 2003/037909

(56) References cited:
- EP-A- 0 978 561
- WO-A-97/26270
- US-A- 5 527 899
- US-A1- 2001 007 902
- BERGMANN F ET AL: "Solid phase synthesis of directly linked PNA-DNA-hybrids" TETRAHEDRON LETTERS, ELSEVIER SCIENCE PUBLISHERS, AMSTERDAM, NL, vol. 36, no. 38, 18 September 1995 (1995-09-18), pages 6823-6826, XP004027395 ISSN: 0040-4039
- DAMHA M J ET AL: "Hybrids of RNA and arabinonucleic acids (ANA and 2'F-ANA) are substrates of ribonuclease H" JOURNAL OF THE AMERICAN CHEMICAL SOCIETY, AMERICAN CHEMICAL SOCIETY, WASHINGTON, DC, US, vol. 120, no. 49, 16 December 1998 (1998-12-16), pages 12976-12977, XP002119329 ISSN: 0002-7863
- P. NIELSEN, F. KIRPEKAR, J. WENGEL: "Incorporation of (R)- and (S)-3',4'-seco-thymidine into oligonucleotides: hybridization properties and enzymatic stability" NUCLEIC ACIDS RESEARCH, vol. 22, no. 5, 1994, pages 703-710, XP001097566 Oxford University press
- AUGUSTYNS K ET AL: "INFLUENCE OF THE INCORPORATION OF (S)-9-(3,4-DIHYDROXY-BUTYL)ADENINE ON THE ENZYMATIC STABILITY AND BASE-PAIRING PROPERTIES OF OLIGODEOXYNUCLEOTIDES" NUCLEIC ACIDS RESEARCH, OXFORD UNIVERSITY PRESS, SURREY, GB, vol. 19, no. 10, 25 May 1991 (1991-05-25), pages 2587-2593, XP000579707 ISSN: 0305-1048
- VOROBJEV, P. E. ET AL: "Nuclease resistance and RNase H sensitivity of oligonucleotides bridged by oligomethylenediol and oligoethylene glycol linkers" ANTISENSE & NUCLEIC ACID DRUG DEVELOPMENT (2001), 11(2), 77-85 , 2001, XP002226341

## Description

### FIELD OF THE INVENTION

The invention relates to modified oligonucleotides and uses thereof, and particularly relates to modified oligonucleotides having one or more acyclic residues at internal positions, and uses thereof.

### BACKGROUND OF THE INVENTION

Oligonucleotides are utilized for a variety of biotechnological applications, including primers, probes, linkers, segments to confer a site or region of interest (e.g. sites for cleavage by nucleases; coding segments), mutagenesis, or to target a particular target region or molecule to fulfill a particular purpose or function. Their ability to confer specificity by virtue of their sequence composition has resulted in their use in a number of applications in biotechnology, in particular cases with various adaptations and modifications to render them more amenable to certain applications. Such modifications may entail the attachment of various groups, or modifications to the individual nucleoside groups or portions (i.e. the sugar and/or the base moieties) thereof, or to the backbone of the oligonucleotide molecule. Given, for example, their ability to be designed to target a protein-encoding molecule, such as RNA, a particular use of oligonucleotides is in antisense technology, to modulate the level, or features of a protein, and in turn modulate the function ascribed to that protein.

### Antisense Oligonucleotides (AON)

Antisense oligonucleotides (AONs) have attracted considerable interest in the biotechnology sector, and have exceptional potential for use in therapeutic strategies against a range of human diseases, including cancer and infectious diseases (Uhlmann, E. & Peyman, A. Chem. *Rev.* 1990, 90, 543). Criteria required of AON for potential clinical use include stability against serum and cellular nucleases, cell-membrane permeability, and stable and specific binding of the AON to its cellular target (usually messenger RNA [mRNA]). The formation of a duplex between the AON and its complementary sequence on the target RNA prevents the translation of such RNA, in part by "translation arrest" (via duplex formation between the AON and the target RNA, thus inhibiting/preventing complete translation by physically or sterically blocking the translational machinery) but more importantly by eliciting degradation of the targeted RNA through the action of ribonuclease H (RNase H), a ubiquitous and endogenous cellular enzyme that specifically degrades the RNA strand in the AON/RNA duplex (Walder, R.T.; Walder, J.A. *Proc. Natl. Acad. Sci. USA* 1988, *85*, 5011).

Current AON technologies are deficient in one or more of the criteria required for clinical utility. Since the natural substrate of RNase H is a DNA/RNA heteroduplex, DNA has been utilized for antisense technology. However, as serum and intracellular nucleases rapidly degrade AONs with phosphodiester (PDE) linkages, AON consisting of PDE-DNA have had limited utility in such systems. DNA strands with phosphorothioate linkages (PS-DNA) have been used successfully in a large number of experiments designed to downregulate gene expression, and they have been and/or are in use in several clinical therapeutic trials (Akhtar, S. & Agrawal, S. *Trends Pharmacol. Sci.* 1997, *18,* 12). PS-DNA induces RNase H degradation of the targeted RNA, and is resistant to degradation by serum and cellular nucleases, however, it forms weaker duplexes with the target RNA compared to PDE-DNA. Furthermore, PS-DNA shows extensive 'non-specific' binding to serum and cellular proteins (Brach, A.D. *TIBS,* 1998, *23*, 45). This can lead -to unfavorable toxicity, especially given the high concentrations of PS-DNA needed to exert an *in vivo* effect. Identification of new AON structures that can bind tightly and specifically to target RNA, and elicit efficient RNase H degradation of that RNA, is a high priority in antisense development.

The structure of the AON determines whether RNase H can cleave the RNA strand of AON/RNA duplexes. As such, various strategies have been utilized to improve binding to the RNA target, to improve duplex formation and stability. For example, AONs that exclusively contain either 2'-O-methylribose (or any substitution at the *ribose* 2'-position) or N3'-P5' phosphoramidate linkages, and DNA molecules containing uncharged internucleotide linkages, composed for example of methylphosphonate or amide linkages, have been described, however, such AON do not elicit RNase H activity (for a review, see Manoharan, M. *Biophys. Biochim.* Acta, 1999, *1489,* 117). Other analogues such as phosphorodiamidate morpholino nucleic acids also lack the ability to elicit RNase H activity (Summerton, J., and Weller, D., *Antisense Nucleic Acid Drug Dev.,* 1997, 7, 187). Peptide nucleic acids (PNA) display remarkable hybridization properties, binding to single stranded RNA, single stranded DNA and duplex DNA with high affinity (Egholm, M. *et al., Nature,* 1993, *365,* 566; Knudsen, H. et *al., Nucl. Acids Res.,* 1997, *25*, 2167). However, PNA:RNA hybrids are not substrates for RNase H. In fact, of the several dozens of modified AON prepared during the period 1978-1998, only PS-DNA, phosphorodithioate DNA (PS₂-DNA), and boranophosphate DNA were reported to elicit RNase H degradation of target RNA (Sanghvi, Y.S. & Cook, P.D. "Carbohydrate Modifications in Antisense Research" *ACS Symposium Series,* vol. 580. American Chemical Society, Washington DC, 1994). As in the case for PS-DNA, the analogues PS₂-DNA and boranophosphate DNA exhibit weaker binding towards target RNA relative to the unmodified PDE-DNA. Therefore, while the above strategies are capable of conferring increased binding to the target, such AON are unable to induce RNase H activity.

Attempts to overcome this limitation include the development of arabinonucleic acid (ANA) and 2'-deoxy-2'-fluoroarabinonucleic acids (FANA). These compounds are the first sugar-modified oligonucleotides ever reported to elicit RNase H activity [Damha, M.J. et *al.,* "Antisense oligonucleotide constructs based on beta-arabinose and its analogues". PCT International Publication No. WO 99/67378; Damha, M.J. *et al. J. Am. Chem. Soc.* 1998, *120*, 12976; Noronha, A.M. *et al. Biochemistry* 2000, *39*, 7050). These oligonucleotides retain a β-D-furanose ring and mimic the conformation of DNA strands (Trempe, J.-F. *et al., J. Am. Chem. Soc.* 2001, *124,* 4896). FANA forms much more stable duplexes with target RNA than does PS-DNA; indeed, the stability of the FANA/RNA duplex generally exceeds that of RNA/RNA duplexes (Damha, M.J. *et al. J. Am. Chem. Soc.* 1998, *120,* 12976; Wilds, C.J. & Damha, M.J. *Nucl. Acids Res.* 2000, *28*, 3625).

Other notable developments in the antisense area include mixed-backbone oligonucleotides (MBO) composed of PS-DNA oligodeoxynucleotide segments flanked on both sides by sugar-modified oligonucleotide segments such as PS-[2'-OMe RNA-(DNA )-2'OMe RNA] (for example, see Crooke, S. T. *et al., Biochem. J.* 1995, *312 (Pt 2) ,* 599). These MBOs are also known as "gapmers". The flanking 2'-O-methyl RNA "wings" increase the binding affinity of the MBO for target RNA, while the PS-DNA segment in the middle of the AON directs RNase H degradation of the target RNA (Zhao, G. *et al., Biochem. Pharmacol.* 1996, *51*, 173; Crooke, S.T. et *al. J. Pharmcol. Exp. Ther.* 1996, *277*, 923). MBOs have increased stability in vivo (*i.e*., resistance to nuclease degradation), and show improved biological activity both *in vitro* and in vivo compared to the corresponding all PS-DNA AON. Examples of this approach incorporating 2'-OMe and other alkoxy substituents in the flanking regions of an oligonucleotide have been demonstrated by Monia *et al.* by enhanced antitumor activity in vivo (Monia, P.B. *et al., Nature Med.* 1996, *2*, 668). Several pre-clinical trials with these analogues are ongoing (Akhtar, S.; Agrawal, S. *TiPS* 1997, *18,* 12).

MBO antisense comprised of FANA flanking internal DNA segments show exceptionally potent target-specific inhibition of gene expression (EC₅₀ < 5 nM) when tested in cell culture assays, and unlike 2'-OMe RNA/DNA MBO, their biological activity is significantly less dependent on the length of the internal DNA gap (Damha *et al.;* International PCT Publication WO 02/20773 published March 14, 2002).

### Elicitation of cellular RNase H degradation of target RNA by AONs

RNase H selectively degrades the RNA strand of a DNA/RNA heteroduplex (Hausen, P.; Stein, H. *Eur. J. Biochem.* 1970, *14*, 279). One of the most important mechanisms for antisense oligonucleotide-directed inhibition of gene expression is the ability of these antisense oligonucleotides to form a structure, when duplexed with the target RNA, that can be recognized by cellular RNase H. This enables the RNase H-mediated degradation of the RNA target, within the region of the antisense oligonucleotide-RNA base-paired duplex (Walder, R.T.; Walder, J.A. *Proc. Natl. Acad. Sci . USA* 1988, *85*, 5011).

RNase H1 from the bacterium *Escherichia coli* is the most readily available and the best characterized enzyme. Studies with eukaryotic cell extracts containing RNase H suggest that both prokaryotic and eukaryotic enzymes exhibit similar RNA-cleavage properties (Monia *et al. J. Biol. Chem.* 1993, *268,* 14514; Crooke *et al. Biochem J.* 1995, *312,* 599; Lima, W.F.; Crooke, S.T. *Biochemistry* 1997, 36, 390). *E. coli* RNase H1 is thought to bind to the minor groove of the DNA/RNA double helix and to cleave the RNA by both endonuclease and processive 3'-to-5' exonuclease activities (Nakamura, H. *et al. Proc. Natl. Acad. Sci. USA* 1991, *88*, 11535; Fedoroff, O.Y. *et al., J. Mol. Biol.* 1993, *233*, 509). The efficiency of RNase H degradation displays minimal sequence dependence and, as mentioned above, is quite sensitive to chemical changes in the antisense oligonucleotide.

Because 2'-OMe RNA cannot elicit RNase H activity, the DNA gap size of the PS-[2'-OMe RNA-DNA-2'OMe RNA] chimeric oligonucleotides must be carefully defined. Thus, while *E. coli* RNase H can recognize and use 2'-OMe RNA MBO with DNA gaps as small as 4 DNA nucleotides (Shen, L.X. *et al. Bioorg. Med. Chem.* 1998, *6*, 1695), the eukaryotic RNase H (such as human RNase HII) requires larger DNA gaps (7 DNA nucleotides or more) for optimal degradative activity (Monia, B.P. *et al. J. Biol. Chem.* 1993, *268,* 14514). In general, with PS-[2'-OMe RNA-DNA-2'OMe RNA] chimeric oligonucleotides, eukaryotic RNase H-mediated target RNA cleavage efficiency decreases with decreasing DNA gap length, and becomes almost negligible with DNA gap sizes of less than 6 DNA nucleotides. Thus, the antisense activity of PS-[2'-OMe RNA-DNA-2'OMe RNA] chimera oligonucleotides is highly dependent on DNA gap size (Monia, B.P. *et al. J. Biol. Chem.* 1993, *268*, 14514; Agrawal, S. and Kandimalla, E.R. *Mol. Med. Today* 2000, *6*, 72). This is not the case for PS-[FANA-DNA-FANA] chimeras which display significant biological activity with DNA gaps as small as 1 deoxynucleotide residue (Damha *et al.;* International PCT Publication WO 02/20773 published March 14, 2002).

Recently, oligonucleotides containing completely altered backbones have been synthesized. Notable examples are the peptide nucleic acids ("PNA") with an acyclic backbone (Nielsen, P.E. *in "Perspectives in Drug Discovery and Design",* vol. 4, pp. 76, Trainor, G.L. (ed.), ESCOM, Leiden, 1996). These compounds have exceptional hybridization properties, and stability towards nucleases and proteases. However, efforts to use PNA oligomers as antisense constructs have been hampered by poor cellular uptake and inability to activate RNase H. Very recently, PNA-[DNA]-PNA chimeras have been designed to maintain RNase H mediated cleavage via the DNA portion of the chimera (Bergman, F. *et al., Tetrahedron Lett*. 1995, *36*, 6823; Van der Laan, A.C. *et al. Trav. Chim. Pays-Bas* 1995, *114,* 295). The PNA segments located at the 5'- and 3'-termini serve to facilitate binding to the target nucleic acid (RNA) and enhance resistance towards degradation by exonuclease enzymes. However, based on the presence of DNA, such a construct may be more prone to degradation in biological systems, as noted above.

There is therefore a need for an improved oligonucleotide for such antisense approaches, to try to address the limitations noted above (e.g. binding, induction of RNase H activity, resistance to degradation).

### SUMMARY OF THB INVENTION

The invention is defined by the claims.

There is provided an oligonucleotide having the structure:

[R¹-X]ₐ-R² **Ia**

wherein a is greater than or equal to 1; wherein each of R¹ and R² are independently at least one nucleotide; and wherein x is an acyclic linker.
PDE- or PS-RNA analogues are selected from the group consisting of 2'-modified RNA wherein the 2'-substituent is selected from the group consisting of alkyl, alkoxy, alkylalkoxy, F and combinations thereof.

The acyclic linker is selected from the group consisting of an acyclic nucleoside and a non-nucleotidic linker. In embodiments, the acyclic nucleoside is selected from the group consisting of purine and pyrimidine seconucleosides. In embodiments, the purine seconucleoside is selected from the group consisting of secoadenosine and secoguanosine. In embodiments, the pyrimidine seconucleoside is selected from the group consisting of secothymidine, secocytidine and secouridine.

In an embodiment, the non-nucleotidic linker comprises a linker selected from the group consisting of an amino acid and an amino acid derivative. In embodiments, the amino acid derivative is selected from the group consisting of (a) an N-(2-aminoethyl)glycine unit in which an heterocyclic base is attached via a methylene carbonyl linker (PNA monomer); and (b) an O-PNA unit.

According to a further aspect of the invention, there is provided an AON chimera of general structure Ib: 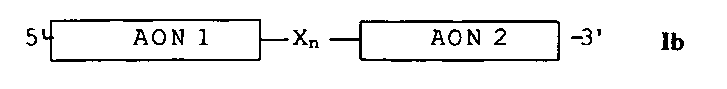 wherein n is greater than or equal to 1. With reference to structure Ib above, "AON1" is an oligonucleotide chain, which in embodiments is selected from the group consisting of ANA (e.g. FANA), DNA, PS-DNA, 5'-RNA-DNA-3' chimeras, as well as other RNase H-competent oligonucleotides, for example arabinonucleic acids (2'-OH substituted ANA) (Damha, M.J. *et al. J*. *Am. Chem. Soc*. 1998, *120*, 12976), cyclohexene nucleic acids (Wang J. *et al. J. Am.* Chem. *Soc.* 2000, *122*, 8595), boranophosphate linked DNA (Rait, V.K. *et al. Antisense Nucleic Acid Drug Dev.* 1999, *9*, 53), and alpha-L-locked nucleic acids (Sørensen, M.D. *et al. J. Am. Chem. Soc.* 2002, *124,* 2164) or combinations thereof; and "AON2" is an oligonucleotide chain, which in embodiments is selected from the group consisting of FANA, DNA, PS-DNA, 5'-DNA-RNA-3' chimeras, as well as other RNase H-competent oligonucleotides such as those described above, or combinations thereof. The internucleotide linkages of the AON1 and AON2 includes but is not necessarily limited to phosphodiester, phosphotriester, phosphorothioate, methylphosphonate, phosphoramidate (5'N-3'P and 5'P-3'N) groups. The substituent directly attached to the C2'-atom of the arabinose sugar in ANA-X-ANA chimera constructs includes but is not limited to fluorine, hydroxyl, amino, azido, alkyl (e.g. 2'-methyl, ethyl, propyl, butyl, etc.), and alkoxy groups (*e.g.,* 2'-OMe, 2'-OEt, 2'-OPr, 2'-OBu, 2'-OCH₂CH₂OMe, etc.).

Examples of the general structures Ia and Ib include PDE- and PS-[FANA]-X-[FANA], PDE- and PS-[FANA-DNA-X-DNA-FANA], PS-[DNA-X-DNA], PDE- and PS-[RNA-DNA-X-DNA-RNA], PDE- and PS-[(2'O-alkyl-RNA)-DNA-X-DNA-(2'O-alkyl-RNA)], and PDE- and PS-[(2'-OCH₂CH₂OMe-RNA)-DNA-X-DNA-(2'-OCH₂CH₂OMe-RNA)].

In an embodiment, an oligonucleotide of the invention has the structure: 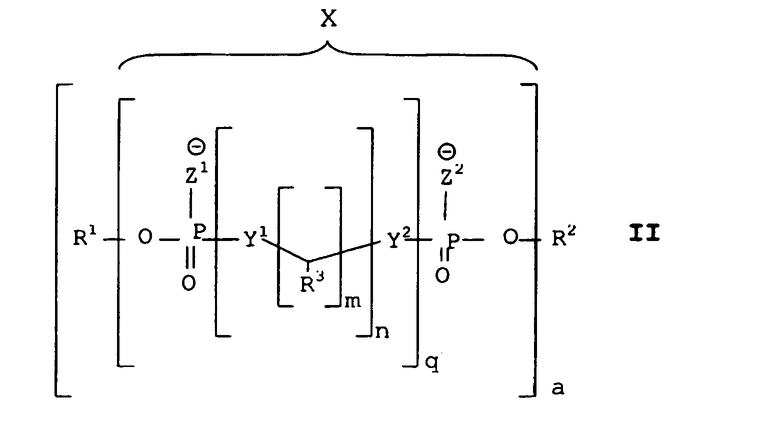 wherein each of m, n, q and a are independently integers greater than or equal to 1; wherein each of R¹ and R² are independently at least one nucleotide, wherein each of Z¹ and Z² are independently selected from the group consisting of an oxygen atom, a sulfur atom, an amino group and an alkylamino group;
wherein each of Y¹ and Y² are independently selected from the group consisting of oxygen, sulfur and NH; and wherein R³ is selected from the group consisting of H, alkyl, hydroxyalkyl, alkoxy, a purine, a pyrimidine and combinations thereof.

In embodiments, R³ is adenine or guanine, or derivatives thereof.

In embodiments, R³ is thymine, cytosine, 5-methylcytosine, uracil, or derivatives thereof.

In embodiments, each of R¹ and R² noted above are independently selected from the group consisting of ANA, PS-ANA, RNA-DNA and DNA-RNA chimeras, PS-[RNA-DNA] and PS- [DNA-RNA] chimeras, PS-[ANA-DNA] and PS-[DNA-ANA] chimeras, alpha-L-LNA, cyclohexene nucleic acids, RNA, PS-RNA, PDE- or PS-RNA analogues, locked nucleic acids (LNA), phosphorodiamidate morpholino nucleic acids, N3'-P5' phosphoramidate DNA, methylphosphonate DNA, and combinations thereof.

In embodiments, each of R¹ and R² noted above independently may comprise at least two nucleotides connected via an internucleotide linkage, wherein said internucleotide linkage is selected from the group consisting of phosphodiester, phosphotriester, phosphorothioate, methylphosphonate, phosphoramidate (5'N-3'P and 5'P-3'N) groups and combinations thereof.

In embodiments, each of R¹ and R² noted above independently comprise ANA.

In embodiments the above-noted ANA comprises a 2'-substituent selected from the group consisting of fluorine, hydroxyl, amino, azido, alkyl (*e.g.* methyl, ethyl, propyl and butyl) and alkoxy (*e.g*. methoxy, ethoxy, propoxy, and methoxyethoxy) groups.

In an embodiment, the 2'-substituent is fluorine and said ANA is FANA.

In embodiments, the alkyl group is selected from the group consisting of methyl, ethyl, propyl and butyl groups. In embodiments, the alkoxy group is selected from the group consisting of methoxy, ethoxy, propoxy, and methoxyethoxy groups.

In embodiments, an oligonucleotide of the invention is selected from the group consisting of: 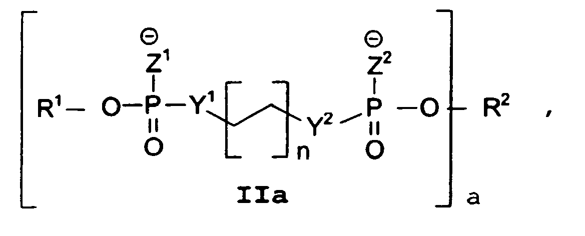 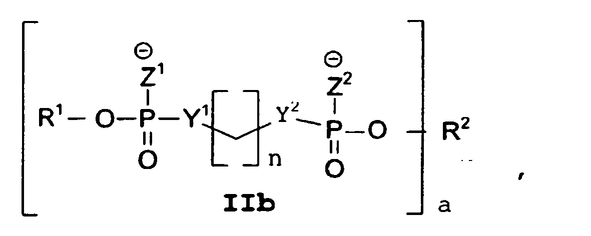 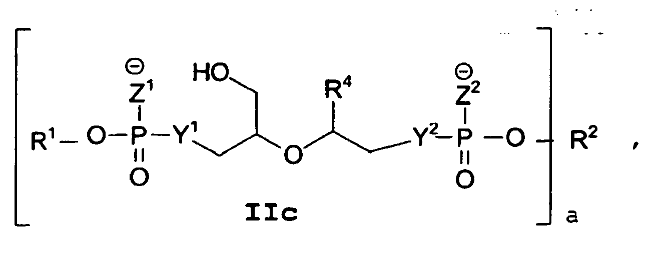 and 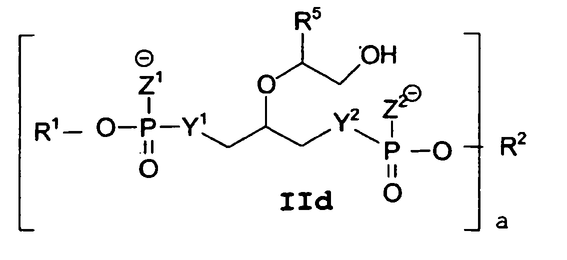 wherein R¹, R², n, a, Z¹, Z², Y¹ and Y² are as defined above and each of R⁴ and R⁵ are independently selected from the group consisting of a purine (*e.g.* adenine and guanine or derviatives thereof) and a pyrimidine (e.g. thymine, cytosine, uracil, or derivatives thereof).

In an embodiment, R¹ and R² are PDE-FANA; and a=1.

In an embodiment, R¹ and R² are PS-FANA; and a=1.

In an embodiment, R¹ is [FANA-DNA]; R² is [DNA-FANA]; and a=1.

In an embodiment, R¹ is [FANA-DNA]; R² is FANA; and a=1.

In an embodiment, R¹ is FANA; R² is [DNA-FANA]; and a=1.

In an embodiment, R¹ and R² are PS-DNA; and a=1.

In an embodiment, R¹ is PDE-[RNA-DNA], R² is PDE-[DNA-RNA] ; and a=1.

In an embodiment, R¹ is RNA; R² is [DNA-RNA]; and a=1.

In an embodiment, R¹ is S-[(2'O-alkyl)RNA-DNA]; R² is S-[DNA-(2'O-alkyl)RNA]; and a=1.

In an embodiment, R¹ is S-[(2'O-alkyl)RNA-DNA]; R² is S-[(2'O-alkyl)RNA]; and a=1.

In an embodiment, R¹ is S-[(2'O-alkyl)RNA]; R² is S-[DNA-(2'O-alkyl)RNA]; and a=1.

In an embodiment, R¹ is S-[(2'O-alkoxyalkyl)RNA-DNA]; R² is S-[DNA-(2'O-alkoxyalkyl)RNA]; and a=1.

In an embodiment, R¹ is S-[(2'O-alkoxyalkyl)RNA-DNA]; R² is S-[(2'O-alkoxyalkyl)RNA]; and a=1.

In an embodiment, R¹ is S-[(2'O-alkoxyalkyl)RNA]; R² is S-[DNA-(2'O-alkoxyalkyl)RNA]; and a=1.

In an embodiment, R¹ is PDE-[(2'O-alkyl-RNA)-DNA]; R² is PDE-[DNA-(2'O-alkyl RNA)]; and a=1.

In an embodiment, R¹ is PS-[(2'O-alkyl-RNA)-DNA]; R² is PS-[DNA-(2'O-alkyl RNA)]; and a=1.

In an embodiment, R¹ is PDE-[(2'O-alkoxyalkyl-RNA)-DNA]; R² is PDE-[DNA-(2'O-alkoxyalkyl RNA)]; and a=1.

In an embodiment, R¹ is PS-[(2'O-alkoxyalkyl-RNA)-DNA]; R² is PS-[DNA-(2'O-alkoxyalkyl RNA)]; and a=1.

In an embodiment, R¹ and R² are PDE-[FANA]; a=1; and the oligonucleotide has structure IIb in which Y¹, Y² are oxygen; Z¹ , Z² are both oxygen or sulfur, and n=4.

In an embodiment, R¹ is PS-[FANA]; R² is PDE-[FANA]; a=1; and the oligonucleotide has structure IIb in which Y¹, Y^{Z} are oxygen; Z¹, Z² are both oxygen or sulfur, and n=4.

In an embodiment, R¹ is FANA; R² is PS-FANA; a=1; and the oligonucleotide has structure IIb in which Y¹, Y², Z¹ and Z² are oxygen and n=4

In an embodiment, R¹ and R² are PS-[FANA]; a=1; and the oligonucleotide has structure IIb in which Y¹, Y² are oxygen; Z¹, Z² are both oxygen or sulfur, and n=4.

In an embodiment, R¹ and R² are PDE-[FANA]; a=1; and the oligonucleotide has structure IIc in which Y¹, Y² are oxygen; Z¹, Z² are both oxygen or sulfur.

In an embodiment, R¹ is PS-[FANA]; R² is POE-[FANA]; a=1; and the oligonucleotide has structure IIc in which Y¹, Y² are oxygen; Z¹, Z² are both oxygen or sulfur.

In an embodiment, R¹ is PDE-[FANA]; R² is PS-(FANA); a=1; and the oligonucleotide has structure IIb in which Y¹, Y² are oxygen; Z¹, Z² are both oxygen or sulfur, and n=4.

In an embodiment, R¹ and R² are PS-[FANA]; a=1: and the oligonucleotide has structure IIc in which Y¹, Y² are oxygen; Z¹ , Z² are both oxygen or sulfur.

In an embodiment, R¹ is PS-[DNA]; R² is PDE-[DNA]: a=1; and the oligonucleotide has structure IIc in which Y¹, Y² are oxygen; Z¹ , Z² are both oxygen or sulfur.

In an embodiment, R¹ is DNA; R² is PS-DNA; a=1; and the oligonucleotide has structure IIc.

In an embodiment, R¹ and R² are PS-[DNA]; a=1; and the oligonucleotide has structure IIc in which Y¹, Y² are oxygen; Z¹, Z² are both oxygen or sulfur.

In an embodiment, a=2 and each of R¹ and R² independently consist of at least 3 nucleotides, in a further embodiment, of 3-8 nucleotides.

In an embodiment, a=3 and each of R¹ and R² independently consist of at least 2 nucleotides, in a further embodiment, wherein each of R¹ and R² independently consist of 2-6 nucleotides.

In an embodiment, the oligonucleotide is antisense to a target RNA.

The invention further provides an in vitro method of preventing or decreasing translation, reverse transcription and/or replication of a target RNA in a system, said method comprising contacting said target RNA with an oligonucleotide as defined above.

The invention further provides an in vitro method of preventing or decreasing translation, reverse transcription and/or replication of a target RNA in a system, said method comprising:
a) contacting said target RNA with an oligonucleotide as defined above; and
b) allowing RNase cleavage of said target RNA.

The invention further provides an in vitro use of an oligonucleotide as defined above for preventing or decreasing translation, reverse transcription and/or replication of a target RNA in a system.

The invention further provides a commercial package comprising the above-noted oligonucleotide together with instructions for its use in preventing or decreasing translation, reverse transcription and/or replication of a target RNA in a system.

### BRIEF DESCRIPTION OF THE DRAWINGS

The invention will now be described in greater detail having regard to the appended drawings in which:
**FIGURE 1** illustrates RNase H mediated cleavage of RNA duplexed with homopolymeric PDE-FANA and FANA-X-FANA. Timed aliquots were taken at 0, 5, 10, and 20 min from each set of incubation. Experimental conditions are given in Example 4A.
**FIGURE 2** illustrates RNase H mediated cleavage of RNA duplexed with homopolymeric PDE-FANA and PDE-[FANA-X-FANA] as a function of time. Degradation of the 5'-labeled target RNA was quantified by densitometry of the gel shown in Figure 1.
**FIGURE 3** illustrates RNase H mediated cleavage of RNA duplexed with mixed-base PDE-FANA and PDE-[FANA-X-FANA]. Timed aliquots were taken at 0, 5, 10, and 20 min from each set of incubation. Experimental conditions are given in Example 4B.
**FIGURE 4** illustrates RNase H mediated cleavage of RNA duplexed with mixed base PDE-FANA and PDE-[FANA-X-FANA] as a function of time. Degradation of the 5'-labeled target RNA was quantified by densitometry of the gel shown in Figure 3.

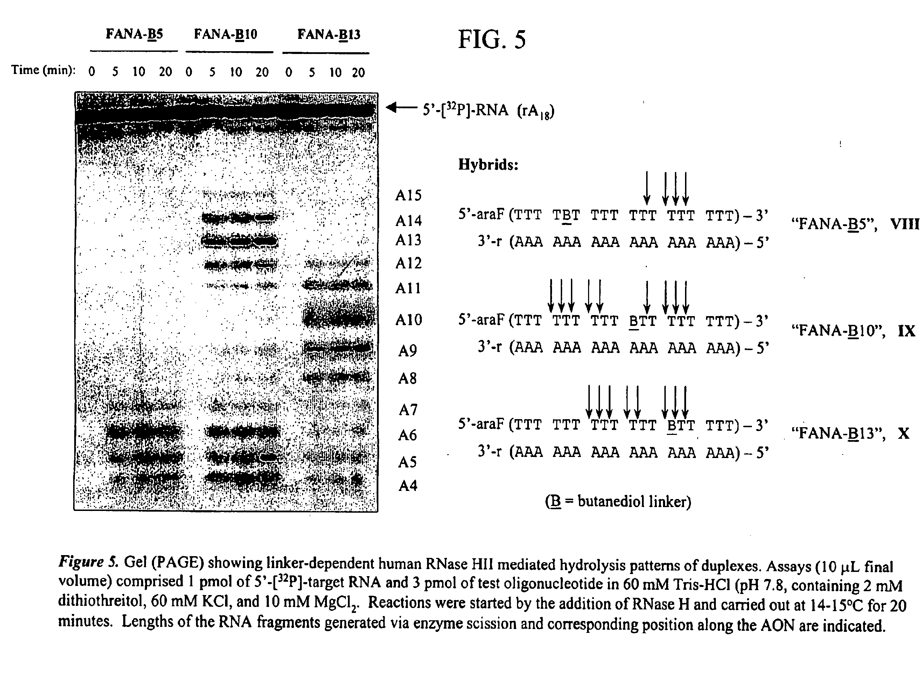

**FIGURE 5** illustrates RNase H mediated cleavage of RNA duplexed with homopolymeric PDE-FANA-X-FANA] -containing the butanediol linker X at positions 5, 10 and 13. Assays (10 µL final volume) comprised 1 pmol of 5'-[³²P]-target RNA and 3 pmol of test oligonucleotide in 60 mM Tris-HCl (pH 7.8, containing 2 mM dithiothreitol, 60 mM KCl, and 10 mM MgCl₂. Reactions were started by the addition of RNase H and carried out at 14-15°C for 20 minutes. Timed aliquots were taken at 0, 5, 10, and 20 min from each set of incubation. Lengths of the RNA fragments generated via enzyme scission and corresponding position along the AON are indicated.
**FIGURE 6** illustrates RNase H mediated cleavage of RNA duplexed with homopolymeric FANA-X-FANA (But-5, 10 and 13) as a function of time. Degradation of the 5'-labeled target RNA was quantified by densitometry of the gel shown in Figure 5.
**FIGURE 7** illustrates RNase H mediated cleavage of RNA duplexed with homopolymeric PDE-[FANA-X-FANA] and PDE-[FANA-X-X-FANA] containing internal secouridine linkers. Timed aliquots were taken at 0, 5, 10, and 20 min from each set of incubation. Experimental conditions are given in Example 6.
**FIGURE 8** illustrates RNase H mediated cleavage of RNA duplexed with homopolymeric PDE-[FANA-X-FANA] (SEC×1), and PDE-[FANA-X-X-FANA] (SEC×2), and PDE-FANA as a function of time. Degradation of the 5'-labeled target RNA was quantified by densitometry of the gel shown in Figure 7.
**FIGURE 9** illustrates RNase H mediated cleavage of RNA duplexed with homopolymeric PDE-DNA and PDE-[DNA-X-DNA] (X = butanediol linker). Timed aliquots were taken at 0, 5, 10, and 20 min from each set of incubation. Experimental conditions are given in Example 7.
**FIGURE 10** illustrates RNase H mediated cleavage of RNA duplexed with homopolymeric PDE-DNA and PDE-[DNA-X-DNA] (X = butanediol linker) as a function of time. Degradation of the 5'-labeled target RNA was quantified by densitometry of the gel shown in Figure 9.
**FIGURE 11** illustrates RNase H mediated cleavage of Ha-Ras RNA duplexed with mixed base PDE-FANA, PDE-[FANA-X-FANA], PDE-DNA, PDE-[DNA-X-DNA], and PDE-[mismatched DNA] containing the butanediol linker X at position 10. Assays were conducted as described in Example 8. Lengths of the RNA fragments generated via enzyme scission and corresponding position along the AON are indicated. Kinetic data (k) of RNA cleavage is provided in Table 1.
**FIGURE 12** illustrates RNase H mediated cleavage of Ha-Ras RNA duplexed with mixed base PS-FANA, PS-[FANA-X-FANA], PS-DNA, and PS-[DNA-X-DNA] containing the butanediol linker X at position 10. Assays were conducted as described in Example 9. Kinetic data (k) of RNA cleavage is provided in Table 1.

### DETAILED DESCRIPTION OF THE INVENTION

The invention relates to modified oligonucleotides that, in an embodiment, are capable of selectively preventing gene expression in a sequence-specific manner. In particular, the invention relates to the selective inhibition of protein biosynthesis via antisense strategy using short strands of for example modified nucleotides, such as modified DNA and modified arabinonucleic acids, containing one or more acyclic residues at internal positions. In a preferred embodiment, an oligonucleotide of the invention comprises at least one modified nucleoside or nucleotide (as compared to native DNA). Examples of acyclic residues include acyclic nucleosides [*e.g*., seconucleosides, PNA monomers (N-(2-aminoethyl)glycine unit in which a heterocyclic base is attached via a methylene carbonyl linker), O-PNA monomers [-NH-CH(CH₂-CH₂-Base)-CH₂-O-CH₂-CO-] and non-nucleotidic linkers (e.g., alkyldiol linker, amino acids, dipeptides and dipeptide derivatives). In embodiments the invention relates to the use of modified oligonucleotides constructed primarily from modified deoxyribonucleotide and modified arabinonucleotide residues containing one or more acyclic residues, to hybridize to complementary RNA such as cellular messenger RNA, viral RNA, etc. In a further embodiment, the invention relates to the use of modified oligonucleotides constructed from modified DNA and modified ANA residues, containing one or more acyclic residues, to hybridize to and induce cleavage of complementary RNA via RNase H activation.

In an embodiment, the invention relates to antisense oligonucleotide chimeras constructed from either modified deoxyribonucleotide or modified arabinonucleotide residues flanking an acyclonucleotide or a modified hydrocarbon chain, that form a duplex with its target RNA sequence. The resulting AON/RNA duplexes are excellent substrates for RNase H, an enzyme that recognizes this duplex and degrades the RNA target portion. RNase H-mediated cleavage of RNA targets is considered to be a major mechanism of action of antisense oligonucleotides.

The present invention relates to the unexpected and surprising discovery that antisense chimeras constructed from a modified nucleotide (e.g. 2'-deoxy-2'-fluoro-β-D-arabinonucleotides [FANA]) and an internal acyclic nucleotide residue (e.g. seconucleotide), or an internal modified hydrocarbon chain are superior at eliciting eukaryotic RNase H activity *in vitro* compared to uniformly modified FANA oligomers. Accordingly, antisense hybrid chimeras comprising a modified nucleotide such as 2'-deoxy-2'-fluoro-β-D-arabinonucleotides (FANA), containing such RNase H-inducing acyclic residues may be useful as therapeutic agents and/or tools for the study and control of specific gene expression in cells and organisms. This "acyclic linker strategy" may also be applied to other modified AONs in order to improve their antisense properties *in vivo.*

The results described herein are truly surprising based on the current wisdom in the art, because a consistent and prevailing goal in antisense technology has always been to introduce modifications that increase duplex stability. Such modifications in many cases result in a type of "pre-organization" of the antisense molecule, whereby the AON is designed to resemble the "bound" conformation even before duplex formation occurs, thus reducing the entropy associated with binding. As such, introduction of a flexible structural element such as an acyclic linker (which is free of the ring strain of a cyclic structure), since it would decrease duplex stability, is considered to be detrimental to RNase H induction. Indeed, the introduction of such acyclic elements results in a lower melting temperature as outlined in the results presented herein. Consistent with this principle, native DNA oligonucleotides bridged by oligomethylenediol or oligoethylene glycol linkers have been described as exhibiting decreased duplex stability and impaired RNase H activity (Vorobjev *et al., Antisense and Nucleic Acid Drug Dev. ,* 2001, *11,* 77).

Conversely, applicants' studies described herein demonstrate that the incorporation of flexible structural elements such as an acyclic linker in for example 2'F-ANA AON results in efficient RNase H-mediated target cleavage. It is shown herein that the enzyme's activity is readily modulated by the systematic placement of flexible units at key sites within for example 2'F-ANA strands of AON/RNA duplexes. Based on the improved induction of RNase H using AON comprising modified nucleotides described herein, it is envisioned that a certain amount of pre-organization (e.g. conferred by including one or more modified nucleotides in the oligonucleotide) in the antisense strand plays a role in maintaining high binding and/or specificity for complementary RNA. While both pre-organization & flexibility on their own are detrimental towards enzyme elicitation, applicants propose herein the surprising finding that their combination gives synergistic inhibition of target mRNA and address the various conformational characteristics that give rise to these enhancements. As such, applicants describe herein that even compounds devoid of DNA can elicit RNase H activity with comparable efficiency to the native (DNA) systems by virtue of an introduced acyclic linker. Further, the improved induction of RNase H conferred by such an acyclic linker is even more pronounced when targeting longer (i.e. more physiologically relevant) RNAs, as described herein. Therefore, it is envisioned that such an acyclic linker strategy may be incorporated into known antisense methodologies and structures to improve RNase H induction and in turn target inhibition.

"Flexible" or "flexibility" as used herein is a relative term referring to the degrees of freedom with respect to allowable motion or conformations available at a particular region of interest in a molecule, thus contributing to the "flexibility" of the molecule overall. As such, a flexible element is one which is introduced into a region where prior to its addition more rigid elements were present. In embodiments, a flexible element in an oligonucleotide is an acyclic linker, which is more flexible than a cyclic backbone structure due to the absence of ring strain as compared to the cyclic structure.

According to an aspect of the invention, there is provided an oligonucleotide of the structure Ia:

[R¹-X]ₐ-R² **Ia**

wherein a is greater than or equal to 1, each of R¹ and R² are independently at least one nucleotide and X is an acyclic linker.

According to a further aspect of the invention there is provided an AON chimera of general structure Ib: 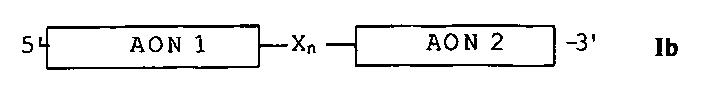 wherein n is greater than or equal to 1. With reference to structure Ib above, "AON1" is an oligonucleotide chain, which in embodiments is selected from the group consisting of FANA, DNA, S-DNA, and 5'-RNA-DNA-3' chimeras and combinations thereof; and "AON2" is an oligonucleotide chain, which in embodiments is selected from the group consisting of FANA, DNA, S-DNA, and 5'-DNA-RNA-3' chimeras and combinations thereof. The internucleotide linkages of the AON1 and AON2 include but are not necessarily limited to phosphodiester, phosphotriester, phosphorothioate, methylphosphonate, and phosphoramidate (5'N-3'P and 5'P-3'N) groups. The 2'-substituent of the arabinose sugar in ANA-containing constructs includes but is not limited to fluorine, hydroxyl, amino, azido, methyl, methoxy and other alkoxy groups (e.g., ethoxy, propoxy, methoxyethoxy, etc.).

Examples of the general structures Ia and Ib include phosphodiester linked FANA-X-FANA, RNA-DNA-X-DNA-RNA, (2'0-alkyl-)RNA-DNA-X-DNA-(2'O-alkyl)RNA, (2'-alkylalkoxy)RNA-DNA-X-DNA-(2'O-alkylalkoxy)RNA, and the corresponding phosphorothioate linked derivatives. Any of the above structures may comprise DNA. In a preferred embodiment, an oligonucleotide of the invention comprises at least one modified nucleotide, in an embodiment a modified deoxyribonucleotide.

"Acyclic" as used herein, with reference to linkers, refers to a linking backbone structure that does not have a cyclic portion. This feature relates to the backbone structure only, e.g. the backbone structure of an acyclic linker may have a branch or substituent extending therefrom comprising a cyclic group. An acyclic linker which links two nucleotides refers to a linker having a non-cyclic backbone structure joining the two nucleotides.

"Modified nucleotide/nucleoside" as used herein refers to a nucleotide/nucleoside that differs from and thus excludes the defined native form. For example, a modified deoxyribonucleotide is a molecule other than native DNA. Further, by such definition, a modified deoxyribonucleotide encompasses native RNA. Modifications may comprises additions, deletions or substitutions at one or more parts of a molecule, e.g. at the base, sugar phosphate and/or backbone portions.

"Nucleoside" refers to a base (e.g. a purine [e.g. A and G] or pyrimidine [e.g. C, 5-methyl-C, T and U]) combined with a sugar (e.g. [deoxy]ribose, arabinose and derivatives). " Nucleotide" refers to a nucleoside having a. phosphate group attached to its sugar moiety. In embodiments these structures may include various modifications, e.g. either in the base, sugar and/or phosphate moieties. "Oligonucleotide" as used herein refers to a sequence comprising a plurality of nucleotides joined together. An oligonucleotide may comprise modified structures in its backbone structure and/or in one or more of its component nucleotides. In embodiments, oligonucleotides of the invention are about 1 to 200 bases in length, in further embodiments from about 5 to about 50 bases, from about 8 to about 40 bases, and yet further embodiments, from about 12 to about 25 bases in length.

"Alkyl" refers to straight and branched chain saturated hydrocarbon groups (e.g. methyl, ethyl, propyl, butyl, isopropyl etc.). "Alkenyl" and "alkynyl" refer to hydrocarbon groups having at least one C-C double and one C-C triple bond, respectively. "Alkoxy" refers to an -O- alkyl structure. "Alkylamino" refers to -NH(alkyl) or - N(alkyl)₂ structures. "Aryl" refers to substituted and unsubstituted aromatic cyclic structures (e.g. phenyl, naphthyl, anthracyl, phenanthryl, pyrenyl, and xylyl groups). "Hetero" refers to an atom other than C; including but not limited to N, O, or S. In embodiments, the above-mentioned groups may be substituted.

In embodiments, an oligonucleotide of the invention has the structure II: 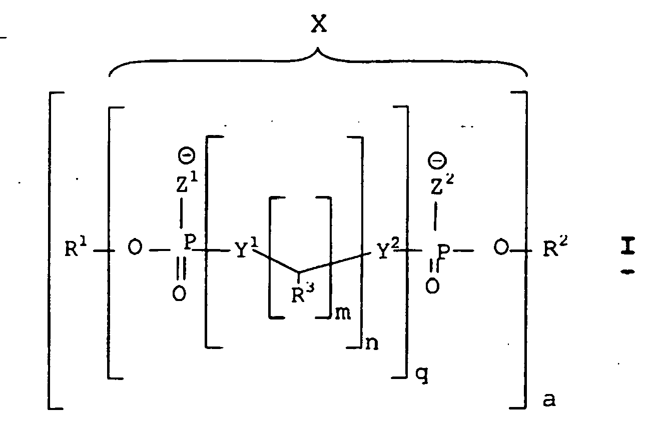 wherein m, n, and q are greater than or equal to 1, P¹ and P² are phosphorus atoms of phosphate groups which are linked to R¹ and R², respectively, each of Z¹ and Z² are independently selected from the group consisting of an oxygen atom, a sulfur atom, an amino group and an alkylamino group, each of Y¹ and Y² are independently selected from the group consisting of oxygen, sulfur and NH; and R³ is selected from the group consisting of H, alkyl, hydroxyalkyl, alkoxy, a "base" (including but not limited to a purine or a pyrimidine) and combinations thereof. In embodiments, the above-noted purine includes adenine and guanine and the above-noted pyrimidine includes thymine, cytosine and uracil. In embodiments, each of R¹ and R² noted above are selected from the group consisting of ANA, DNA, S-DNA, and 5'-DNA-RNA-3' chimeras or combinations thereof. In embodiments, the above-noted ANA comprises a 2'-substituent selected from the group consisting of fluorine, hydroxyl, amino, azido, alkyl (e.g. methyl, ethyl, propyl and butyl), alkylamino (e.g., propylamino), alkenyl (e.g., -CH=CH₂), alkynyl (e.g., -C≡CH), and alkoxy (*e.g*. methoxy, ethoxy, propoxy, and methoxyethoxy) groups. When the 2'-substituent is fluorine, the ANA is FANA. In embodiments, R¹ and/or R² comprise at least two nucleotides having at least one internucleotide linkage. In embodiments, the internucleotide linkage is selected from the group consisting of phosphodiester, phosphotriester, phosphorothioate, methylphosphonate, phosphoramidate (5'N-3'P and 5'P-3'N) groups and combinations thereof.

In certain embodiments, an oligonucleotide of the invention is selected from the group consisting of the compounds as set forth in structures IIa, IIb, IIc and IId given below: 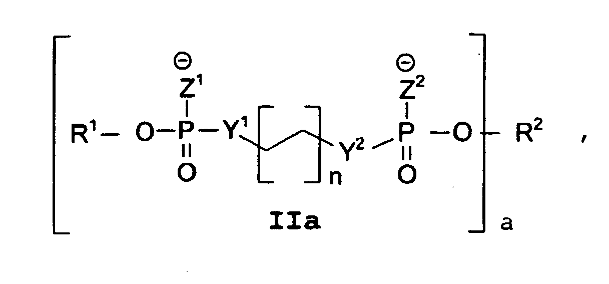 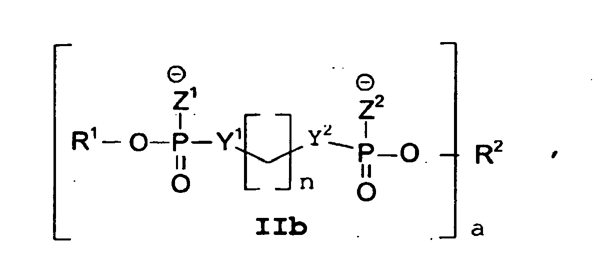 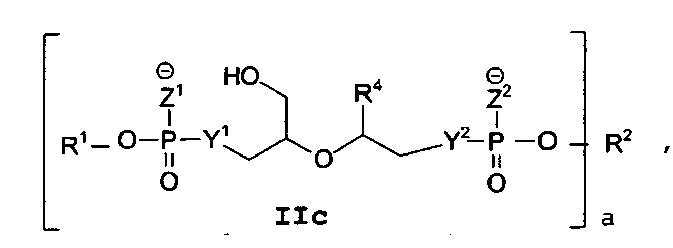 and 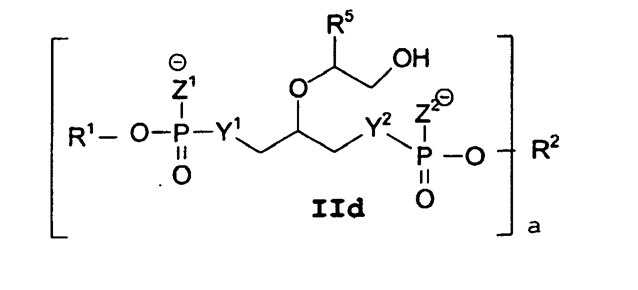 wherein R¹, R², n, a, Z¹, Z², Y¹ and Y² are as defined above. In embodiments, each of R⁴ and R⁵ are independently selected from the group consisting of a "base", which in embodiments includes but is not limited to a purine or a pyrimidine, examples of which are noted above.

In embodiments, oligonucleotides of the invention are those having the structure FANA-X-FANA, where, in embodiments, X is located at or near the middle of the oligonucleotide sequence, and the oligonucleotide has structure IIb (Y¹=Y²=Z¹=Z²=oxygen, and n=4) or structure IIc (Y¹=Y²=Z¹=Z²=oxygen, and a=1).

According to a further aspect of the invention, there are provided oligonucleotides of the general formula V:

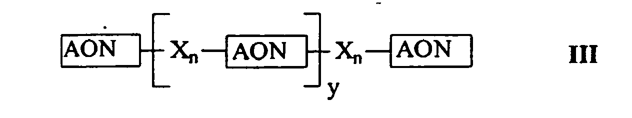

With reference to structure III above, each of y and n are independently an integer greater than or equal to 1; linker X is defined as described above. In embodiments, the oligonucleotide backbone in the definition of AON is selected from the group consisting of ANA (e.g. FANA), DNA, and PS-DNA, and other RNase H competent backbones such as alpha-L-LNA, cyclohexene nucleic acids, or combinations thereof. In embodiments, the internucleotide linkages of the AON includes but is not necessarily limited to phosphodiester, phosphotriester, phosphorothioate, methylphosphonate, phosphoramidate (5'N-3'P and 5'P-3'N) groups. The 2'-substituent of the arabinose sugar when the AON segment is ANA includes but is not limited to fluorine (i.e. FANA), hydroxyl, amino, azido, alkyl (e.g. methyl, ethyl, propyl, butyl, etc.), alkylamino (e.g., propylamino), alkenyl (e.g., -CH=CH₂), alkynyl (e.g., -C=CH), methoxy and other alkoxy groups (*e.g*., ethoxy, propoxy, methoxyethoxy, *etc.*). In embodiments the AON includes but is not necessarily limited to PS-RNA, PDE- or PS-RNA analogues (e.g., 2'-modified RNA in which the 2'-substituent comprises alkyl, 2'-alkoxy, 2'-alkylalkoxy, or 2'-F), locked nucleic acids (LNA), phosphorodiamidate morpholino nucleic acids, N3'-P5' phosphoramidate DNA, methylphosphonate DNA, and combinations thereof. In certain embodiments, examples of these oligonucleotides include: 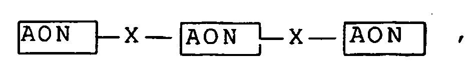 where, in an embodiment,
AON is 3-8 nt in length; and 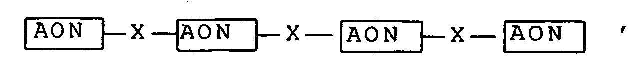 where, in an embodiment,
AON is 2-6 nt in length

It will be understood that other structures for the X linkers can be considered, e.g., biodegradable acyclic residues, and acyclic residues containing two types of monomers linked together by for example peptide bonds. Examples include but are not limited to the dipeptide glycine-glycine, and any combination of the naturally occurring amino acids or derivatives thereof. In embodiments, X is an N-(2-aminoethyl)glycine unit in which an heterocyclic base is attached via a methylene carbonyl linker. Other related acyclic peptide monomers may be considered, for example, the O-PNA monomers [-NH-CH(CH₂-CH₂-Base)-CH₂-O-CH₂-CO-] described by Kuwahara *et al., J*. *Am. Chem. Soc.* 2001, *123,* 4356.

In the case of a PNA-based acyclic linker, the 3' flanking group may have an amino group at its 5' terminus, which is linked to the acyclic (X) linker via an amide bond. Other acyclic linkers such as spermine and derivatives, as well as ethylene glycols (*e.g*. polyethylene glycol or PEG) and derivatives can be considered.

In various embodiments, the oligonucleotide may be designed such that the acyclic linker may or may not "loop out" when the oligonucleotide forms a duplex with its target molecule. "Loop out" as used herein refers to the case where the linker does not itself occupy a position in the oligonucleotide corresponding to a position in the target molecule, effectively forming a loop from the duplex once formed. In the case where the linker does not "loop out", it occupies a position in the duplex corresponding to a position in the bound target molecule.

The AONs of this invention contain a sequence that is complementary (in certain embodiments partially complementary, and in other embodiments exactly complementary) to a "target RNA", based on hybridization. "Hybridization" as used herein refers to hydrogen bonding between complementary nucleotides. The degree of complementarity between an AON and its target sequence may be variable, and in embodiments the AON is exactly complementary to its target sequence as noted above. It is understood that it is not essential that an AON be exactly complementary to its target sequence to achieve sufficient specificity, i.e. to minimize non-specific binding of the oligonucleotide to non-target sequences under the particular binding conditions being used (e.g. *in vivo* physiological conditions or *in vitro* assay conditions). "Target RNA" refers to an RNA molecule of interest which is the target for hybridizing with/binding to an oligonucleotide of the invention to prevent or decrease for example the translation, reverse transcription and or replication of the RNA. In embodiments, such prevention and inhibition is via an induction of RNase H-mediated cleavage of the target RNA, and therefore in an embodiment, the invention provides a method of cleaving a target RNA, said method comprising contacting the RNA with an oligonucleotide of the invention. In embodiments, such cleavage may be further facilitated by additionally providing conditions conducive to RNase H activity, such as buffer means (*e.g.* to control pH and ionic strength), temperature control means, and any other components which may contribute to an induction in RNase H activity. In certain embodiments, RNase H activity is of an RNase H enzyme or of a multifunctional enzyme possessing RNase H activity. In certain embodiments, such RNase H activity includes, but is not limited to RNase H activity associated with the reverse transcriptases of human pathogenic viruses such as HIV (e.g. the retroviruses HIV-1 and HIV-2) and the hepadnavirus hepatitis B virus. In further embodiments, such RNase H activity includes, but is not limited to RNase H activity associated with an RNase H enzyme of prokaryotic or eukaryotic origin, in an embodiment, of mammalian origin, in an embodiment, of human origin. In further embodiments, such RNase H activity includes, but is not limited to RNase H activity associated with RNase H1 and RNase H2 of eukaryotic or prokaryotic origin.

In embodiments, the above-noted RNA includes messenger RNA, or viral genomic RNA, such that the oligonucleotide can specifically inhibit the biosynthesis of proteins encoded by the mRNA, or inhibit virus replication, respectively. Partial modifications to the oligonucleotide directed to the 5' and/or 3'-terminus, or the phosphate backbone or sugar residues to enhance their antisense properties (e.g. nuclease resistance) are within the scope of the invention. As demonstrated in this invention (*vide infra*), these oligonucleotides meet one of the most important requirements for antisense therapeutics, i.e., they bind to target RNA forming an AON/RNA duplex that is recognized and degraded by human RNase H. Furthermore, as shown below, the efficiency by which the AON-X-AON chimera promotes RNA cleavage is superior to that seen with AONs lacking the acyclic modification (X or Xₙ linker).

Therefore, applicants' results presented herein establish that [R¹-X]ₐ-R², AON-X-AON and AON-Xₙ-AON chimeras are excellent models of antisense agents, and should serve as therapeutics and/or valuable tools for studying and controlling gene expression in cells and organisms.

As such, in alternative embodiments, the invention provides antisense molecules that bind to, induce degradation of and/or inhibit the translation of a target RNA (e.g. mRNA). Examples of therapeutic antisense oligonucleotide applications, incorporated herein by reference, include: U.S. Pat. No. 5,135,917, issued Aug. 4, 1992; U.S. Pat. No. 5,098,890, issued Mar. 24, 1992; U.S. Pat. No. 5,087,617, issued Feb. 11, 1992; U.S. Pat. No. 5,166,195 issued Nov. 24, 1992; U.S. Pat. No. 5,004,810, issued Apr. 2, 1991; U.S. Pat. No. 5,194,428, issued Mar. 16, 1993; U.S. Pat. No. 4,806,463, issued Feb. 21, 1989; U.S. Pat. No. 5,286,717 issued Feb. 15, 1994; U.S. Pat. No. 5,276,019 and U.S. Pat. No. 5,264,423; BioWorld Today, Apr. 29, 1994, p. 3.

Preferably, in antisense molecules, there is a sufficient degree of complementarity to the target RNA to avoid non-specific binding of the antisense molecule to non-target sequences under conditions in which specific binding is desired, such as under physiological conditions in the case of *in vivo* assays or therapeutic treatment or, in the case of *in vitro* assays, under conditions in which the assays are conducted. The target RNA for antisense binding may include not only the information to encode a protein, but also associated ribonucleotides, which for example form the 5'-untranslated region, the 3'-untranslated region, the 5' cap region and intron/exon junction ribonucleotides. A method of screening for antisense and ribozyme nucleic acids that may be used to provide such molecules as PLA₂ inhibitors of the invention is disclosed in U.S. Patent No. 5,932,435.

Antisense molecules (oligonucleotides) of the invention may include those which contain intersugar backbone_linkages such as phosphotriesters, methyl phosphonates, short chain alkyl or cycloalkyl intersugar linkages or short chain heteroatomic or heterocyclic intersugar linkages, phosphorothioates and those with CH₂--NH--O--CH₂, CH₂--N(CH₃)--O--CH₂ (known as methylene(methylimino) or MMI backbone), CH₂--O--N(CH₃)--CH₂, CH₂--N(CH₃)--N(CH₃)--CH₂ and O--N(CH₃)-CH₂ --CH₂ backbones (where phosphodiester is O-P(O)₂--O--CH₂). Oligonucleotides having morpholino backbone structures may also be used (U.S. Pat. No. 5,034,506). In alternative embodiments; antisense oligonucleotides may have a peptide nucleic acid (PNA, sometimes referred to as "protein" or "peptide" nucleic acid) backbone, in which the phosphodiester backbone of the oligonucleotide may be replaced with a polyamide backbone wherein nucleosidic bases are bound directly or indirectly to aza nitrogen atoms or methylene groups in the polyamide backbone (Nielsen *et al.,* Science, 1991, 254, 1497 and U.S. Pat. No. 5,539,082). The phosphodiester bonds may be substituted with structures that are chiral and enantiomerically specific. Persons of ordinary skill in the art will be able to select other linkages for use in practice of the invention.

As noted above, oligonucleotides may also include species which include at least one modified nucleotide base. Thus, purines and pyrimidines other than those normally found in nature may be used. Similarly, modifications on the pentofuranosyl portion of the nucleotide subunits may also be effected. Examples of such modifications are 2'-O-alkyl- and 2'-halogen-substituted nucleotides. Some specific examples of modifications at the 2' position of sugar moieties which are useful in the present invention are OH, SH, SCH₃, F, OCN, O(CH₂)ₙ NH₂ or O(CH₂)ₙ CH₃ where n is from 1 to about 10; C₁ to C₁₀ lower alkyl, substituted lower alkyl, alkaryl or aralkyl; Cl; Br; CN; CF₃ ; OCF₃ ; O-, S-, or N-alkyl; O-, S-, or N-alkenyl; SOCH₃ ; SO₂ CH₃; ONO₂ ; NO₂ ; N₃; NH₂; heterocycloalkyl; heterocycloalkaryl; aminoalkylamino; polyalkylamino; substituted silyl; an RNA cleaving group; a reporter group; an intercalator; a group for improving the pharmacokinetic properties of an oligonucleotide; or a group for improving the pharmacodynamic properties of an oligonucleotide and other substituents having similar properties. One or more pentofuranosyl groups may be replaced by another sugar, by a sugar mimic such as cyclobutyl or by another moiety which takes the place of the sugar.

Accordingly, in various embodiments, a modified oligonucleotide of the invention may be used therapeutically in formulations or medicaments to prevent or treat a disease characterized by the expression of a particular target RNA. In certain embodiments, such a target nucleic acid is contained in or derived from an infectious agent and/or is required for the function and/or viability and/or replication/propagation of the infectious agent. In certain embodiments, such an infectious agent is a virus, in certain embodiments, a retrovirus, in a further embodiment, HIV. In further embodiments the expression of such a target nucleic acid is associated with the diseases including but not limited to inflammatory diseases, diabetes, cardiovascular disease (e.g. restinosis), and cancer. The invention provides corresponding methods of medical treatment, in which a therapeutic dose of a modified oligonucleotide of the invention is administered in a pharmacologically acceptable formulation. In embodiments, an oligonucleotide may also be administered as a prodrug, whereby it is modified to a more active form at its site of action. Accordingly, the invention also provides therapeutic compositions comprising a modified oligonucleotide of the invention, and a pharmacologically acceptable excipient or carrier. The therapeutic composition may be soluble in an aqueous solution at a physiologically acceptable pH.

In an embodiment, such compositions include an oligonucleotide of the invention in a therapeutically or prophylactically effective amount sufficient to treat or prevent a disease characterized by the expression of a particular target nucleic acid, and a pharmaceutically acceptable carrier.

A "therapeutically effective amount" refers to an amount effective, at dosages and for periods of time necessary, to achieve the desired therapeutic result, such as a decrease in or a prevention of the expression of a particular target nucleic acid. A therapeutically effective amount of a modified nucleic acid of the invention may vary according to factors such as the disease state, age, sex, and weight of the individual, and the ability of the modified nucleic acid to elicit a desired response in the individual. Dosage regimens may be adjusted to provide the optimum therapeutic response. A therapeutically effective amount is also one in which any toxic or detrimental effects of the compound are outweighed by the therapeutically beneficial effects. A "prophylactically effective amount" refers to an amount effective, at dosages and for periods of time necessary, to achieve the desired prophylactic result, such as preventing or treating a disease characterized by the expression of a particular target nucleic acid. A prophylactically effective amount can be determined as described above for the therapeutically effective amount. For any particular subject, specific dosage regimens may be adjusted over time according to the individual need and the professional judgement of the person administering or supervising the administration of the compositions.

As used herein "pharmaceutically acceptable carrier" or "excipient" includes any and all solvents, dispersion media, coatings, antibacterial and antifungal agents, isotonic and absorption delaying_ agents, and the like that are physiologically compatible. In one embodiment, the carrier is suitable for parenteral administration. Alternatively, the carrier can be suitable for intravenous, intraperitoneal, intramuscular, sublingual or oral administration. Pharmaceutically acceptable carriers include sterile aqueous solutions or dispersions and sterile powders for the extemporaneous preparation of sterile injectable solutions or dispersion. The use of such media and agents for pharmaceutically active substances is well known in the art. Except insofar as any conventional media or agent is incompatible with the active compound, use thereof in the pharmaceutical compositions of the invention is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent which delays absorption, for example, monostearate salts and gelatin. Moreover, an oligonucleotide of the invention can be administered in a time release formulation, for example in a composition which includes a slow release polymer. The modified oligonucleotide can be prepared with carriers that will protect the modified oligonucleotide against rapid release, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, polylactic acid and polylactic, polyglycolic copolymers (PLG). Many methods for the preparation of such formulations are patented or generally known to those skilled in the art.

Sterile injectable solutions can be prepared by incorporating an active compound, such as an oligonucleotide of the invention, in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof. In accordance with an alternative aspect of the invention, an oligonucleotide of the invention may be formulated with one or more additional compounds that enhance its solubility.

Since the oligonucleotides of the invention are capable of inducing the RNase H-mediated cleavage of a target RNA, thus decreasing the production of the protein encoded by the target RNA, the modified oligonucleotides of the invention may be used in any system where the selective inactivation or inhibition of a particular target RNA is desirable. As noted above, examples of such uses include antisense therapeutics, in which expression of the target RNA is associated with illness or disease.

A further example of such a use is the selective depletion of a particular target gene product in a system to study the phenotypic effect(s) of such depletion on the system. Observations made via such depletion studies may thus allow the determination of the function of the target gene product. In certain embodiments, such uses include "target validation", in which the above-described strategy enables the confirmation as to whether a particular target nucleic acid is associated with a particular phenotype or activity, and thus allows "validation" of the target. The above noted system may be cell or cell-free; *in vitro* prokaryotic or eukaryotic.

The invention further provides commercial packages comprising an oligonucleotide of the invention. In certain embodiments, such commercial packages further comprise at least one of the following instructions for use of the oligonucleotide for (a) decreasing the expression of a target RNA sequence; (b) inducing the RNase H cleavage of a target RNA sequence; (c) preventing or treating a disease characterized by the expression of a particular RNA target; and (d) validating a particular gene target.

Although various embodiments of the invention are disclosed herein, many adaptations and modifications may be made within the scope of the invention in accordance with the common general knowledge of those skilled in this art. Such modifications include the substitution of known equivalents for any aspect of the invention in order to achieve the same result in substantially the same way. Numeric ranges are inclusive of the numbers defining the range. In the claims, the word "comprising" is used as an open-ended term, substantially equivalent to the phrase "including, but not limited to". The following examples are illustrative of various aspects of the invention, and do not limit the broad aspects of the invention as disclosed herein.

### EXAMPLES

### EXAMPLE 1

### Synthesis of Acyclic Precursors Suitable for their Incorporation into Oligonucleotides

### Precursor to Acyclic Residue IIb

Dimethoxytrityl-O-CH₂CH₂CH₂CH₂-O-P(Ni-Pr₂) OCH₂CH₂CN (1) was purchased from ChemGenes Corp. (Ashland, MA), and was used as received for the synthesis of AON-X-AON chimeras (See Example 3) .

### Precursor to Acyclic Residues IIc and IId

The acyclic nucleoside residues (herein referred to as 2',3'-seconucleotides) consist of a 1-[1,5-dihydroxy-4(*S*)-hydroxymethyl-3-oxapent-2(*R*)-yl]-uracil unit which has been appropriately protected and functionalized for oligonucleotide incorporation as described below.

### Step A. Synthesis of 5'-monomethoxytrityl-2',3'-seco-β-D-ribouracil (2).

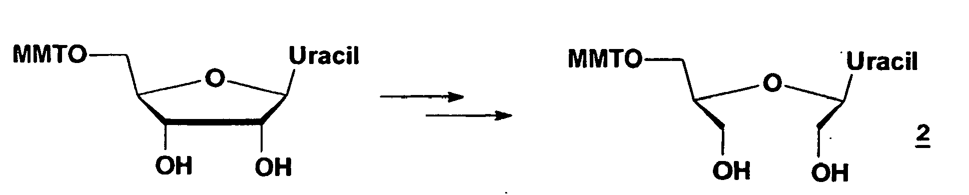

To a 0.1 M solution of 5'-monomethoxytrityluridine (5'-MMT-rU, 5.16 g, 10 mmol; prepared as described in T. Wu, K.K. Ogilvie, R.T. Pon. 1989. "Prevention of Chain Cleavage in the Chemical Synthesis of 2'-Silylated Oligoribonucleotides." *Nucleic Acids Res.,* 3501-17.) in dioxane was added a saturated solution of NaIO₄ in H₂O (2.26 g, 10.6 mmol, 1.06 eq) and the reaction allowed to proceed at r.t. for 2-3 h until complete conversion to the dialdehyde was observed by TLC visualization (R_{f} 0.52 in CH₂Cl₂:MeOH, 9:1). The reaction was diluted with dioxane (100 mL), filtered to remove NaIO₃ salts and followed by *in situ* reduction of the dialdehyde via treatment with NaBH₄ (0.378 g, 10 mmol, 1.0 eq) for 10-20 min. at r.t. The reaction mixture was quenched with acetone, neutralized with 20% acetic acid and concentrated to an oil under reduced pressure. The residue was then diluted with CH₂Cl₂ (200 mL) and washed with H₂O (2 x 75 mL). The aqueous layer was back-extracted and the combined organic layers were dried using anhydrous Na₂SO₄, filtered and evaporated to give the product as a pure white foam in 98% isolated yield (5.08 g; 9.8 mmol). R_{f} (CH₂Cl₂:MeOH, 9:1) 0.18; FAB-MS (NBA) : 519.6; Calc: 518.57.

### Step B. Synthesis of 5'-O-MMT-2'-O-t-butyldimethysilyl-2',3'-secouridine (3a) and 5'-O-MMT-3'-O-t-butyldimethysilyl-2',3'-secouridine (3b).

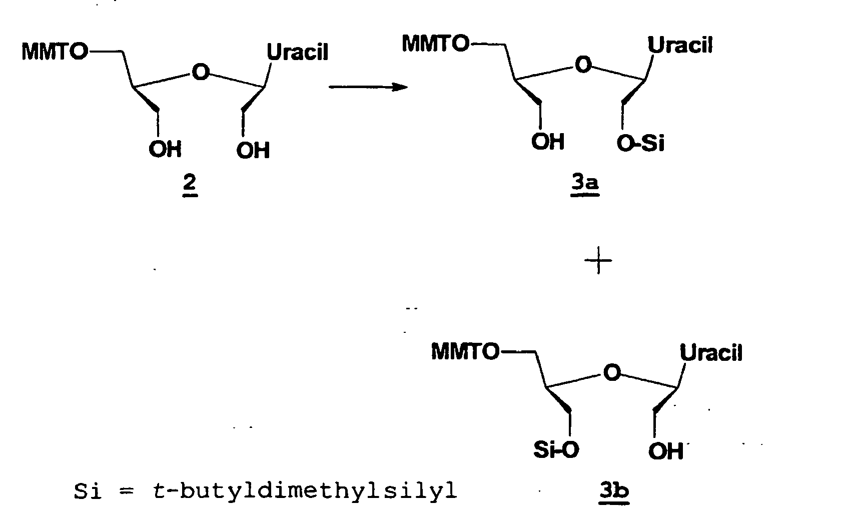

Monoprotection of either of the free hydroxyl functions of **2** was achieved nonselectively by adding *t-*butyldimethylsilyl chloride (0.81 g, 5.4 mmol, 1.1 eq) to a stirred 0.1 M solution of **2** (2.55 g, 4.9 mmol) in dry THF at 0°C containing a suspension of AgNO₃ (0.92 g, 5.39 mmol, 1.1 eq). The reaction temperature was returned to r.t. after 20 min and maintained as such for 24 h. The workup was initiated by filtering the mixture directly into an aqueous solution of 5% NaHCO₃ (50 mL), followed by extraction of the aqueous layer twice with CH₂Cl₂. The combined organic layers were dried (anhydrous Na₂SO₄), filtered and evaporated under reduced pressure to give the crude product as a yellow oil. The residue was purified by flash silica gel column chromatography using a gradient of 0-25% acetone in CH₂Cl₂ to recover both monosilyl isomers as pure white foams. Isolated yields for **3a** and **3b** were 22% and 14%, respectively. R_{f} (CH₂Cl₂:Et₂O, 3:1) **3a**, 0.18; **3b**, 0.05. FAB-MS (NBA): 633.4; Calc: 632.83.

The regioisomers are distinguished on the basis of COSY-NMR cross-peak correlations that are used to demonstrate the connectivity of the protons in the acyclosugar. In both spectra, the H1' protons are split by the nonequivalent H2' and H2" protons into a doublet of doublets which suggests a certain degree of structural rigidity around the C1'-C2' bond. More significantly, a single well-resolved hydroxyl peak is observed for both **3a** and **3b** in DMSO-*d*_{*6*} which negates rapid chemical exchange of these moieties. As a result, the effect of the protons at C2' of **3b** is transmitted to the 2'-hydroxyl proton which in turn appears as an overlapping doublet of doublets. In **3a,** splitting of the hydroxyl peak is also observed, however it shows correlations with H4' and H4" and therefore rules out the presence of a silyl group at the 3'-position. Taken together, these data confirm the assignment of **3a** and **3b** as the 2'- and 3'-monosilylated isomers, respectively.

### Step C. (a) Synthesis of 5'-O-MMT-2'-O-t-butyldimethysilyl] 2',3'-secouridine-3'-O-[N,N-diisopropylamino-(2-cyanoethyl)]phosphoramidite (4a).

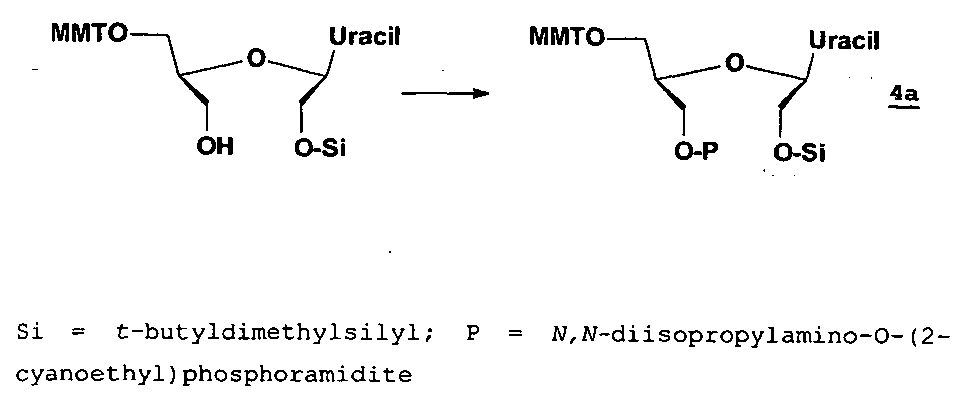

To a nitrogen purged solution of 4-dimethylaminopyridine (DMAP; 12 mg, 0.10 mmol, 0.1 eq), N,N-diisopropylethylamine (DIPEA; 0.68 mL, 3.9 mmol, 4 eq) and **3a** (620 mg, 0.98 mmol) in THF (0.2 M) at 0°C was added N,N-diisopropylamino-β-cyanoethylphosphonamidic chloride (0.24 mL, 1.1 mmol, 1.1 eq) dropwise over 5 min. The immediate appearance of a white precipitate due to the rapid formation of diisopropylethylammonium hydrochloride signified sufficiently anhydrous conditions, and the reaction was allowed to warm to r.t. whereupon it was stirred for 2.5 h prior to the reaction workup. Briefly, the reaction mixture was diluted with EtOAc (50 mL, prewashed with 5% NaHCO₃) and washed with saturated brine (2 x 20 mL). The recovered organic layer was dried (anhydrous Na₂SO₄), filtered and the solvent removed via reduced pressure, yielding a crude yellow oil. Coevaporation of the crude product with Et₂O afforded a pale yellow foam. Purification of the product by flash silica gel column chromatography using a CH₂Cl₂:Hexanes:TEA gradient system (25:74:1 adjusted to 50:49:1) afforded a white foam in 97% isolated yield. R_{f} (EtOAc:Tol, 4:1) 0.86, 0.72. FAB-MS (NBA): 833.4; Calc: 833.05.

### (b) Synthesis of 5'-O-MMT-3'-O-t-butyldimethysilyl-2',3'-secouridine-2'-O-[N,N-diisopropylamino-(2-cyanoethyl)]phosphoramidite (4b).

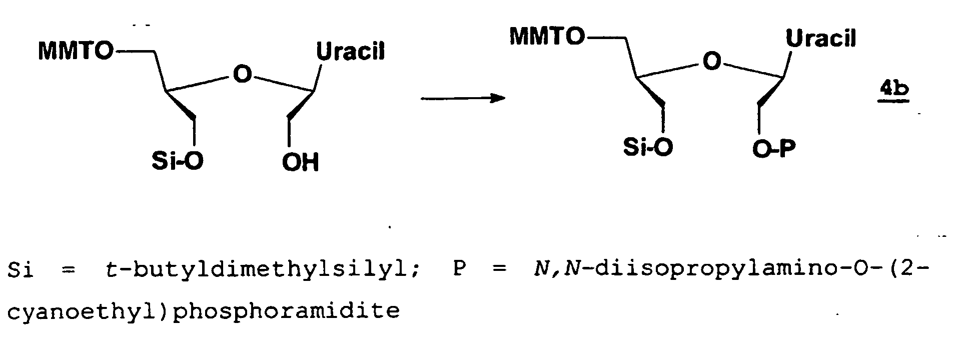

All conditions used in the preparation of the 3'-phosphoramidite **(4b)** were identical to those performed on its regioisomeric counterpart, **4a** (see step B). Flash column purification of this isomer afforded a white foam in 99% isolated yield. R_{f} (EtOAc:Tol, 4:1) 0.77, 0.65. FAB-MS (NBA): 833.3; Calc: 833.05.

### EXAMPLE 2

### Preparation of AONs constructed from 2'-deoxy-2'-fluoro-β-D-arabinonucleotides (FANA) flanking an acyclic butanediol or secouridine residues

### 1. Synthesis of FANA-X-FANA chimeras, where X= butanediol linker = IIb (Y=Z=oxygen;n= 4)

The synthesis of PDE-FANA oligomers was conducted as previously described (Damha *et al. J. Am. Chem. Soc.* 1998, *120,* 12976; Wilds, C.J. & Damha, M.J. *Nucleic Acids Res.* 2000, *28*, 3625). Their structure was confirmed via Maldi-TOF mass spectrometry.

Synthesis of PDE-(FANA-But-FANA) chimeras were performed on a 1 micromole scale using an Expedite 8909 DNA-synthesizer. Long-chain alkylamine controlled-pore glass (LCAA-CPG) was used as the solid support. - The synthesis cycle consisted of the following steps:
1) Detritylation of nucleoside/tide bound to CPG (3% trichloroacetic acid/dichloroethane): 150 sec for MMT; 60 sec for DMT removal.
2) Coupling of 2'-F-arabinonucleoside or dimethoxytrityl-butanediol phosphoramidite monomers: 15 min. Concentration of monomers used were 50 mg/mL for araF-T, araF-C and 60 mg/mL for araF-A and butanediol monomers (acetonitrile as solvent).
3) Acetylation using the standard capping step: 20 sec. The capping solution consisted of 1:1 (v/v) of "capA" and "capB" reagents. CapA: acetic anhydride/collidine/THF (1:1:8 ml); cap B: N-Methylimidazole/THF (4:21 ml).
4) Extensive washing with acetonitrile (50 pulses).
5) Oxidation with a fresh solution of I₂:collidine:THF: 5 sec.
6) Washing with acetonitrile: 20 pulses.
7) Drying of the solid support by addition of the capping reagent (see step 3): 5 sec.
8) Washing with acetonitrile (20 pulses).

Following chain assembly, oligonucleotides were cleaved from the solid support and deprotected as previously described (Noronha, A.M. *et al. Biochemistry* 2000, *39,* 7050). The crude oligomers were purified by anion-exchange HPLC followed by desalting (Sephadex G-25 or SepPak cartridges). Yields: 10-15 A₂₆₀ units

### Conditions for HPLC Purification:

Column: Protein Pak DEAE-5PW (7.5mm X 7.5cm, Waters),
Solvents: Buffer A: H₂O; Buffer B: 1M LiClO₄ (or NaClO₄),
Gradient: 0 - 20% B, linear over 60 min.

Loading was 0.5 - 1 A₂₆₀ units for analysis and 50-80 A₂₆₀ units for preparative separation. Flow rate was set at 1 mL/min, temperature was adjusted at 50°C. The detector was set at 260 nm for analytical and 290 nm for preparative chromatography. Under these conditions, the desired full-length oligomer eluted last.

The base sequence and hybridization properties of the oligonucleotides synthesized are given in Table 1.

### 2. Synthesis of FANA-X-FANA chimeras, where X is secouridine (SEC) linker IIc.

Phosphodiester FANA-SEC-FANA and FANA-SEC-SEC-FANA chimeras were synthesized analogously to the butanediol chimeric constructs (vide *supra)* using a concentration of 50 mg/mL of 2',3'-*seco*uridine monomers for the coupling step. Yields of the oligonucleotides after their cleavage from the solid support, deprotection, purification (HPLC) and desalting (SepPak cartridges) as described above were 10 A₂₆₀ units. Their structure was confirmed via Maldi-TOF mass spectrometry.

The base sequence and hybridization properties of the oligonucleotides synthesized are given in Table 1.

### 3. Synthesis of DNA-X-DNA chimeras, where X=butanediol linker=IIb (Y=Z= oxygen, and n= 4)

The synthesis and purification of phosphodiester DNA-But-DNA chimeras proceeded in the same manner as described above for phosphodiester FANA-But-FANA oligomers with few minor exceptions. The concentration of 2'-deoxyribonucleoside monomers and butanediol phosphoramidite used was 50 and 60 mg/mL, respectively in conjunction with a shorter coupling time (2 min) per addition of each type of monomer. Yields after purification (HPLC) and desalting (Sephadex G-25): 22 A₂₆₀ units. Their structure was confirmed by Maldi-TOF mass spectrometry.

The base sequence and hybridization properties of the oligonucleotides synthesized are given in Table 1.

### 4. Synthesis of phosphorothioate FANA-X-FANA and phosphorothioate DNA-X-DNA chimeras, where X=butanediol linker=IIb (Y= oxygen, Z= sulfur, and n= 4)

Synthesis of phosphorothioate FANA-But-FANA and phosphorothioate DNA-But-DNA oligomers was performed as described above for the phosphodiester (PDE) oligonucleotides. The main difference being the replacement of the iodine/water oxidation reagent with 0.1 M solution of 3-amino-1,2,4-dithiazoline-5-thione (ADTT) in pyridine/acetonitrile (1/1, v/v). Specifically, the phosphorothioate compounds were synthesized on a 1 micromol scale using an Expedite 8909 DNA-synthesizer. Long-chain alkylamine controlled-pore glass (LCAA-CPG) was used as the solid support. The synthesis cycle consisted of the following steps: (a) Detritylation of nucleoside/tide bound to CPG (3% trichloroacetic acid/dichloromethane): 150 sec.; (b) Coupling of 2'-F-arabinonucleoside or 2'-deoxyribonucleoside 3'-phosphoramidite monomers: 15 min or 1.5 min respectively. Concentration of monomers used were 50 mg/mL for araF-T, araF-C, DNA and butanediol linker monomers, and 60 mg/mL for araA and ara F-G (acetonitrile as solvent); (c) Acetylation using the standard capping step: 20 sec. The capping solution consisted of 1:1 (v/v) of "capA" and "capB" reagents. CapA: acetic anhydride/collidine/THF (1:1:8 ml); cap B: N-Methylimidazole/THF (4:21 ml); (d) Extensive washing with acetonitrile (50 pulses); (e) Sulfurization with a solution of 0.1 M 3-amino-1,2,4-dithiazoline-5-thione (ADTT) in pyridine/acetonitrile (1/1, v/v), 10 min. (f) Washing with acetonitrile: 20 pulses; (g) Drying of the solid support by addition of the capping reagent (see step 3): 5 sec; (h) Washing with acetonitrile (20 pulses).

Following chain assembly, oligonucleotides were cleaved from the solid support and deprotected by treatment with conc. aqueous ammonia (r.t., 16 h). The crude oligomers were purified by either (a) preparative gel electrophoresis (24% acrylamide, 7M Urea) following by desalting (Sephadex G-25), or (b) anion-exchange HPLC following by desalting (SepPak cartridges). Yields: 30-70 A₂₆₀ units. Conditions for HPLC Purification: Column: Protein Pak DEAE-5PW (7.5mm X 7.5cm, Waters), Solvents: Buffer A: H₂O; Buffer B: 1M NaClO₄, Gradient: 100% buffer A isocratic for 12 min, 100% A- 15% B , linear (over 5 min), 15% B - 55% B , linear (over 60 min); Flow rate was set at 1 ml/min, temperature was adjusted at 50°C. The detector was set at 260 nm for analytical and 290 nm for preparative chromatography. Under these conditions, the desired full-length oligomer eluted last. The structure of oligonucleotides was confirmed via Maldi-TOF mass spectrometry.

**Table 1. Melting Temperatures (Tₘ) and RNase H Mediated Hydrolysis Profiles for the AON/RNA Heteroduplexes^{a}**

| | Sequence type^{*b,c*} (5'±3') | SEQ ID NO: | *T*ₘ (°C) | Relative rates (*k*_{*rel*}) of enzyme cleavage^{*d*} |
|---|---|---|---|---|
| | **(i) DNA** | | | |
| I | ttt ttt ttt ttt ttt ttt | 1 | 39 | 1 |
| II | ttt ttt ttt Btt ttt ttt | 2 | 33 | 2.7 |
| III | tta tat ttt ttc ttt ccc | 3 | 53 | 1 |
| IV | tta tat ttt Btc ttt ccc | 4 | 48 | 3.4 |
| V | tta tat ttt ctc ttt ccc | 5 | 40 | 0.7 |
| VI | tta tat ttt B ttc ttt ccc | 6 | 48 | 2.5 |
| | **(ii) 2'F-ANA** | | | |
| VII | TTT TTT TTT TTT TTT TTT | 7 | 53 | 1 |
| VIII | TTT TBT TTT TTT TTT TTT | 8 | 49 | 0.6 |
| IX | TTT TTT TTT BTT TTT TTT | 9 | 47 | 7.9 |
| X | TTT TTT TTT TTT BTT TTT | 10 | 49 | 5.1 |
| XI | TTT TTT TTT STT TTT TTT | 11 | 47 | 1.6 |
| XII | TTT TTT TTS STT TTT TTT | 12 | 42 | 2.8 |
| XIII | TTA TAT TTT TTC TTT CCC | 13 | 64 | 1 |
| XIV | TTA TAT TTT BTC TTT CCC | 14 | 55 | 3.5 |
| XV | TTA TAT TTT *C*TC TTT CCC | 15 | 55.5 | 0.9 |
| XVI | TTA TAT TTT tTC TTT CCC | 16 | 63 | 1.6 |
| XVII | TTA TAT TTT B TTC TTT CCC | 17 | 57 | 2.3 |
| | **(iii) Ha-ras AON**^{**e**} | | | |
| XVIII | att ccg tca tcg ctc ctc | 18 | 69.9 | 33.8 |
| XIX | att ccg tca Bcg ctc ctc | 19 | 58.2 | 31.6 |
| XX | att ccg tca *c*cg ctc ctc | 20 | 63.1 | 31.9 |
| XXI | ATT CCG TCA TCG CTC CTC | 21 | 82.0 | 1 |
| XXII | ATT CCG TCA BCG CTC CTC | 22 | 71.7 | 23.3 |
| | **(iv) Phosphorothioate-AON sequences** ^{***f***} | | | |
| XXIII | tat tcc gtc ate get cct ca | 23 | 64 | >>33 |
| XXIV | tat tcc gtc ate Bct cct ca | 24 | 50 | 32.7 |
| XXV | TAT TCC GTC ATC GCT CCT CA | 25 | 74 | 1 |
| XXVI | TAT TCC GTC ATC BCT CCT CA | 26 | 64 | 13 |

| | | | | |
|---|---|---|---|---|
| ^{*a*}Aqueous solutions of 2.8 x 10⁻⁶ M of each oligonucleotide, 140 mM KCl, 1 M MgCl₂, 5 mM Na₂HPO₄ buffer (pH 7.2); uncertainty in *T*ₘ is ±0.5°C. ^{*b*}Target RNA sequences correspond to rA₁₈ (SEQ ID NO: 27), or 5'-r(GGGAAAGAAAAAAUAUAA)-3' (SEQ ID NO: 28). ^{*c*}Upper case letters, 2'F-ANA nucleotides; lower case letters, deoxynucleotides; *c*, arabinofluoro- or deoxycytidine mismatch residue; B, butanediol linker; S, 2'-secouridine insert. ^{*d*}Human enzyme; rates shown have been obtained at 22°C and are normalized according to the parent strand of each series except within Ha-ras sequences in which data are normalized to all-FANA AON (entry XXI). ^{*e,f*}Target RNA (40mer) sequence: 5'- r(CGCAGGCCCC**UGAGGAGCGAUGACGGAAUA**UAAGCUGGUG)-3'(SEQ ID NO: 29); ^{*e*}underlined and ^{*f*}bold residues denote the region in the RNA to which the AON binds. | | | | |

### EXAMPLE 3

### Expression and Purification of human RNase HII

### Expression of human RNase HII

A *hrnh* gene fragment from pcDNA/GS/hrnh (Invitrogen) was obtained by PCR using the primers: AGC TAT CTC GAG ATG AGC TGG CTT CTG TTC CTG GCC (XhoI) (SEQ ID NO: 30) and GGC CGC AAG CTT TCA GTC TTC CGA TTG TTT AGC TCC (HindIII) (SEQ ID NO: 31). This fragment was cloned in the XhoI/HindIII sites of the bacterial expression vector pBAD/His (Invitrogen). Recombinant human RNase HII from pBAD/His/hrnh expression plasmid was purified as follows. To overcome codon bias in *E*. *coli,* we transformed the expression vector in *E*. *coli* BL21 codonplus (Stratagene) and cultured in LB broth containing 100 µg/ml ampicillin at 37°C until the OD₆₀₀ reached 0.5-0.6. Then, the recombinant protein was induced with 0.002 % arabinose for 4 h.

### Purification of human RNase HII

After induction, *E*. *coli* cells were harvested by centrifugation, washed in chilled wash buffer (100 mM phosphate, pH 8.0, 300 mM NaCl), resuspended in chilled lysis buffer (100 mM phosphate, pH 8.0, 10 Units/ml DNase, 2 mM phenylrnethylsulfonyl fluoride, 300 mM NaCl, 200 µg/ml lysozyme), and lysed by 0.5 % NP-40. The supernatant was applied to a Ni²⁺-nitrilotriacetate-agarose column after being centrifuged, and the protein was purified according to the manufacturer's directions (Qiagen). The eluate was treated with 2 mM EDTA to chelate any residual Ni²⁺ ions, dialyzed against 10 mM Tris-HCl, pH 8.0, and then concentrated by ultrafiltration. The protein was treated with enterokinase (1 unit/20 microgram of protein) in 50 mM Tris, pH 8.0, 1 mM CaCl₂, 0.1% Tween-20 overnight at 37°C. Then the digested sample was loaded on a Heparin-Sepharose column (Amersham-Pharmacia) pre-equilibrated with 20 mM phosphate buffer (pH 8.0). After elution with a gradient of 0.1 - 0.5 M NaCl, the desired peak was pooled, dialyzed, and concentrated.

### EXAMPLE 4

### Induction of Human Ribonuclease H (RNase HII) Activity by AON-X-AON Oligonucleotides

### PDE-FANA versus PDE-[FANA-X-FANA], where x= butanediol linker = IIb (Y=Z= oxygen, and n= 4)

### A. Homopolymeric Sequences.

Defined-sequence oligonucleotides, 18-units in length, were used in these experiments:

| | |
|---|---|
| 5'-araF(TTT TTT TTT TTT TTT TTT)-3' | (SEQ ID NO: 7) |
| T series "FANA" | |
| 5'-araF(TTT TTT TTT XTT TTT TTT)-3' | (SEQ ID NO: 9) |
| T series "FANA-But" | |

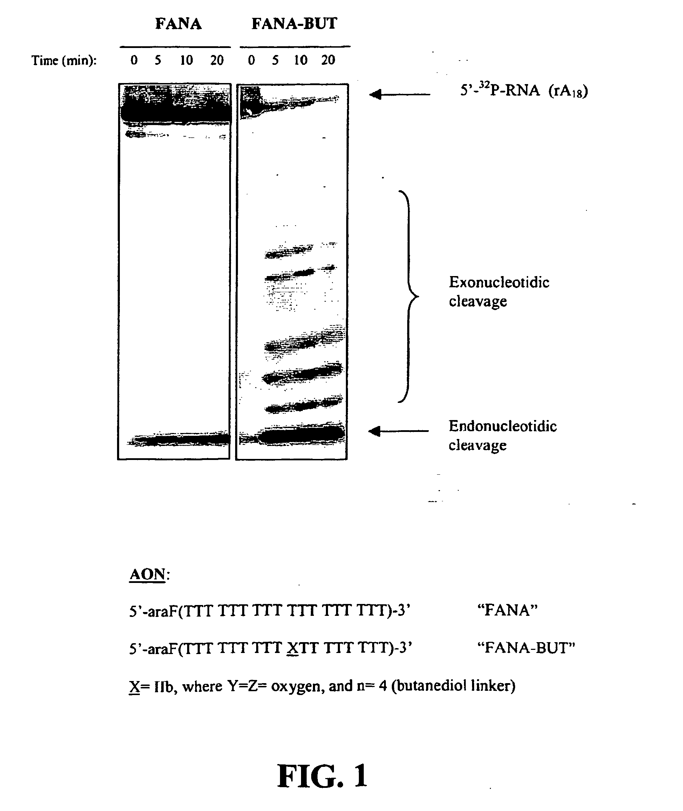

The residue X in the sequences above corresponds to acyclic residue IIb, where Y=Z= oxygen, and n= 4 (or butanediol linker). The target RNA used was octadecariboadenylate (rA₁₈) complementary to the sequence of the above oligonucleotides. The ability of the above oligonucleotides to elicit RNase H degradation of target RNA was determined in assays (10 µL final volume) that comprised 1 pmol of 5'-[³²P]- target RNA and 3 pmol of test oligonucleotide in 60 mM Tris-HCl containing 2mM dithiothreitol, 60 mM KCl, and 2.5 mM MgCl₂ (pH 7.8, 22°C). Prior to addition of the enzyme, the mixture was heated at 75 °C for 2 minutes, and then cooled slowly to room temperature to allow duplex formation. Reactions were started by- the addition of human RNase HII at room temperature. Reactions were quenched by the addition of 10 µL of loading buffer (98% deionized formamide containing 10 mM EDTA and 1mg/mL each of bromophenol blue and xylene cyanol), and heating at 100°C for 5 minutes. The reaction products were resolved by electrophoresis using a 16% polyacrylamide sequencing gel containing 7 M urea, and visualized by autoradiography. The result of such an experiment is shown in FIGURE 1.

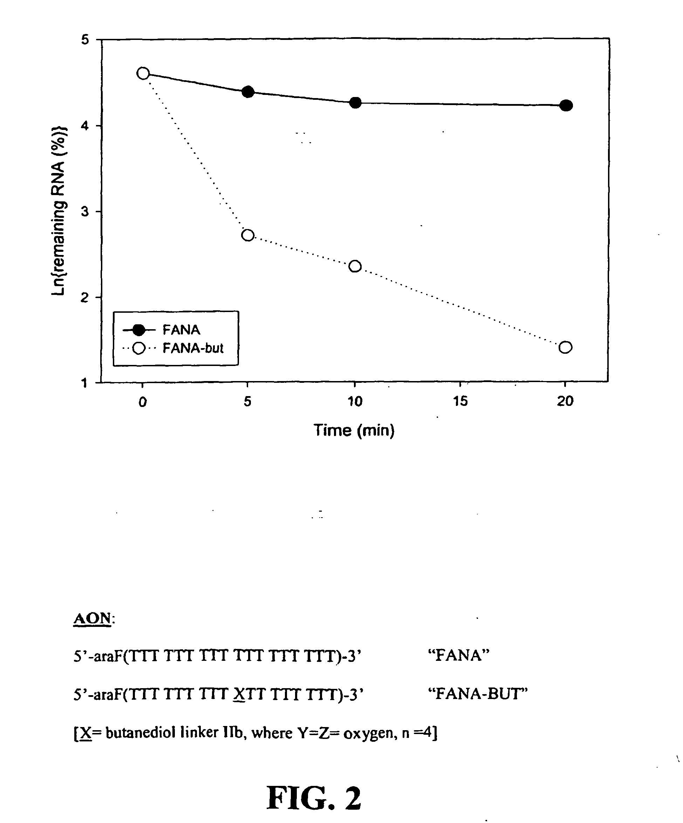

The results show that both "FANA" and "FANA-BUT" oligomers (T series) are able to form duplexes with target RNA that serve as substrates for the activity of human RNase HII, as indicated by the degradation products of the target RNA in FIGURE 1. In the case of FANA this RNase H degradation was noted by the appearance of a fast moving band formed by the endonuclease activity of RNase HII. In the case of the "FANA-BUT" oligomer, degradation results from both the endo- and exonuclease activity of the enzyme, as evidenced by the appearance of numerous smaller sized RNA degradation products. Quantitation of rA₁₈ remaining as a function of time indicates that the rate of cleavage is 8 times faster with "FANA-But" than with "FANA" (TABLE 1 and FIGURE 2).

The same trend was observed when mixed-based phosphodiester oligonucleotides were targeted against complementary RNA sequences (Examples 4B and 8). In Example 8, oligonucleotides containing the four naturally occurring heterocyclic bases (A, G, C and T) were designed to target 40- and 50-nt long RNA targets. In these cases, the rate enhancement of RNase H-mediated RNA cleavage observed was even more dramatic, reaching 23-fold in favor of the FANA-But-FANA over the FANA compounds.

### B. Mixed Base Sequence

Defined-sequence oligonucleotides, 18-units in length, were used in these experiments:

| | |
|---|---|
| 5'-araF (TTA TAT TTT TTC TTT CCC)-3' | (SEQ ID NO: 13) |
| CAT "FANA" | |
| 5'-araF(TTA TAT TTT XTC TTT CCC)-3' | (SEQ ID NO: 14) |
| CAT "FANA-But" | |
| 5'-araF(TTA TAT TTT CTC TTT CCC)-3' | (SEQ ID NO: 15) |
| CAT "FANA-Mismatch" | |

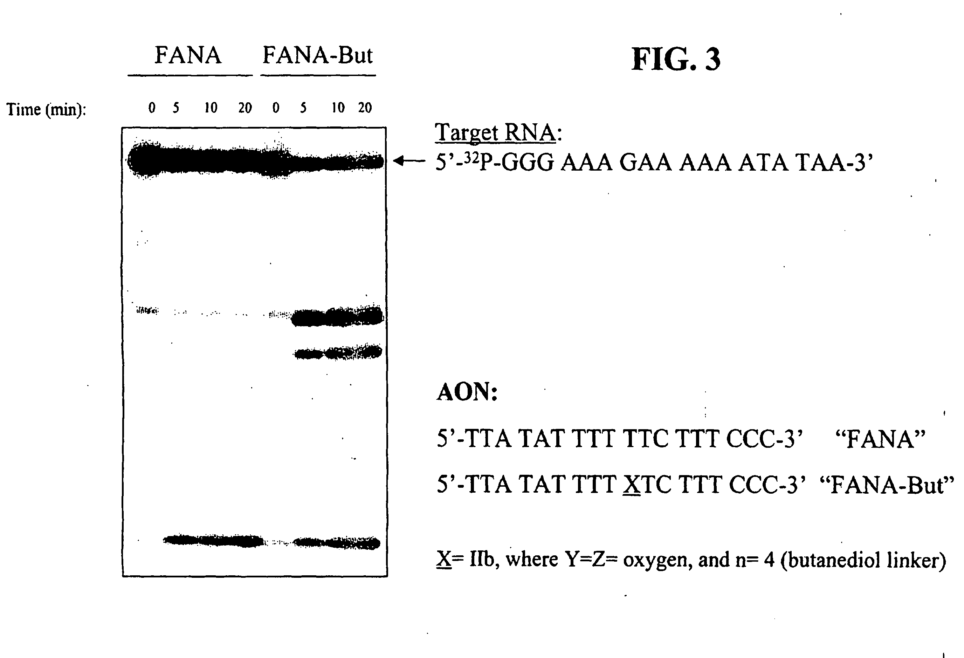

The residue X in the sequences above corresponds to acyclic residue IIb, where Y=Z= oxygen, and n= 4 (or butanediol linker). The target RNA used was r(GGGAAAGAAAAAAUAUAA) (SEQ ID NO: 28), exactly complementary to the sequence of the first two oligonucleotides. The third oligonucleotide, CAT "FANA-Mismatch" contains an araF-C mismatch at position 10. This oligomer exhibits the same binding affinity as the "FANA-But" sequence, and was tested in order to assess the effect of a butanediol "linker" versus an araF-C mismatch "linker". Therefore, the ability of "FANA", "FANA-But", and "FANA-Mismatch" (CAT series) to elicit RNase H degradation of target RNA was determined in assays (10 µL final volume) that comprised 1 pmol of 5'-[³²P]- target RNA and 3 pmol of test oligonucleotide in 60 mM Tris-HCl containing 2mM dithiothreitol, 60 mM KCl, and 2.5 mM MgCl₂ (pH 7.8, 22°C). Assays were carried out as described above for the homopolymeric sequences (Example 4A). The result of such an experiment is shown in FIGURE 3.

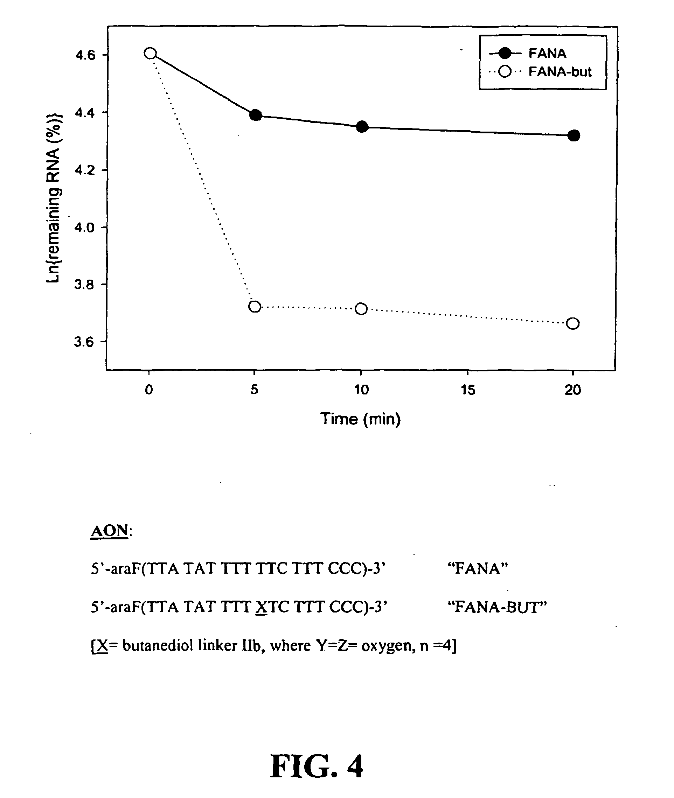

The results show that both "FANA" and "FANA-But" (CAT series) are able to form duplexes with target RNA that serve as substrates for the activity of human RNase HII, as indicated by the appearance of numerous smaller sized degradation products. Quantitation of RNA target remaining as a function of time indicate that the rate of cleavage is significantly faster (3.5 fold) with "FANA-But" than with "FANA" (FIGURE 4).

The data also show that RNase H activity is diminished in the FANA-mismatch oligomer, where the more rigid araF-C "linker" replaces the more flexible butanediol linker (TABLE 1). Because the araF-C mismatch also induces an equivalent drop in duplex thermal stability relative to the butanediol insertion (Δ*T*m = -9 °C, TABLE 1), it can be concluded that increased turnover (i.e., enhanced rate of dissociation from target RNA) is not the sole basis for preferential enzyme discrimination towards the more flexible But linker.

### EXAMPLE 5

### Human Ribonuclease H (RNAse HII) Activity as a Function of Position of Butanediol Linker IIb (Y=Z= oxygen, and n= 4)

The following oligonucleotides, 18-units in length, were used in these experiments:

| | |
|---|---|
| 5'-araF(TTT TXT TTT TTT TTT TTT)-3' | (SEQ ID NO: 8) |
| FANA "BUT 5" | |
| 5'-araF(TTT TTT TTT XTT TTT TTT)-3' | (SEQ ID NO: 9) |
| FANA "BUT 10" | |
| 5'-araF(TTT TTT TTT TTT XTT TTT)-3' | (SEQ ID NO: 10) |
| FANA "BUT 13" | |

The X linkers are placed at various positions in order to determine whether optimal activity is dependent upon the location of the linker. X corresponds to acyclic residue IIb, where Y=Z= oxygen, and n= 4 (butanediol linker). It was also desirable to determine the precise pattern and rate of cleavage that accompanies the movement of the linker along the FANA backbone. The exact location of primary cuts is difficult to measure under ambient temperature for the homopolymers, which are already known to be good substrates for the enzyme. As it was of interest to see where the first cuts were occurring, this information was instead extracted from assays conducted at the lower temperature, under which enzyme activity is retarded just enough to enable a qualitative comparison on the preferred cleavage modes toward each substrate (FIGURE 5). At higher temperatures, the pattern becomes less interpretable as it results from the superimposition of multiple cleavages on a single target by the enzyme.

The target RNA used was rA₁₈, complementary to the above oligonucleotides. Assays (10 µL final volume) comprised 1 pmol of 5'-[³²P]-target RNA and 3 pmol of test oligonucleotide in 60 mM Tris-HCl (pH 7.8, containing 2 mM dithiothreitol, 60 mM KCl, and 10 mM MgCl₂. Reactions were started by the addition of RNase H and carried out at 14-15°C for 20 minutes. The result of such an experiment is shown in FIGURE 5.

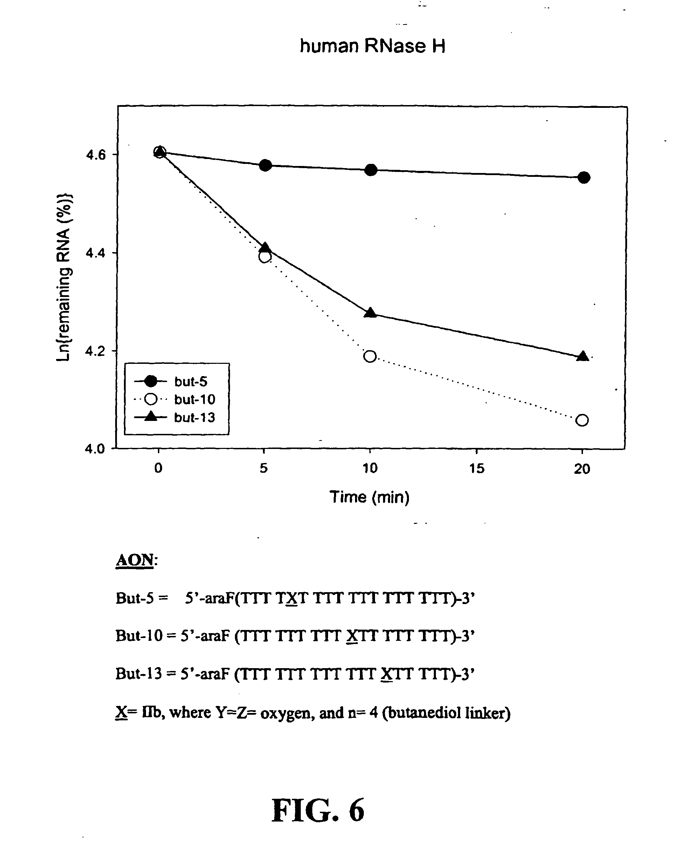

The relative rates follow the order: BUT-10 > BUT-13 > BUT-5 (TABLE 1 & FIGURE 6). Furthermore, all of the linker-containing oligonucleotides induce additional primary cuts at the 3'-end of the RNA except for FANA-BUT5, which coincidentally, is the only oligonucleotide superceded in rate by the FANA oligomer lacking the linker. As such, the FANA-BUT5 and FANA-BUT13 substrates show large differences in activation potency, in spite of the fact that their sequences are virtually identical and equally thermostable (TABLE 1), yet with opposite directionalities with respect to the butyl site in the oligonucleotide. Indeed, the different activities of these two oligomers suggest a minor - if not absent - role for the turnover effect. Alternatively, the diminished rate enhancement seen for FANA-BUT5 may reflect the remote positioning of RNase H along the substrate, which is known to bind near the 3'-end of the antisense oligonucleotide in the hybrid duplex and so may be unaffected by the linker insertion.

### EXAMPLE 6

### PDE-FANA versus PDE-[FANA-X-FANA] & PDE-[FANA-X-X-FANA] (X= seconucleotide IIc)

Defined-sequence oligonucleotides, 18-units in length, were used in these experiments:

| | |
|---|---|
| 5'-araF(TTT TTT TTT TTT TTT TTT)-3' | (SEQ ID NO: 7) |
| "FANA" | |
| 5'-araF(TTT TTT TTT XTT TTT TTT)-3' | (SEQ ID NO: 11) |
| "SEC×1" | |
| 5'-araF(TTT TTT TTX XTT TTT TTT)-3' | (SEQ ID NO: 12) |
| "SEC×2" | |

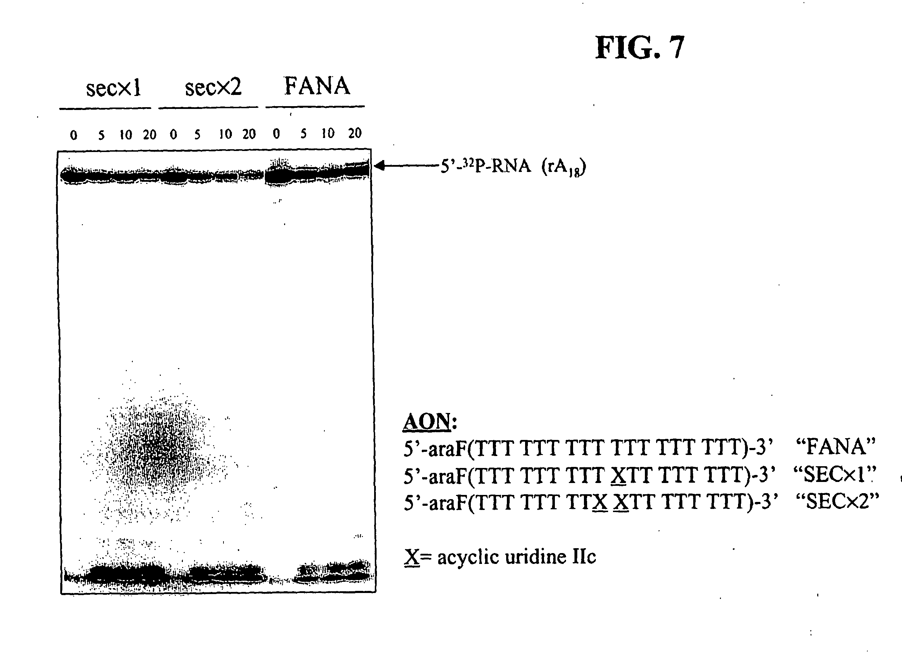

The residue X in the sequences above corresponds to acyclic residue IIc (secouridine). The target RNA used was octadecariboadenylate (rA₁₈) complementary to the sequence of the above oligonucleotides. The ability of the above oligonucleotides to elicit RNase H degradation of target RNA was determined in assays (10 µL final volume) that comprised 1 pmol of 5'-[³²P]- target RNA and 3 pmol of test oligonucleotide in 60 mM Tris-HCl containing 2mM dithiothreitol, 60 mM KCl, and 2.5 mM MgCl₂ (pH 7.8, -15°C). Prior to addition of the enzyme, the mixture was heated at 75 °C for 2 minutes, and then cooled slowly to room temperature to allow duplex formation. Reactions were started by the addition of human RNase HII at room temperature. Reactions were quenched by the addition of 10 µL of loading buffer (98% deionized formamide containing 10 mM EDTA and 1mg/mL each of bromophenol blue and xylene cyanol), and heating at 100°C for 5 minutes. The reaction products were resolved by electrophoresis using a 16% polyacrylamide sequencing gel containing 7 M urea, and visualized by autoradiography. The result of such an experiment is shown in FIGURE 7.

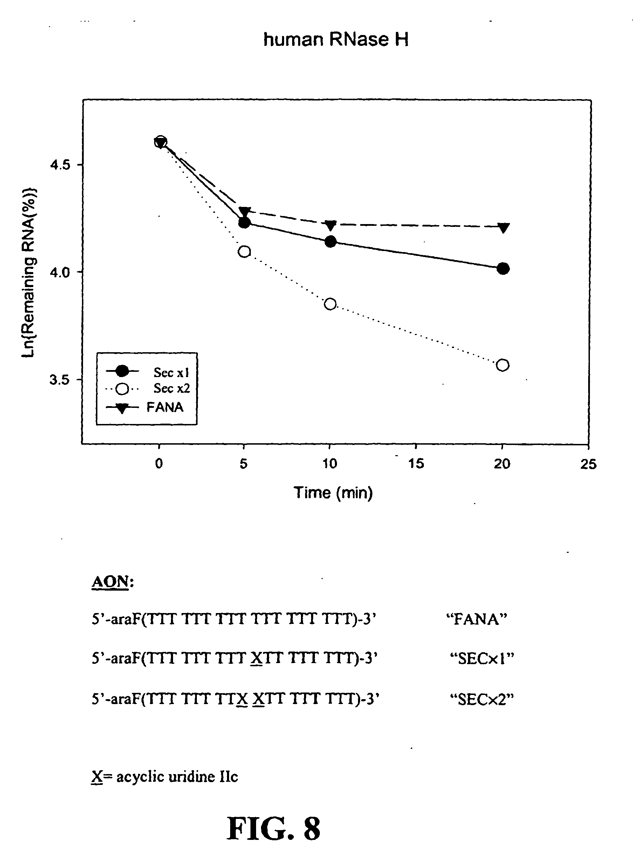

The results show that all "FANA", "SEC×1" and "SEC×2" are able to form duplexes with target RNA that serve as substrates for the activity of human RNase HII, as indicated by the disappearance (degradation) of the band corresponding to the full length target RNA (FIGURE 7). Quantitation of rA₁₈ remaining as a function of time indicates that the rate of RNA cleavage is greater when the hybridized AON is "SECx2" (FIGURE 8). The order observed is "FANA-SEC×2" > "FANA-SEC×1" > "FANA", demonstrating that an unprecedented enhancement in targeted RNA cleavage is imparted to the parent FANA strand by the seconucleotide linkers (IIc). As for the butanediol insertions (Example 5), the same trend is observed - i.e. reduced thermal stability relative to the all-FANA counterpart, yet enhanced RNase H activity. Thus, the drop in melting temperature caused by linker insertions is outweighed by the observed rate enhancement of the target RNA relative to the all-FANA constructs.

### EXAMPLE 7

### PDE-DNA versus PDE-[DNA-X-DNA] (X= butanediol linker = IIb, Y=Z= oxygen, and n= 4)

### A. Homopolymeric Sequences.

Defined-sequence oligonucleotides, 18-units in length, were used in these experiments:

| | |
|---|---|
| 5'-d(TTT TTT TTT TTT TTT TTT)-3' | (SEQ ID NO: 1) |
| "DNA" | |
| 5'-d(TTT TTT TTT XTT TTT TTT)-3' | (SEQ ID NO: 2) |
| "DNA-But" | |

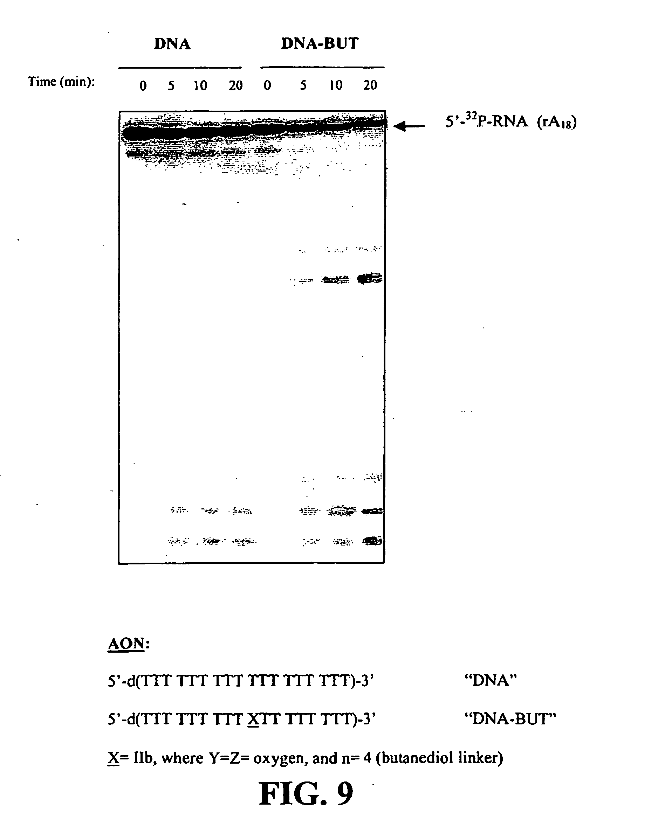

The residue X in the sequences above corresponds to acyclic residue IIb, where Y=Z= oxygen, n= 4 (or butanediol linker). The target RNA used was octadecariboadenylate (rA₁₈) complementary to the sequence of the above oligonucleotides. The ability of the above oligonucleotides to elicit RNase H degradation of target RNA was determined in assays (10 µL final volume) that comprised 1 pmol of 5'-[³²P]- target RNA and 3 pmol of test oligonucleotide in 60 mM Tris-HCl containing 2 mM dithiothreitol, 60 mM KCl, and 2.5 mM MgCl₂ (pH 7.8, 15°C). Assays were carried out as described above for Example 4A. The result of such an experiment is shown in FIGURE 9.

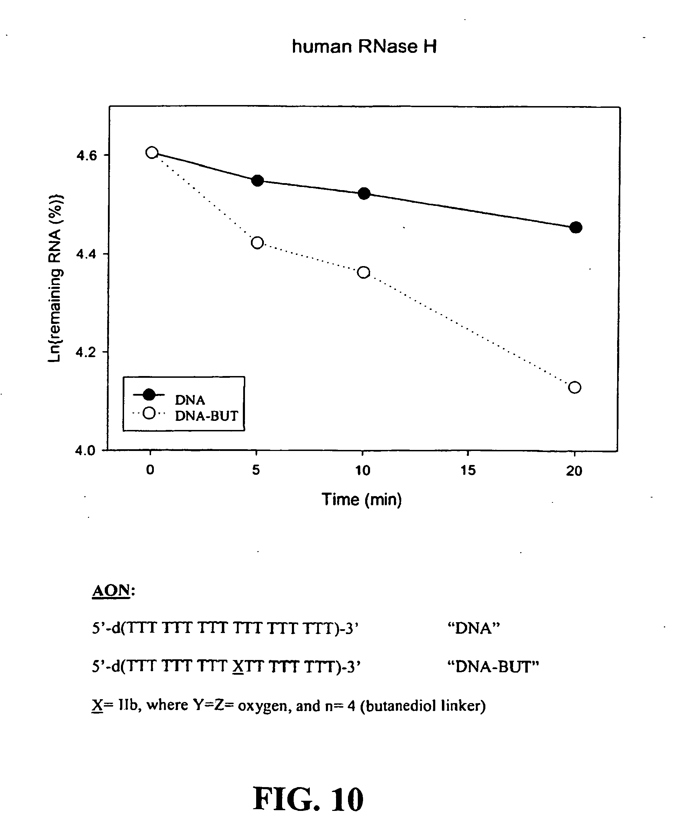

The results show that both "DNA" and "DNA-BUT" oligomers are able to form duplexes with target RNA that serve as substrates for the activity of human RNase HII, as indicated by the degradation products of the target RNA in FIGURE 9. Quantitation of rA₁₈ remaining as a function of time indicates that the rate of cleavage is significantly faster (*ca.* 3-fold) with "DNA-BUT" than with "DNA" (FIGURE 10).

### B. Mixed Base Sequence

Defined-sequence oligonucleotides, 18-units in length, were used in these experiments:

| | |
|---|---|
| 5'-d(TTA TAT TTT TTC TTT CCC)-3' | (SEQ ID NO: 3) |
| CAT "DNA" | |
| 5'-d(TTA TAT TTT XTC TTT CCC)-3' | (SEQ ID NO: 4) |
| CAT "DNA-But" | |
| 5'-d(TTA TAT TTT CTC TTT CCC)-3' | (SEQ ID NO: 5) |
| CAT "DNA-Mismatch" | |

The residue X in the sequences above corresponds to acyclic residue IIb, where Y=Z= oxygen, and n= 4 (or butanediol linker). The target RNA used was r(GGGAAAGAAAAAAUAUAA) (SEQ ID NO: 28), exactly complementary to the sequence of the first two DNA oligonucleotides. The third oligonucleotide, CAT "DNA-Mismatch" contains a dC mismatch at position 10. The ability of "DNA", "DNA-But", and "DNA-Mismatch" (CAT series) to elicit RNase H degradation of target RNA was determined in assays (10 µL final volume) that comprised 1 pmol of 5'-[³²P]- target RNA and 3 pmol of test oligonucleotide in 60 mM Tris-HCl containing 2 mM dithiothreitol, 60 mM KCl, and 2.5 mM MgCl₂ (pH 7.8, 15°C). Assays were carried out as described above for Example 4A.

The kinetic data given in TABLE 1 show that all DNA oligomers are able to form duplexes with target RNA that serve as substrates for the activity of human RNase HII. Quantitation of RNA target remaining as a function of time indicates that the rate of cleavage is significantly faster with "DNA-BUT" than with "DNA" or "DNA-Mismatch" (3 and 4-fold, respectively; TABLE 1).

### EXAMPLE 8

### Targeting higher molecular weight RNA. Comparison between Phosphodiester FANA, FANA-X-FANA, DNA, mismatched DNA, and DNA-X-DNA (X= butanediol linker = IIb, Y=Z= oxygen, and n= 4).

The following phosphodiester oligonucleotides, 18-units in length, were used in these experiments:
5'- d(ATT CCG TCA TCG CTC CTC)-3' (SEQ ID NO: 18) Ha-RAS "PDE-DNA"
5'- d(ATT CCG TCA XCG CTC CTC)-3' (SEQ ID NO: 19) Ha-RAS "PDE-DNA-But"
5'- d(ATT CCG TCA CCG CTC CTC)-3' (SEQ ID NO: 20) Ha-RAS "PDE-DNA-Mismatch"
5'-araF(ATT CCG TCA TCG CTC CTC)-3' (SEQ ID NO: 21) Ha-RAS "PDE-FANA"
5'-araF(ATT CCG TCA XCG CTC CTC)-3' (SEQ ID NO: 22) Ha-RAS "PDE-DNA-But"

The residue X in the sequences above corresponds to acyclic residue IIb, where Y=Z= oxygen, n= 4 (or butanediol linker). The target RNA used was a polyribonucleotide 40 nucleotide units in length. Their base sequences are derived from the naturally occurring Ha-Ras mRNA sequence (derived from the c-ras protooncogene). The ability of each of the above oligonucleotides to elicit RNase H degradation of target RNA was determined in assays (10 µL final volume) that comprised 1 pmol of 5'-[³²P]- target RNA and 3 pmol of test oligonucleotide in 60 mM Tris-HCl containing 2 mM dithiothreitol, 60 mM KCl, and 2.5 mM MgCl₂ (pH 7.8). Reactions were started by the addition of RNase H and carried out at 37°C. Timed aliquots were taken at various time intervals from each set of incubation.

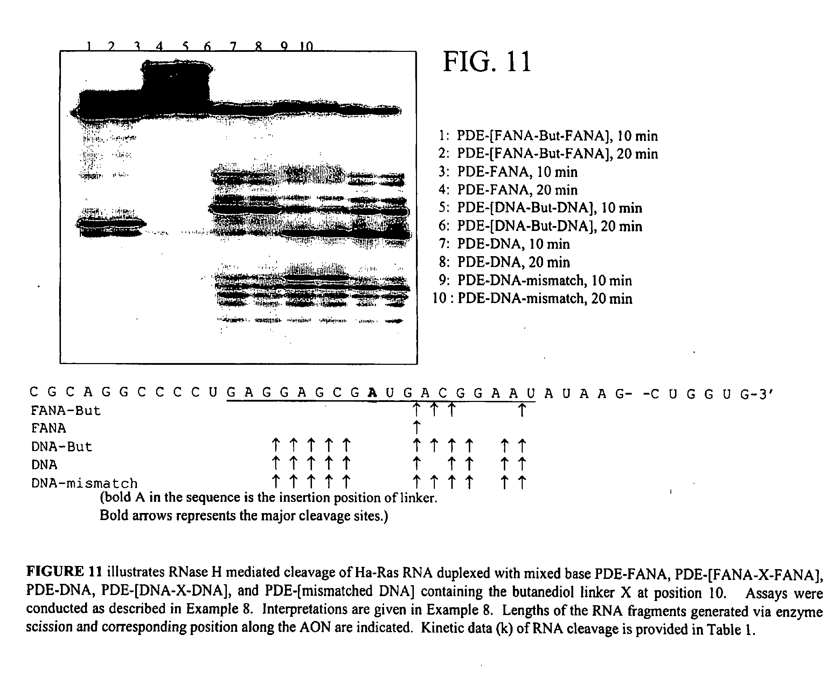

For this particular sequence, an increase in target degradation is not apparent upon interchanging a deoxynucleotide residue in DNA for a butanediol linker (oligomers XVIII and XIX, TABLE 1, and FIGURE 11). However, this is not the case for the FANA constructs. As demonstrated in all of the previous Examples, substitution of the arabinofluoronucleoside residue in phosphodiester FANA with a more flexible butanediol linker elevates the activity of RNase HII. In fact, such a substitution closes the efficiency gap between FANA (kᵣₑₗ 23.3) and DNA-derived (kᵣₑₗ 33.8) antisense compounds considerably (TABLE 1).

### EXAMPLE 9

### Targeting higher molecular weight RNA. Comparison between PS-FANA, PS-[FANA-X-FANA], PS-DNA, and PS-[DNA-X-DNA] (X= butanediol linker = IIb, Y= oxygen, Z=sulfur, and n= 4).

The following phosphorothioate oligonucleotides, 18-units in length, were used in these experiments:
5'- d(ATT CCG TCA TCG CTC CTC)-3' (SEQ ID NO: 23) Ha-RAS "PS-DNA"
5'- d(ATT CCG TCA XCG CTC CTC)-3' (SEQ ID NO: 24) Ha-RAS "PS-DNA-But"
5'-araF(ATT CCG TCA TCG CTC CTC)-3' (SEQ ID NO: 25) Ha-RAS "PS-FANA"
5'-araF(ATT CCG TCA XCG CTC CTC)-3' (SEQ ID NO: 26) Ha-RAS "PS-DNA-But"

The residue X in the sequences above corresponds to acyclic residue IIb, where Y= oxygen, Z= sulfur, n= 4 (butanediol linker). The target RNA used was a polyribonucleotide 40 nucleotide units in length. Their base sequences are derived from the naturally occurring Ha-Ras mRNA sequence (derived from the c-ras protooncogene). The ability of each of the above oligonucleotides to elicit RNase H degradation of target RNA was determined in assays (10 µL final volume) that comprised 1 pmol of 5'-[³²P]-target RNA and 3 pmol of test oligonucleotide in 60 mM Tris-HCl containing 2 mM dithiothreitol, 60 mM KCl, and 2.5 mM MgCl₂ (pH 7.8). Reactions were started by the addition of RNase H and carried out at 37°C. Timed aliquots were taken at various time intervals from each set of incubation.

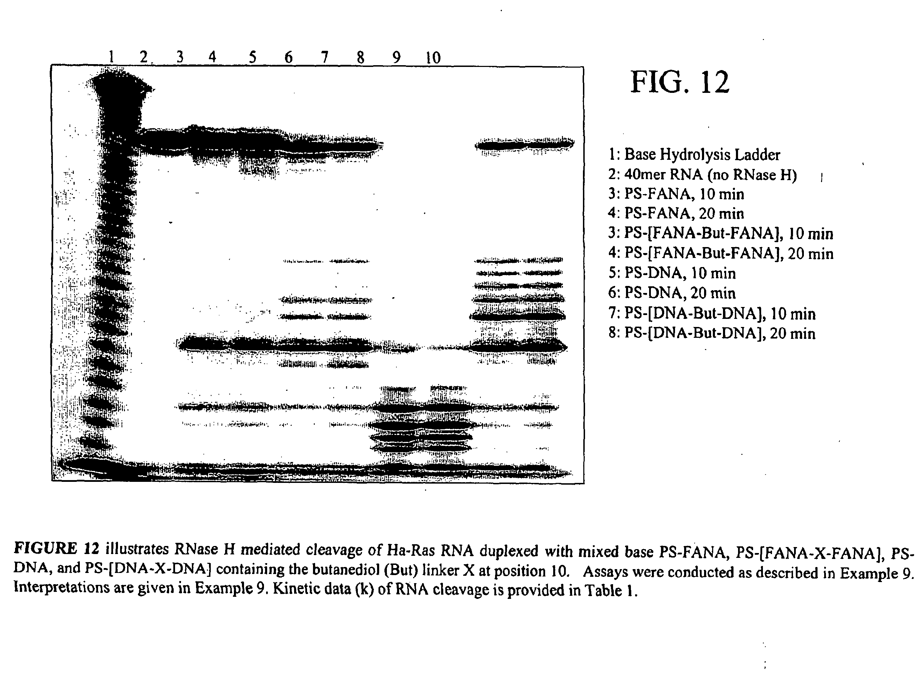

For this particular sequence, a decrease in target degradation is apparent upon interchanging a deoxynucleotide residue in DNA for a butanediol linker (TABLE 1, and FIGURE 12). This is in contrast to what is observed for the FANA based constructs. In this case, substitution of the arabinofluoronucleoside residue in PS-FANA with a more flexible butanediol linker elevates the activity of RNase HII (TABLE 1).

### EXAMPLE 10

### Oligonucleotide constructs containing 'looping out' acyclic linkers.

| | |
|---|---|
| 5'-araF(TTA TAT TTT TTC TTT CCC)-3' | (SEQ ID NO: 13) |
| CAT "FANA" | |
| 5'-araF(TTA TAT TTT XTC TTT CCC)-3' | (SEQ ID NO: 14) |
| CAT "FANA-But" | |
| 5'-araF(TTA TAT TTT X TTC TTT CCC)-3' | (SEQ ID NO: 17) |
| CAT "FANA-But-loop" | |

The residue X in the sequences above corresponds to acyclic residue IIb, where Y=Z= oxygen, n= 4 (or butanediol linker). The target RNA used was r(GGGAAAGAAAAAAUAUAA) (SEQ ID NO: 28), exactly complementary to each of the above sequences. The ability of phosphodiester linked "FANA", "FANA-But", and "FANA-But-loop" (CAT series) to elicit RNase H degradation of target RNA was determined in assays (10 µL final volume) that comprised 1 pmol of 5'-[³²P]- target RNA and 3 pmol of test oligonucleotide in 60 mM Tris-HCl containing 2 mM dithiothreitol, 60 mM KCl, and 2.5 mM MgCl₂ (pH 7.8, 15°C). Assays were carried out as described above for Example 4A.

The "FANA-But-loop" sequence contains unifying elements of both the "FANA" and "FANA-But" oligonucleotides. These consist of a localized flexible site in the center of the sequence (similar to "FANA-But") as well as the ability of this oligonucleotide to fully hybridize with the target RNA (similar to "FANA"). As a result, the looping linker likely extends away from the duplex core to maximize the number of residues that form base pairs between this particular sequence and the RNA. Forcing the linker out of the helix in this way may disrupt some of the interactions between RNase H and the two strands by reducing the number of stable contacts between the enzyme and the duplex minor groove. Surprisingly, this sequence still considerably enhances RNA degradation (compare oligomers XIII and XVII, TABLE 1), which suggests that flexibility in the antisense strand is important for effective RNase H induction, irrespective of whether the flexible linker resides directly within or away from the helix axis.

### Abbreviations

ANA, arabinonucleic acid derivative (with a variable 2'-substituent)
AON, antisense oligonucleotide
BUT, 1,4-butanediol unit
DMSO, dimethylsulfoxide
DNA, deoxyribonucleic acid
EC₅₀, effective concentration
EDTA, ethylenediaminetetraacetate
Et₂O, diethyl ether
EtOAc, ethyl acetate
FAB-MS, fast-atom bombardment mass spectrometry
FANA, 2'-deoxy-2'-fluoroarabinonucleic acid
HPLC, high performance liquid chromatography
LCAA-CPG, long-chain alkylamine controlled pore glass
MBO, mixed-backbone oligonucleotide
MeOH, methanol
NBA, *p*-nitrobenzyl alcohol O-PNA monomer, NH₂-CH(CH₂-CH₂-Base)-CH₂-O-CH₂-CO₂H
PDE-DNA, phosphodiester linked DNA
PNA, peptide nucleic acid -
PNA monomer, N-(2-aminoethyl)glycine unit in which an heterocyclic base is attached via a methylene carbonyl linker.
PS-DNA, phosphorothioate linked DNA
R_{f}, retention factor
RNA, ribonucleic acid
RNase H, ribonuclease H
SEC, seconucleotide unit
TEA, triethylamine
THF, tetrahydrofuran
*T*_{*m*}*,* melting temperature
TLC, thin-layer chromatography
Tol, toluene

## Claims

1. An oligonucleotide having the structure:
[R¹-X]ₐ-R² **Ia**
wherein a is an integer greater than or equal to 1;
wherein either R¹, R² each independently comprise at least one nucleotide;
wherein X is an acyclic linker; and
wherein said oligonucleotide comprises at least one of (i) at least one modified nucleotide and (ii) a chimera; and
wherein the modified nucleotide is selected from the group consisting of ANA, PS-ANA, and the chimera is selected from the group contisting of PS-[ANA-DNA] and PS-[DNA-ANA] chimeras; and wherein the ANA is FANA.

2. The oligonucleotide of claim 1 wherein the FANA is selected from the group consisting of POE-FANA and PS-FANA.

3. The oligonucleotide of claim 1, wherein the acyclic linker is selected from the group consisting of an acyclic nucleoside and a non-nucleotidic linker.

4. The oligonucleotide of claim 3, wherein the acyclic nucleoside is selected from the group consisting of purine and pyrimidine seconucleosides.

5. The oligonucleotide of claim 4 wherein the purine seconucleoside is selected from the group consisting of secoadenosine and secoguanosine.

6. The oligonucleotide of claim 4 wherein the pyrimidine seconucleoside is selected from the group consisting of secothymidine, secocytidine and secouridine.

7. The oligonucleotide of claim 1, wherein the non-nucleotidic linker comprises a linker selected from the group consisting of an amino acid and an amino acid derivative.

8. The oligonucleotide of claim 7, wherein the amino acid derivative is selected from the group consisting of (a) an N-(2-aminoethyl)glycine unit in which an heterocyclic base is attached via a methylene carbonyl linker (PNA monomer); and (b) an O-PNA unit.

9. The oligonucleotide of claim 1, wherein said oligonucleotide has the structure: 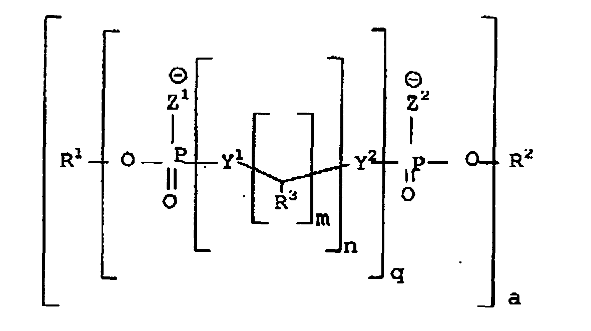 wherein each of m, n, q and a are independently integers greater than or equal to 1;
wherein each of R¹ and R² are independently at least one nucleotide;
wherein each of Z¹ and Z² are independently selected from the group consisting of an oxygen atom, a sulfur atom, an amino group and an alkylamino group;
wherein each of Y¹ and Y² are independently selected from the group consisting of oxygen, sulfur and NH; and
wherein R³ is selected from the group consisting of H, alkyl, hydroxyalkyl, alkoxy, a purine, a pyrimidine and combinations thereof.

10. The oligonucleotide of claim 9, wherein said purine is selected from the group consisting of adenine, guanine, and derivatives thereof.

11. The oligonucleotide of claim 9, wherein said pyrimidine is selected from the group consisting of thymine, cytosine, 5-methylcytosine, uracil, and derivatives thereof.

12. The oligonucleotide of claim 1, wherein each of R¹ and R² independently comprise at least two nucleotides having an internucleotide linkage, wherein said internucleotide linkage is selected from the group consisting of phosphodiester, phosphotriester, phosphorothioate, methylphosphonate, phosphoramidate (5'N-3'P and 5'P-3'N), and combinations thereof.

13. The oligonucleotide of claim 9, wherein each of R¹ and R² independently comprise FANA.

14. The oligonucleotide of claim 9, wherein said oligonucleotide is selected from the group consisting of: 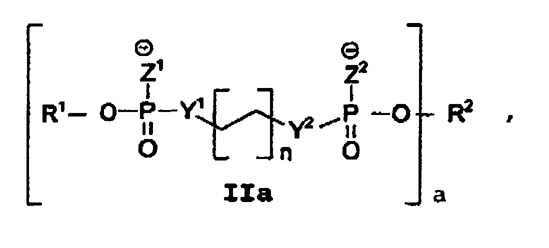 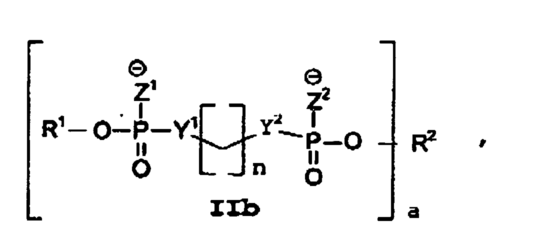 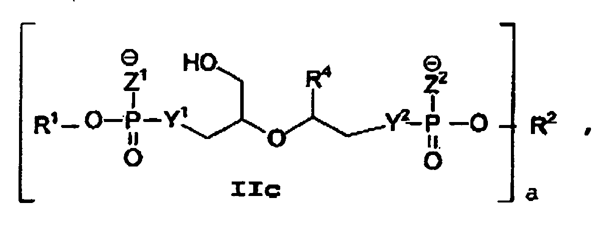 and 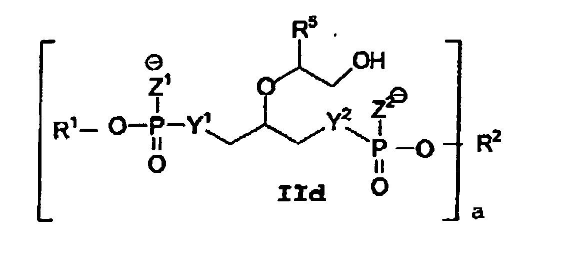 wherein n, a, R¹, R², Z¹, Z², Y¹ and Y² are as defined in claim 9; and
wherein each of R⁴ and R⁵ are independently selected from the group consisting of a purine and a pyrimidine.

15. The oligonucleotide of claim 14, wherein said purine is selected from the group consisting of adenine, guanine and derivatives thereof.

16. The oligonucleotide of claim 14, wherein said pyrimidine is selected from the group consisting of thymine, cytosine, uracil, and derivatives thereof.

17. The oligonucleotide of claim 1; wherein R¹ and R² are FANA; and wherein a=1.

18. The oligonucleotide of claim 1; wherein R¹ is [FANA-DNA]; wherein R² is [DNA-FANA]; and wherein a=1.

19. The oligonucleotide of claim 1; wherein R¹ is [FANA-DNA]; wherein R² is FANA; and wherein a=1.

20. The oligonucleotide of claim 1; wherein R¹ is FANA; wherein R² is [DNA-FANA]; and wherein a=1.

21. The oligonucleotide of claim 14; wherein R¹ is FANA; wherein R² is PS-FANA; wherein a=1; and wherein said oligonucleotide has structure IIb in which Y¹, Y², Z¹ and Z² are oxygen and n=4.

22. The oligonucleotide of claim 14; wherein R¹ is PS-FANA; wherein R² is FANA; wherein a=1; and wherein said oligonucleotide has structure IIb in which Y¹, Y², Z² are oxygen, and Z¹ are sulfur and n=4.

23. The oligonucleotide of claim 14; wherein R¹ is PS-FANA; wherein R² is FANA; wherein a=1; and wherein said oligonucleotide has structure IIc.

24. The oligonucleotide of claim 14; wherein R¹ is FANA; wherein R² is PS-FANA; wherein a=1; and wherein said oligonucleotide has structure IIc.

25. The oligonucleotide of claim 1, wherein a=2 and each of R¹ and R² independently consist of at least 3 nucleotides.

26. The oligonucleotide of claim 25, wherein each of R¹ and R² independently consist of 3-8 nucleotides.

27. The oligonucleotide of claim 1, wherein a=3 and each of R¹ and R² independently consist of at least 2 nucleotides.

28. The oligonucleotide of claim 27, wherein each of R¹ and R² independently consist of 2-6 nucleotides.

29. The oligonucleotide of any one of claim 28, wherein said oligonucleotide is antisense to a target RNA.

30. An in vitro method of preventing or decreasing translation, reverse transcription and/or replication of a target RNA in a system, said method comprising contacting said target RNA with the oligonucleotide of claim 29.

31. An in vitro method of preventing or decreasing translation, reverse transcription and/or replication of a target RNA in a system, said method comprising:
a) contacting said target RNA with the oligonucleotide of claim 29; and
b) allowing RNase cleavage of said target RNA.

32. In intro use of the oligonucleotide according to claim 29 for preventing or decreasing translation, reverse transcription and/or replication of a target RNA in a system.

33. A commercial package comprising the oligonucleotide according to claim 29 together with instructions for its use for preventing or decreasing translation, reverse transcription and/or replication of a target RNA in a system.

34. The oligonucleotide of claim 29 for use as antisense therapeutic.

35. A method for increasing RNase H induction activity of an oligonucleotide comprising incorporating an acyclic linker into said oligonucleotide to produce an oligonucleotide having the structure:
[R¹-X]ₐ-R² **Ia**
wherein a is an integer greater than or equal to 1;
wherein either R¹, R² each independently comprise at least one nucleotide; wherein X is an acyclic linker; and wherein said oligonucleotide comprises at least one of (i) at least one modified nucleotide and (ii) a chimera; and
wherein the modified nucleotide is selected from the group consisting of ANA, PS-ANA, RNA, PS-RNA, PDE-or PS-RNA analogues, locked nucleic acids (LNA), phosphorodiamidate morpholino nucleic acids, N3'-P5'phosphoramidate DNA, cyclohexene nucleic acid, alpha-L-LNA, boranophosphate DNA, methylphosphonate DNA, and combinations thereof, and the chimera is selected from the group consisting of RNA-DNA and DNA-RNA chimeras, PS-[RNA-DNA] and PS-[DNA-RNA] chimeras, PS-[ANA-DNA] and PS~[DNA-ANA] chimeras.

36. The oligonucleotide of claim 35 wherein the ANA is FANA.

37. The oligonucleotide of claim 36 wherein the FANA is selected from the group consisting of PDE-FANA and PS-FANA.

38. The oligonucleotide of claim 35, wherein the PDE-or PS-RNA analogues are selected from the group consisting of 2'-modified RNA wherein the 2'-substituent is selected from the group consisting of alkyl, alkoxy, alkylalkoxy, F and combinations thereof.

39. The method of claim 35, wherein the acyclic linker is selected from the group consisting of an acyclic nucleoside and a non-nucleotidic linker.

40. The method of claim 39, wherein the acyclic nucleoside is selected from the group consisting of purine and pyrimidine seconucleosides.

41. The method of claim 40 wherein the purine seconucleoside is selected from the group consisting of secoadenosine and secoguanosine.

42. The method of claim 41 wherein the pyrimidine seconucleoside is selected from the group consisting of secothymidine, secocytidine and secouridine.

43. The method of claim 35, wherein the non-nucleotidic linker comprises a linker selected from the group consisting of an amino acid and an amino acid derivative.

44. The method of claim 43, wherein the amino acid derivative is selected from the group consisting of (a) an N-(2-aminoethyl)glycine unit in which an heterocyclic base is attached via a methylene carbonyl linker (PNA monomer); and (b) an O-PNA unit.

45. The method of claim 35, wherein said oligonucleotide has the structure: 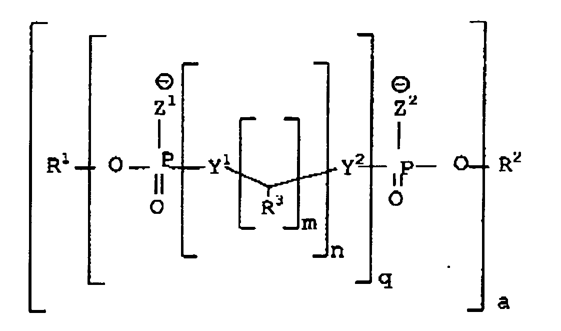 wherein each of m, n, q and a are independently integers greater than or equal to 1;
wherein each of R¹ and R² are independently at least one nucleotide;
wherein each of Z¹ and Z² are independently selected from the group consisting of an oxygen atom, a sulfur atom, an amino group and an alkylamino group;
wherein each of Y¹ and Y² are independently selected from the group consisting of oxygen, sulfur and NH; and
wherein R³ is selected from the group consisting of H, alkyl, hydroxyalkyl, alkoxy, a purine, a pyrimidine and combinations thereof.

46. The method of claim 45, wherein said purine is selected from the group consisting of adenine, guanine, and derivatives thereof.

47. The method of claim 45, wherein said pyrimidine is selected from the group consisting of thymine, cytosine, 5-methylcytosine, uracil, and derivatives thereof.

48. The method of claim 35, wherein each of R¹ and R² independently comprise at least two nucleotides having an internucleotide linkage, wherein said internucleotide linkage is selected from the group consisting of phosphodiester, phosphotriester, phosphorothioate, methylphosphonate, phosphoramidate (5'N-3'P and 5'P-3'N), and combinations thereof.

49. The method of claim 45, wherein each of R¹ and R² independently comprise ANA.

50. The method of claim 49, wherein said ANA comprises a 2'-substituent selected from the group consisting of fluorine, hydroxyl, amino, azido, alkyl, alkenyl, alkynyl, and alkoxy groups.

51. The method of claim 50, wherein said 2'-substituent is fluorine and said ANA is FANA.

52. The method of claim 50, wherein said alkyl group is selected from the group consisting of methyl, ethyl, propyl and butyl groups.

53. The method of claim 50, wherein said alkoxy group is selected from the group consisting of methoxy, ethoxy, propoxy, and methoxyethoxy groups.

54. The method of claim 45, wherein said oligonucleotide is selected from the group consisting of: 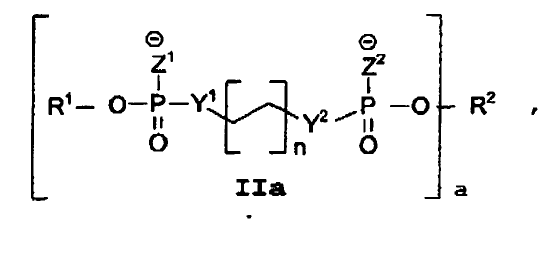 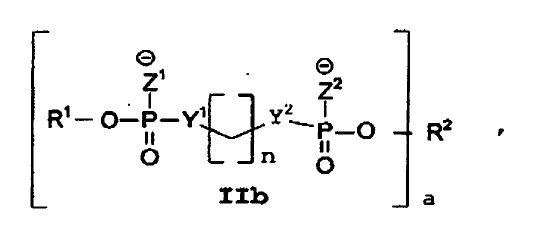 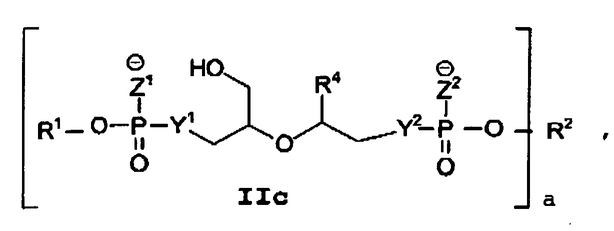 and 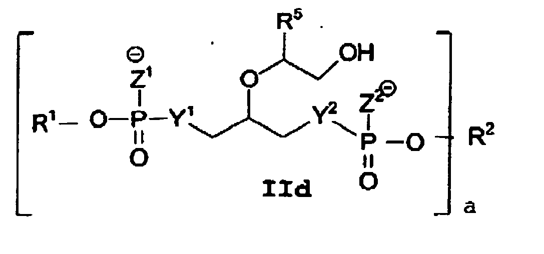 wherein n, a, R¹, R², Z¹, Z², Y¹ and Y² are as defined in claim 45; and
wherein each of R⁴ and R⁵ are independently selected from the group consisting of a purine and a pyrimidine.

55. The method of claim 54, wherein said purine is selected from the group consisting of adenine, guanine and derivatives thereof.

56. The method of claim 54, wherein said pyrimidine is selected from the group consisting of thymine, cytosine, uracil, and derivatives thereof.

57. The method of claim 35; wherein R¹ and R² are FANA; and wherein a=1.

58. The method of claim 35; wherein R¹ is [FANA-DNA]; wherein R² is [DNA-FANA]; and wherein a=1.

59. The method of claim 35; wherein R¹ is [FANA-DNA]; wherein R² is FANA; and wherein a=1.

60. The method of claim 35; wherein R¹ is FANA; wherein R² is [DNA-FANA]; and wherein a=1.

61. The method of claim 35; wherein R¹ is [RNA-DNA]; wherein R² is [DNA-RNA]; and wherein a=1.

62. The method of claim 35; wherein R¹ is [RNA-DNA] ; wherein R² is RNA; and wherein a=1.

63. The method of claim 35; wherein R¹ is RNA; wherein R² is [DNA-RNA]; and wherein a=1.

64. The method of claim 35; wherein R¹ is S-[(2'O-alkyl)RNA-DNA]; wherein R² is S-[DNA-(2'O-alkyl)RNA]; and wherein a=1.

65. The method of claim 35; wherein R¹ is S-[(2'O-alkyl)RNA-DNA]; wherein R² is S-[(2'O-alkyl)RNA]: and wherein a=1.

66. The method of claim 35; wherein R¹ is S-[(2'0-alkyl)RNA]: wherein R² is S-[DNA-(2'O-alkyl)RNA]; and wherein a=1.

67. The method of claim 35; wherein R¹ is S-[(2'O-alkoxyalkyl)RNA-DNA]; wherein R² is S-[DNA-(2'O-alkoxyalkyl)RNA]; and wherein a=1.

68. The method of claim 35; wherein R¹ is S-[(2'O-alkoxyalkyl)RNA-DNA]; wherein R² is S-[(2'O-alkoxyalkyl)RNA]; and wherein a=1.

69. The method of claim 35; wherein R¹ is S-[(2'O-alkoxyalkyl)RNA]; wherein R² is S-[DNA-(2'O-alkoxyalkyl)RNA]; and wherein a=1.

70. The method of claim 54; wherein R¹ is FANA; wherein R² is PS-FANA; wherein a=1; and wherein said oligonucleotide has structure IIb in which Y¹, Y², Z¹ and Z² are oxygen and n=4.

71. The method of claim 54; wherein R¹ is PS-FANA; wherein R² is FANA; wherein a=1; and wherein said oligonucleotide has structure IIb in which Y¹, Y², Z² are oxygen, and Z¹ are sulfur and n=4.

72. The method of claim 54; wherein R¹ is PS-FANA;
wherein R² is FANA; wherein a=1; and wherein said oligonucleotide has structure IIc.

73. The method of claim 54; wherein R¹ is FANA; wherein R² is PS-FANA; wherein a=1; and wherein said oligonucleotide has structure IIc.

74. The method of claim 35, wherein a=2 and each of R¹ and R² independently consist of at least 3 nucleotides.

75. The method of claim 74, wherein each of R¹ and R² independently consist of 3-8 nucleotides.

76. The method of claim 35, wherein a-3 and each of R¹ and R² independently consist of at least 2 nucleotides.

77. The method of claim 76, wherein each of R¹ and R² independently consist of 2-6 nucleotides.

78. The method of claim 35, wherein said oligonucleotide is antisense to a target RNA.

## Patentansprüche

1. Oligonucleotid der Struktur:
[R¹-X]ₐ-R² **Ia**
wobei a eine ganze Zahl größer oder gleich 1 ist;
R¹ oder R² unabhängig voneinander je wenigstens ein Nucleotid umfassen;
X ein acyclischer Linker ist; und
das Oligonucleotid wenigstens eines umfasst aus (i) wenigstens einem modifizierten Nucleotid und (ii) einer Chimäre; und
worin das modifizierte Nucleotid ausgewählt ist aus der Gruppe bestehend aus ANA, PS-ANA, und die Chimäre ausgewählt ist aus der Gruppe bestehend aus PS-[ANA-DNA] und PS-[DNA-ANA] Chimären; und wobei ANA ist FANA.

2. Oligonucleotid gemäß Anspruch 1, wobei FANA ausgewählt ist aus der Gruppe bestehend aus PDE-FANA und PS-FANA.

3. Oligonucleotid gemäß Anspruch 1, wobei der acyclische Linker ausgewählt ist aus der Gruppe bestehend aus einem acyclischen Nucleosid und einem nicht-nueleotidischen Linker.

4. Oligonucleotid gemäß Anspruch 3, wobei das acyclische Nucleosid ausgewählt ist aus der Gruppe bestehend aus Purin- und Pyrimidin-Seconucleosiden.

5. Oligonucleotid gemäß Anspruch 4, wobei das Purinseconucleosid ausgewählt ist aus der Gruppe bestehend aus Secoadenosine und Secoguanosin.

6. OligonLicleotid nach Anspruch 4, wobei das Pyrimidinseconucleosid ausgewählt ist aus der Gruppe bestehend aus Secothymidin, Secocytidin und Secouridin.

7. Oligonucleotid nach Anspruch 1, wobei der nicht-nucleotidische Linker einen Linker umfasst, der ausgewählt ist aus der Gruppe bestehend aus einer Aminosäure und einem Aminosäurederivat.

8. Oligonucleotid nach Anspruch 7, wobei das Aminosäurederivat ausgewählt ist aus der Gruppe bestehend aus (a) einem N-(2-Aminoethyl)-Glycineinheit in der eine heterocyclische Base über einen Methylencarbonyl-Linker (PNA-Monomer) gebunden ist; und (b) einer O-PNA-Einheit.

9. Oligonucleotid nach Anspruch 1, wobei das Oligonucleotid die folgende Struktur aufweist: 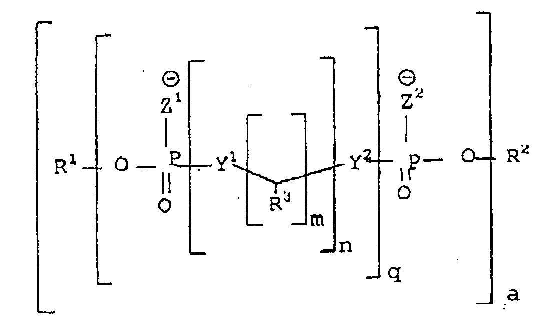 , wobei m, n, q und a jeweils und unabhängig ganze Zahlen, größer oder gleich 1 sind;
wobei R¹ und R² je unabhängig voneinander wenigstens ein Nucleotid sind;
wobei Z¹ und Z² unabhängig voneinander je ausgewählt sind aus der Gruppe bestehend aus einem Sauerstoffatom, einem Schwefelatom, einer Aminogruppe und einer Alkylaminogruppe;
wobei Y¹ und Y² je unabhängig ausgewählt sind aus der Gruppe bestehend aus Sauerstoff, Schwefel und NH; und
wobei R³ ausgewählt ist aus der Gruppe bestehend aus H, Alkyl, Hydroxyalkyl, Alkoxy, einem Purin, einem Pyrimidin und Kombinationen davon.

10. Oligonucleotid gemäß Anspruch 9, wobei das Purin ausgewählt ist aus der Gruppe bestehend aus Adenin, Guanin und Derivaten davon.

11. Oligonucleotid nach Anspruch 9, wobei das Pyrimidin ausgewählt ist aus der Gruppe bestehend aus Thymin, Cytosin, 5-Methylcytosin, Uracil und Derivaten davon.

12. Oligonucleotid gemäß Anspruch 1, wobei R¹ und R² je unabhängig voneinander wenigstens zwei Nucleotide umfassen, die eine Internucleotidbindung aufweisen, wobei die Internucleotid-Bindung ausgewählt ist aus der Gruppe bestehend aus Phosphodiester, Phosphotriester, Phosphorothioat, iVlethylphosphonat, Phosphoramidat (5'N-3'P und 5'P-3'N) und Kombinationen davon.

13. Oligonucleotid nach Anspruch 9, wobei R¹ und R² je unabhängig voneinander FANA umfassen.

14. Oligonucleotid gemäß Anspruch 9, wobei das Oligonucleotid ausgewählt ist aus der Gruppe bestehend aus: 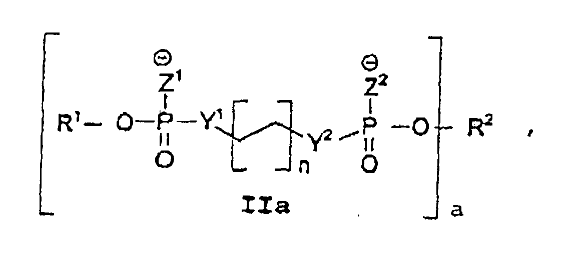 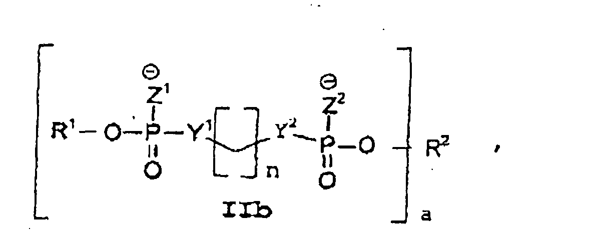 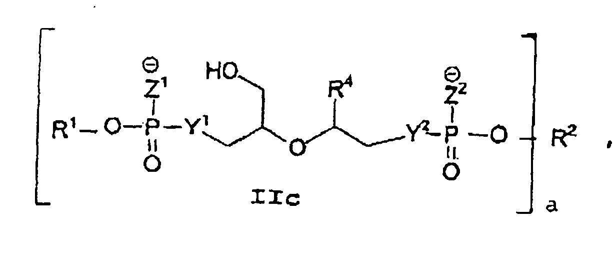 und 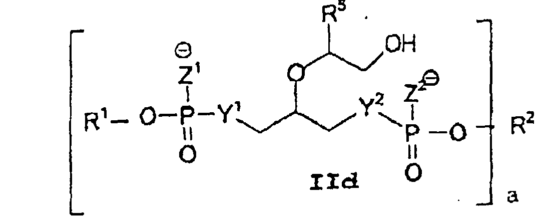 wobei n, a, R¹, R², Z¹, Z², Y¹ und Y² definiert sind wie in Anspruch 9 und
wobei R⁴ und R⁵ je unabhängig voneinander ausgewählt sind aus der Gruppe bestehend aus Purin und Pyrimidin.

15. Oligonucleotid nach Anspruch 14, wobei das Purin ausgewählt ist aus der Gruppe bestehend aus Adenin, Guanin und Derivaten davon.

16. Oligonucleotid nach Anspruch 14, wobei das Pyrimidin ausgewählt ist aus der Gruppe bestehend aus Thymin, Cytosin, Uracil und Derivaten davon.

17. Oligonucleotid nach Anspruch 1, wobei R¹ und R² FANA sind und wobei a = 1 ist.

18. Oligonucleotid nach Anspruch 1, wobei R¹ [FANA-DNA] ist, wobei R² [DNA-FANA] ist und wobei a = 1 ist.

19. Oligonucleotid nach Anspruch 1, wobei R¹ [FANA-DNA] ist, R² FANA ist und wobei a = 1 ist.

20. Oligonucleotid nach Anspruch 1, wobei R¹ FANA ist, wobei R² [DNA-FANA] ist und wobei a = 1 ist.

21. Oligonucleotid nach Anspruch 14, wobei R¹ FANA ist; wobei R² PS-FANA ist, wobei a = 1 ist und wobei das Oligonucleotid die Struktur II b aufweist, in der Y¹, Y², Z¹ und Z² Sauerstoff sind und n = 4 ist.

22. Oligonucleotid gemäß Anspruch 14, wobei R¹ PS-FANA ist; wobei R² FANA ist, wobei a = 1 ist und wobei das Oligonucleotid die Struktur II b aufweist, in der Y¹, Y², Z² Sauerstoff und Z¹ Schwefel sind und n = 4 ist.

23. Oligonucleotid gemäß Anspruch 14, wobei R¹ PS-FANA ist; wobei R² FANA ist, wobei a = 1 ist und wobei das Oligonucleotid die Struktur II c aufweist.

24. Oligonucleotid gemäß Anspruch 14, wobei R¹ FANA ist, wobei R² PS-FANA ist; wobei a = 1 ist und wobei das Oligonucleotid die Struktur IIc aufweist.

25. Oligonucleotid gemäß Anspruch 1, wobei a = 2 ist und R¹ und R² je unabhängig voneinander aus mindestens drei Nucleotiden bestehen.

26. Oligonucleotid gemäß Anspruch 25, wobei R¹ und R² je unabhängig voneinander aus 3 bis 8 Nucleotiden bestehen.

27. Oligonucleotid gemäß Anspruch 1, wobei a = 3 ist, und R¹ und R² je unabhängig voneinander aus wenigstens zwei Nucleotiden bestehen.

28. Oligonucleotid gemäß Anspruch 27, wobei R¹ und R² je unabhängig voneinander aus 2 bis 6 Nucleotiden bestehen.

29. Oligonucleotid gemäß einem der Anspruch 1 bis 28, wobei das Oligonucleotid antisense zu einer Target-RNA ist.

30. In-vitro-Verfahren zur Verhinderung oder Verminderung der Translation, Reverse Transkription oder Replikation einer Target-RNA in einem System, umfassend das In-Kontakt-Bringen der Target-RNA mit dem Oligonucleotid gemäß Anspruch 29.

31. In-vitro-Verfahren zur Verhinderung oder Verminderung der Translation, Reverse Transkription und/oder Replikation einer Target-RNA in einem System, umfassend:
a) Inverbindungbringen der Target RNA mit dem Oligonucleotid nach Anspruch 29; und
b) Geschehenlassen der RNA-Spaltung der Target RNA.

32. In-vitro-Verwendung eines Oligonucleotids gemäß Anspruch 29 zur Verhinderung oder Vermeindung der Translation, Reverse Transkription und/oder Replikation einer Target-RNA in einem System.

33. Verkaufsfähige Zusammenstellung umfassend das Oligonucleotid gemäß Anspruch 29 zusammen mit Gebrauchsanweisungen zur Verhinderung oder Verminderung der Translation, Reverse Transkription und/oder Replication einer Target-RNA in einem System

34. Das Oligonucleotid gemäß Anspruch 29 zur Verwendung als antisense-Medikament.

35. Verfahren zur Steigerung der RNase H-Induktionsaktivität eines Oligonukleotids umfassend Einbau eines acyclischen Linkers in das Oligonucleotid, wodurch ein Oligonucleotid erhalten wird der Struktur:
[R¹-X]ₐ-R² **Ia**
wobei a eine ganze Zahl größer oder gleich 1 ist;
wobei R¹ oder R² unabhängig voneinander wenigstens 1 Nucleotid umfassen; wobei X ein acyclischer Linker ist; und das Oligonucleotid wenigstens eines umfasst aus (i) wenigstens einem modifizierten Nucleotid und (ii) einer Chimäre; und
worin das modifizierte Nucleotid ausgewählt ist aus der Gruppe bestehend aus ANA, PS-ANA, RNA, PS-RNA, PDE- oder PS-RNA-Analoga, locked Nucleinsäuren (LNA), Phosphorodiamiciatmorpholinonucleinsäuren, N3'-P5'-Phosphoroamidat DNA, Cyclohexennukleinsäuren, alpha-L-LNA, Boranophosphat-DNA, Methylphosphonat-DNA und Kombinationen daraus, und wobei die Chimäre ausgewählt ist aus der Gruppe bestehend aus RNA-DNA- und DNA-RNA-Chimären, PS-[RNA-DNA]- und PS-[DNA-RNA]- Chimären, PS-[ANA-RNA]- und PS-[DNA-ANA]-Chimären.

36. Oligonucleotid gemäß Anspruch 35, worin ANA ist FANA.

37. Oligonucleotid gemäß Anspruch 36, wobei FANA ausgewählt ist aus der Gruppe bestehend aus PDE-FANA und PS-FANA.

38. Oligonucleotid gemäß Anspruch 35, wobei die PDE- oder PS-RNA Analoga ausgewählt sind aus der Gruppe bestehend aus 2'-modifizierter RNA, wobei der 2'-Substituent ausgewählt ist aus der Gruppe bestehend aus Alkyl, Alkoxy, Alkylalkoxy, F und Kombinationen daraus.

39. Verfahren gemäß Anspruch 35, wobei der acyclische Linker ausgewählt ist aus der Gruppe bestehend aus einem acyclischen Nucleosid- und einem nicht-nucleotidischen Linker.

40. Verfahren gemäß Anspruch 39, wobei das acyclische Nucleosid ausgewählt ist aus der Gruppe bestehend aus Purin und Pyrimidinseconucleodide.

41. Verfahren gemäß Anspruch 40, wobei das Purinseconucleosid ausgewählt ist aus der Gruppe bestehend aus Secoadenosin und Secoguanosin.

42. Verfahren gemäß Anspruch 41, wobei das Pyrimidinseconucleosid ausgewählt ist aus der Gruppe bestehend aus Secothymidine, Secocytidine und Secouridine.

43. Verfahren gemäß Anspruch 35, wobei der Nicht-nucleotidische Linker einen Linker umfasst, ausgewählt aus der Gruppe bestehend aus einer Aminosäure und einem Aminosäurederivat.

44. Verfahren gemäß Anspruch 43, wobei das Aminosäurederivat ausgewählt ist aus der Gruppe bestehend aus (a) einer N-(2-Aminoethyl)glycineinheit, in der eine heterocyclische Base über einen Methylencarbonyllinker (PNA Monomer) angebunden ist; und (b) einer O-PNA-Einheit.

45. Verfahren nach Anspruch 35, wobei das Oligonucleotid die folgende Struktur aufweist: 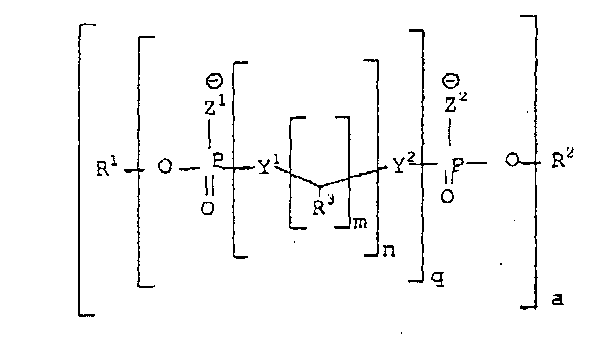 , wobei m, n, q und a jeweils und unabhängig ganze Zahlen sind, größer oder gleich 1;
wobei R¹ und R² je unabhängig voneinander wenigstens ein Nucleotid sind;
wobei Z¹ und Z² unabhängig voneinander je ausgewählt sind aus der Gruppe bestehend aus einem Sauerstoffatom, einem Schwefelatom, einer Aminogruppe und einer Alkylaminogruppe;
wobei Y¹ und Y² je unabhängig ausgewählt sind aus der Gruppe bestehend aus Sauerstoff, Schwefel und NH; und
wobei R³ ausgewählt ist aus der Gruppe bestehend aus H, Alkyl, Hydroxyalkyl, Alkoxy, einem Purin, einem Pyrimidin und Kombinationen davon.

46. Verfahren gemäß Anspruch 45, wobei das Purin ausgewählt ist aus der Gruppe bestehend aus Adenin, Guanin und Derivaten davon.

47. Verfahren nach Anspruch 45, wobei das Pyrimidin ausgewählt ist aus der Gruppe bestehend aus Thymin, Cytosin, 5-Methylcytosin, Uracil und Derivaten davon.

48. Verfahren gemäß Anspruch 35, wobei R¹ und R² je unabhängig voneinander wenigstens zwei Nucleotide umfassen, die eine Internucleotidbindung aufweisen, wobei die Internucleotid-Bindung ausgewählt ist aus der Gruppe bestehend aus Phosphodiester, Phosphotriester, Phosphorothioat, Methylphosphonat, Phosphoramidat (5'N-3'P und 5'P 3'-N) und Kombinationen davon.

49. Verfahren nach Anspruch 45, wobei R¹ und R² je unabhängig voneinander ANA umfassen.

50. Verfahren gemäß Anspruch 49, wobei ANA einen 2'-Substiuenten umfasst, ausgewählt aus der Gruppe bestehend aus Fluor-, Hydroxyl-, Amino-, Azido-, Alkyl-, Alkenyl-, Alkynyl- und Alkoxygruppen.

51. Verfahren gemäß Anspruch 50, wobei der 2'-Substituent Fluor ist und ANA ist FANA.

52. Verfahren gemäß Anspruch 50, wobei die Alkylgruppe ausgewählt ist aus der Gruppe bestehend aus Methyl-, Ethyl-, propyl- und Butylgruppen.

53. Verfahren gemäß Anspruch 50, wobei die Alkoxygruppe ausgewählt aus der Gruppe bestehend aus Methoxy-, Ethoxy-, Propoxy- und Methoxyethoxygruppen.

54. Verfahren gemäß Anspruch 45, wobei das Oligonucleotid ausgewählt ist aus der Gruppe bestehend aus: 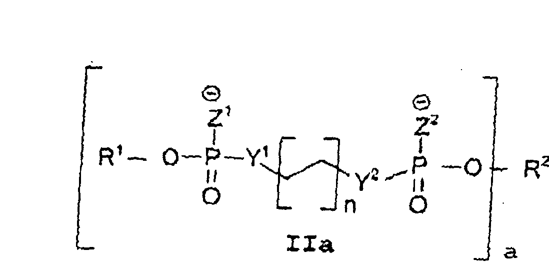 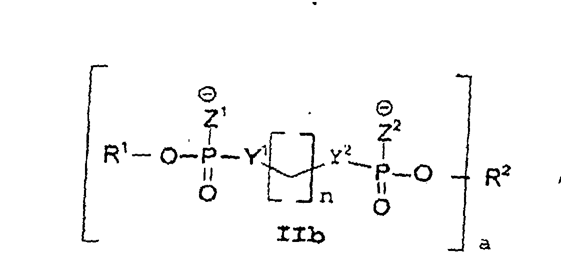 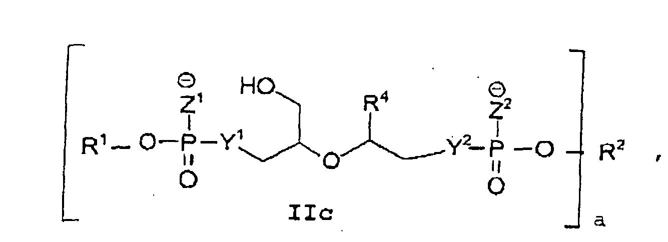 und 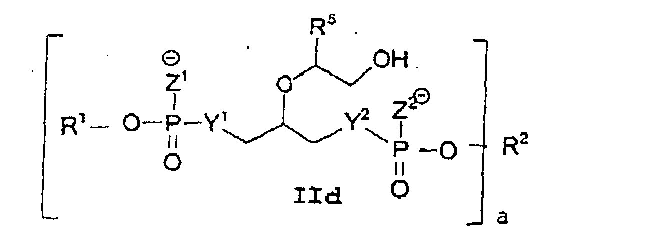 wobei n, a, R¹, R², Z¹, Z², Y¹ und Y² definiert sind wie in Anspruch 45 und
wobei R⁴ und R⁵ je unabhängig voneinander ausgewählt sind aus der Gruppe bestehend aus Purin und Pyrimidin.

55. Verfahren nach Anspruch 54, wobei das Purin ausgewählt ist aus der Gruppe bestehend aus Adenin, Guanin und Derivaten davon.

56. Verfahren nach Anspruch 54, wobei das Pyrimidin ausgewählt ist aus der Gruppe bestehend aus Thymin, Cytosin, Uracil und Derivaten davon.

57. Verfahren nach Anspruch 35, wobei R¹ und R² FANA sind und wobei a = 1 ist.

58. Verfahren nach Anspruch 35, wobei R¹ [FANA-DNA] ist, wobei R² [DNA-FANA] ist und wobei a = 1 ist.

59. Verfahren nach Anspruch 35, wobei R¹ [FANA-DNA] ist, R² FANA ist, und wobei a = 1 ist.

60. Verfahren nach Anspruch 35, wobei R¹ FANA ist, wobei R² [DNA-FANA] ist und wobei a = 1 ist.

61. Verfahren nach Anspruch 35, wobei R¹ [RNA-DNA] ist; R² [DNA-RNA] und wobei a = 1 ist.

62. Verfahren gemäß Anspruch 35, wobei R¹ [RNA-DNA] ist, wobei R² RNA ist und wobei a = 1 ist.

63. Verfahren gemäß Anspruch 35, wobei R¹ RNA ist, R² [DNA-RNA] ist und wobei a = 1 ist.

64. Verfahren gemäß Anspruch 35, wobei R¹ S-[(2'O-Alkyl)RNA-DNA] ist, wobei R² S-[DNA-(2'O-alkyl)RNA] ist und wobei a = 1 ist.

65. Verfahren gemäß Anspruch 35, wobei R¹ S-[(2'-O-Alkyl)RNA-DNA] ist, wobei R² S[(2'O-Alkyl)RNA] ist und wobei a = 1 ist

66. Verfahren gemäß Anspruch 35, wobei R¹ S-[2'O-Alkyl)RNA ist, ist R² S-[DNA-(2'-O-Alkyl)RNA] ist und wobei a = 1 ist.

67. Verfahren gemäß Anspruch 35, wobei R¹ S-[(2'-O-Alkoxyalkyl) RNA-DNA] ist, wobei R² S-[DNA-(2'O-Alkoxyalkyl)RNA] ist und wobei a = 1 ist.

68. Verfahren gemäß Anspruch 35, wobei R¹ S-[(2'O-Alkoxyalkyl)RNA-DNA] ist, wobei R² S-[(2'-O-Alkoxyalkyl)RNA] ist und wobei a = 1 ist.

69. Verfahren gemäß Anspruch 35, wobei R¹ S-[(2'-O-Alkoxyalkyl)RNA] ist, wobei R² S-[DNA-(2'-O-Alkoxyalkyl)RNA] ist und wobei a = 1 ist.

70. Verfahren gemäß Anspruch 54, wobei R¹ FANA ist, wobei R² PS-FANA ist, wobei a = 1 ist und wobei das Oligonucleotid die Struktur II b hat, in der Y¹, Y², Z¹ und Z² Sauerstoff sind und n = 4 ist.

71. Verfahren gemäß Anspruch 54, wobei R¹ PS-FANA ist; wobei R² FANA ist, wobei a = 1 ist und wobei das Oligonucleotid die Struktur II b aufweist, in der Y¹, Y², Z² Sauerstoff und Z¹ Schwefel sind und n = 4 ist.

72. Verfahren gemäß Anspruch 54, wobei R¹ PS-FANA ist; wobei R² FANA ist, wobei a = 1 ist und wobei das Oligonucleotid die Struktur IIc aufweist.

73. Verfahren gemäß Anspruch 54, wobei R¹ FANA ist, wobei R² PS-FANA ist; wobei a = 1 ist und wobei das Oligonucleotid die Struktur IIc aufweist.

74. Verfahren gemäß Anspruch 35, wobei a = 2 ist und R¹ und R² je unabhängig voneinander aus mindestens drei Nucleotiden bestehen.

75. Verfahren gemäß Anspruch 74, wobei R¹ und R² je unabhängig voneinander aus 3 bis 8 Nucleotiden bestehen.

76. Verfahren gemäß Anspruch 35, wobei a = 3 ist, und R¹ und R² je unabhängig voneinander aus wenigstens zwei Nucleotiden bestehen.

77. Verfahren gemäß Anspruch 76, wobei R¹ und R² je unabhängig voneinander aus 2 bis 6 Nucleotiden bestehen.

78. Verfahren gemäß Anspruch 35, wobei das Oligonucleotid antisense zu einer Target-RNA ist.

## Revendications

1. Oligonucléotide ayant la structure
[R¹-X]ₐ-R² Ia
dans laquelle
a est un entier supérieur ou égal à 1;
R¹, R² comprennent chacun indépendamment au moins un nucléotide;
X est un lieur acyclique; et
ledit oligonucléotide comprend au moins un élément parmi (i) au moins un nucléotide modifié et (ii) une chimère; et
le nucléotide modifié est choisi dans le groupe constitué par un ANA, un PS-ANA, et la chimère est choisie dans le groupe constitué par des chimères PS-[ANA-ADN] et PS-[ADN-ANA]; et
l'ANA est un FANA.

2. Oligonucléotide selon la revendication 1, dans lequel le FANA est choisi dans le groupe constitué par un PDE-FANA et un PS-FANA.

3. Oligonucléotide selon la revendication 1, dans lequel le lieur acyclique est choisi dans le groupe constitué par un nucléoside acyclique et un lieur non nucléotidique.

4. Oligonucléotide selon la revendication 3, dans lequel le nucléoside acyclique est choisi dans le groupe constitué par des séconucléosides puriques et pyrimidiques.

5. Oligonucléotide selon la revendication 4, dans lequel le séconucléoside purique est choisi dans le groupe constitué par une sécoadénosine et une sécoguanosine.

6. Oligonucléotide selon la revendication 4, dans lequel le séconucléoside pyrimidique est choisi dans le groupe constitué par une sécothymidine, une sécocytidine et une sécouridine.

7. Oligonucléotide selon la revendication 1, dans lequel le lieur non nucléotidique comprend un lieur choisi dans le groupe constitué par un aminoacide et un dérivé d'aminoacide.

8. Oligonucléotide selon la revendication 7, dans lequel le dérivé d'aminoacide est choisi dans le groupe constitué par (a) une unité N-(2-aminoéthyl)glycine dans laquelle une base hétérocyclique est liée par l'intermédiaire d'un lieur méthylènecarbonyle (monomère de PNA); et (b) une unité d'O-PNA.

9. Oligonucléotide selon la revendication 1, ledit oligonucléotide ayant la structure: 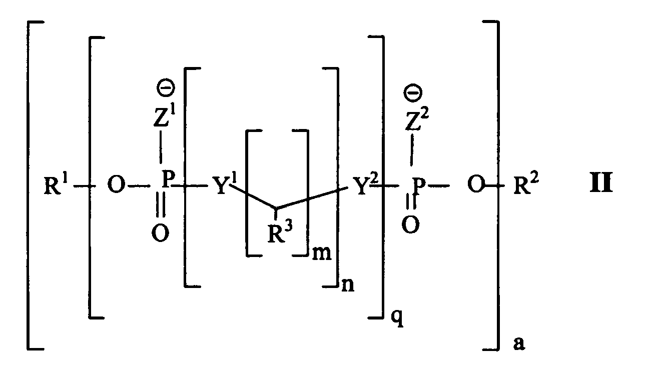 dans laquelle
m, n, q et a sont chacun indépendamment des entiers supérieurs ou égaux à 1;
R¹ et R² sont chacun indépendamment au moins un nucléotide;
Z¹ et Z² sont choisis chacun indépendamment dans le groupe constitué par un atome d'oxygène, un atome de soufre, un groupe amino et un groupe alkylamino;
Y¹ et Y² sont choisis chacun indépendamment dans le groupe constitué par l'oxygène, le soufre et NH; et
R³ est choisi dans le groupe constitué par H, alkyle, hydroxyalkyle, alcoxy, une purine, une pyrimidine et leurs combinaisons.

10. Oligonucléotide selon la revendication 9, dans lequel ladite purine est choisie dans le groupe constitué par l'adénine, la guanine et leurs dérivés.

11. Oligonucléotide selon la revendication 9, dans lequel ladite pyrimidine est choisie dans le groupe constitué par la thymine, la cytosine, la 5-méthylcytosine, l'uracile et leurs dérivés.

12. Oligonucléotide selon la revendication 1, dans lequel R¹ et R² comprennent chacun indépendamment au moins deux nucléotides ayant une liaison internucléotidique, ladite liaison internucléotidique étant choisie dans le groupe constitué par un phosphodiester, un phosphotriester, un phosphorothioate, un méthylphosphonate, un phosphoramidate (5'N-3'P et 5'P-3'N), et leurs combinaisons.

13. Oligonucléotide selon la revendication 9, dans lequel R¹ et R² comprennent chacun indépendamment un FANA.

14. Oligonucléotide selon la revendication 9, ledit oligonucléotide étant choisi dans le groupe constitué par: 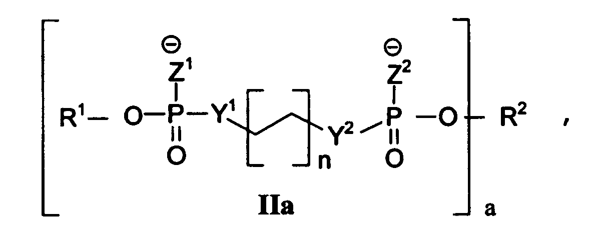 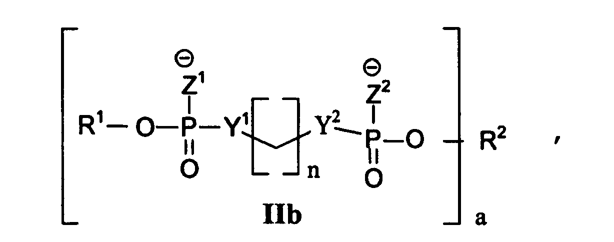 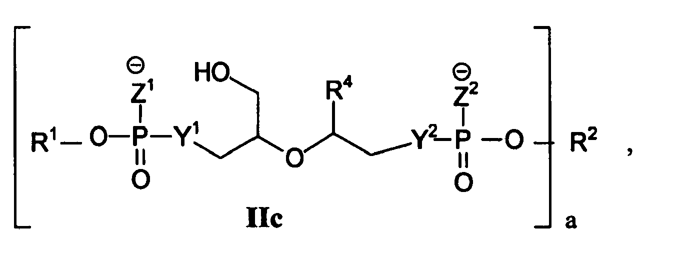 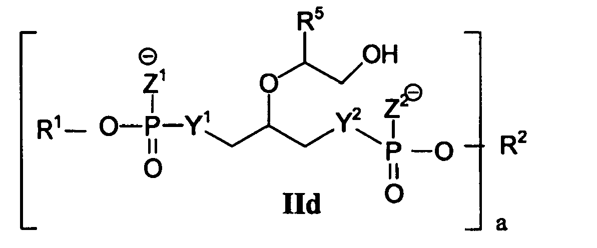 où n, a, R¹, R², Z¹, Z², Y¹ et Y² sont tels que définis dans la revendication 9; et
R⁴ et R⁵ sont choisis chacun indépendamment dans le groupe constitué par une purine et une pyrimidine.

15. Oligonucléotide selon la revendication 14, dans lequel ladite purine est choisie dans le groupe constitué par l'adénine, la guanine et leurs dérivés.

16. Oligonucléotide selon la revendication 14, dans lequel ladite pyrimidine est choisie dans le groupe constitué par la thymine, la cytosine, l'uracile et leurs dérivés.

17. Oligonucléotide selon la revendication 1, dans lequel R¹ et R² sont FANA; et a = 1.

18. Oligonucléotide selon la revendication 1, dans lequel R¹ est [FANA-ADN]; R² est [ADN-FANA]; et a = 1.

19. Oligonucléotide selon la revendication 1, dans lequel R¹ est [FANA-ADN]; R² est FANA; et a = 1.

20. Oligonucléotide selon la revendication 1, dans lequel R¹ est FANA; R² est [ADN-FANA]; et a = 1.

21. Oligonucléotide selon la revendication 14, dans lequel R¹ est FANA; R² est PS-FANA; a = 1; et ledit oligonucléotide a la structure IIb dans laquelle Y¹, Y², Z¹ et Z² sont oxygène et n = 4.

22. Oligonucléotide selon la revendication 14, dans lequel R¹ est PS-FANA; R² est FANA; a = 1; et ledit oligonucléotide a la structure IIb dans laquelle Y¹, Y², Z² sont oxygène, et Z¹ est soufre et n = 4.

23. Oligonucléotide selon la revendication 14, dans lequel R¹ est PS-FANA; R² est FANA; a = 1; et ledit oligonucléotide a la structure IIc.

24. Oligonucléotide selon la revendication 14, dans lequel R¹ est FANA; R² est PS-FANA; a = 1; et ledit oligonucléotide a la structure IIc.

25. Oligonucléotide selon la revendication 1, dans lequel a = 2 et R¹ et R² sont constitués chacun indépendamment d'au moins 3 nucléotides.

26. Oligonucléotide selon la revendication 25, dans lequel R¹ et R² sont constitués chacun indépendamment de 3-8 nucléotides.

27. Oligonucléotide selon la revendication 1, dans lequel a = 3 et R¹ et R² sont constitués chacun indépendamment d'au moins 2 nucléotides.

28. Oligonucléotide selon la revendication 27, dans lequel R¹ et R² sont constitués chacun indépendamment de 2-6 nucléotides.

29. Oligonucléotide selon l'une quelconque des revendications 1-28, ledit oligonucléotide étant antisens pour un ARN cible.

30. Procédé *in vitro* de prévention ou de diminution de la traduction, de la transcription inverse et/ou de la réplication d'un ARN cible dans un système, ledit procédé comprenant la mise en contact dudit ARN cible avec l'oligonucléotide de la revendication 29.

31. Procédé *in vitro* de prévention ou de diminution de la traduction, de la transcription inverse et/ou de la réplication d'un ARN cible dans un système, ledit procédé comprenant:
a) la mise en contact dudit ARN cible avec l'oligonucléotide de la revendication 29; et
b) la coupure par une RNase dudit ARN cible.

32. Utilisation *in vitro* de l'oligonucléotide selon la revendication 29 pour la prévention ou la diminution de la traduction, de la transcription inverse et/ou de la réplication d'un ARN cible dans un système.

33. Emballage commercial comprenant l'oligonucléotide selon la revendication 29 avec des instructions pour son utilisation en vue de la prévention ou la diminution de la traduction, de la transcription inverse et/ou de la réplication d'un ARN cible dans un système.

34. Oligonucléotide selon la revendication 29 à utiliser comme thérapeutique antisens.

35. Procédé pour augmenter l'activité d'induction de RNase H d'un oligonucléotide, comprenant l'incorporation d'un lieur acyclique dans ledit oligonucléotide pour produire un oligonucléotide ayant la structure
[R¹-X]ₐ-R² Ia
dans laquelle
a est un entier supérieur ou égal à 1;
R¹, R² comprennent chacun indépendamment au moins un nucléotide;
X est un lieur acyclique; et
ledit oligonucléotide comprend au moins un élément parmi (i) au moins un nucléotide modifié et (ii) une chimère; et
le nucléotide modifié est choisi dans le groupe constitué par un ANA, un PS-ANA, un ARN, un PS-ARN, des analogues de PDE- ou PS-ARN, des acides nucléiques verrouillés (LNA), des acides nucléiques morpholino phosphodiamidate, des ADN N3'-P5'-phosphoramidate, un acide nucléique cyclohexène, un α-L-LNA, un ADN boranophosphate, un ADN méthylphosphonate, et leurs combinaisons, et la chimère est choisie dans le groupe constitué par des chimères ARN-ADN et ADN-ARN, des chimères PS-[ARN-ADN] et PS-[ADN-ARN], des chimères PS-[ANA-ADN] et PS-[ADN-ANA].

36. Oligonucléotide selon la revendication 35, dans lequel l'ANA est un FANA.

37. Oligonucléotide selon la revendication 36, dans lequel le FANA est choisi dans le groupe constitué par un PDE-FANA et un PS-FANA.

38. Oligonucléotide selon la revendication 35, dans lequel les analogues de PDE- ou PS-ARN sont choisis dans le groupe constitué par des ARN modifiés en 2' dans lesquels le substituant en 2' est choisi dans le groupe constitué par alkyle, alcoxy, alkylalcoxy, F et leurs combinaisons.

39. Procédé selon la revendication 35, dans lequel le lieur acyclique est choisi dans le groupe constitué par un nucléoside acyclique et un lieur non nucléotidique.

40. Procédé selon la revendication 39, dans lequel le nucléoside acyclique est choisi dans le groupe constitué par des séconucléosides puriques et pyrimidiques.

41. Procédé selon la revendication 40, dans lequel le séconucléoside purique est choisi dans le groupe constitué par la sécoadénosine et la sécoguanosine.

42. Procédé selon la revendication 41, dans lequel le séconucléoside pyrimidique est choisi dans le groupe constitué par la sécothymidine, la sécocytidine et la sécouridine.

43. Procédé selon la revendication 35, dans lequel le lieur non nucléotidique comprend un lieur choisi dans le groupe constitué par un aminoacide et un dérivé d'aminoacide.

44. Procédé selon la revendication 43, dans lequel le dérivé d'aminoacide est choisi dans le groupe constitué par (a) une unité N-(2-aminoéthyl)glycine dans laquelle une base hétérocyclique est liée par l'intermédiaire d'un lieur méthylcarbonyle (monomère de PNA); et (b) une unité d'O-PNA.

45. Procédé selon la revendication 35, dans lequel l'oligonucléotide a la structure: 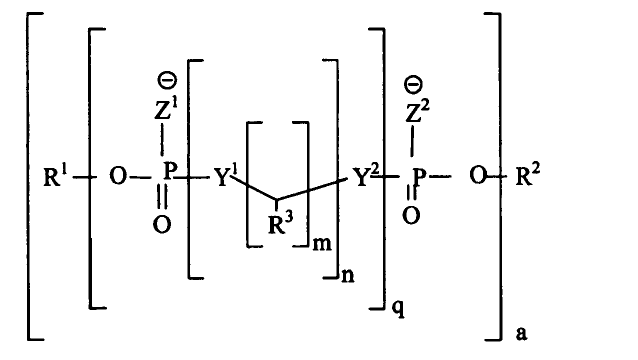 dans laquelle
m, n, q et a sont chacun indépendamment des entiers supérieurs ou égaux à 1;
R¹ et R² sont chacun indépendamment au moins un nucléotide;
Z¹ et Z² sont choisis chacun indépendamment dans le groupe constitué par un atome d'oxygène, un atome de soufre, un groupe amino et un groupe alkylamino;
Y¹ et Y² sont choisis chacun indépendamment dans le groupe constitué par l'oxygène, le soufre et NH; et
R³ est choisi dans le groupe constitué par H, alkyle, hydroxyalkyle, alcoxy, une purine, une pyrimidine et leurs combinaisons.

46. Procédé selon la revendication 45, dans lequel ladite purine est choisie dans le groupe constitué par l'adénine, la guanine et leurs dérivés.

47. Procédé selon la revendication 45, dans lequel ladite pyrimidine est choisie dans le groupe constitué par la thymine, la cytosine, la 5-méthylcytosine, l'uracile et leurs dérivés.

48. Procédé selon la revendication 35, dans lequel R¹ et R² comprennent chacun indépendamment au moins deux nucléotides ayant une liaison intemucléotidique, ladite liaison internucléotidique étant choisie dans le groupe constitué par un phosphodiester, un phosphotriester, un phosphorothioate, un méthylphosphonate, un phosphoramidate (5'N-3'P et 5'P-3'N), et leurs combinaisons.

49. Procédé selon la revendication 45, dans lequel R¹ et R² comprennent chacun indépendamment un ANA.

50. Procédé selon la revendication 49, dans lequel ledit ANA comprend un substituant en 2' choisi dans le groupe constitué par les groupes fluor, hydroxyle, amino, azido, alkyle, alcényle, alcynyle et alcoxy.

51. Procédé selon la revendication 50, dans lequel ledit substituant en 2' est le fluor et ledit ANA est un FANA.

52. Procédé selon la revendication 50, dans lequel ledit groupe alkyle est choisi dans le groupe constitué par les groupes méthyle, éthyle, propyle et butyle.

53. Procédé selon la revendication 50, dans lequel ledit groupe alcoxy est choisi dans le groupe constitué par les groupes méthoxy, éthoxy, propoxy et méthoxyéthoxy.

54. Procédé selon la revendication 45, dans lequel ledit oligonucléotide est choisi dans le groupe constitué par: 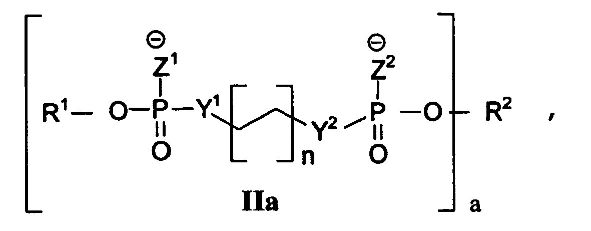 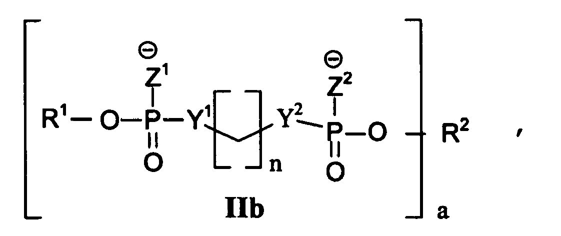 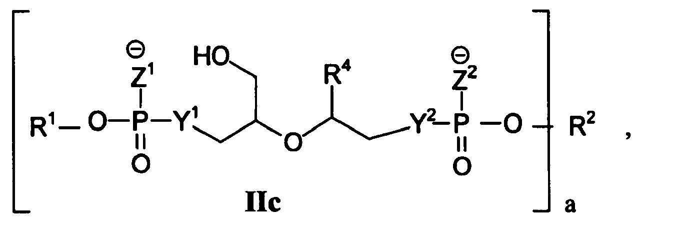 et 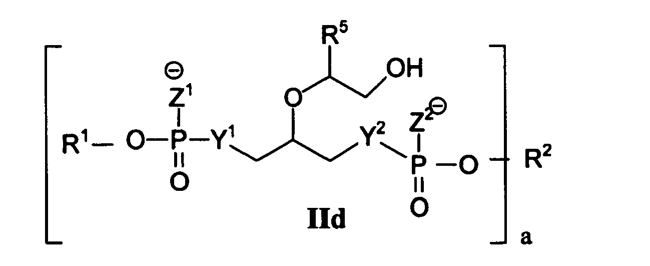 où n, a, R¹, R², Z¹, Z², Y¹ et Y² sont tels que définis dans la revendication 45; et
R⁴ et R⁵ sont choisis chacun indépendamment dans le groupe constitué par une purine et une pyrimidine.

55. Procédé selon la revendication 54, dans lequel ladite purine est choisie dans le groupe constitué par l'adénine, la guanine et leurs dérivés.

56. Procédé selon la revendication 54, dans lequel ladite pyrimidine est choisie dans le groupe constitué par la thymine, la cytosine, l'uracile et leurs dérivés.

57. Procédé selon la revendication 35, dans lequel R¹ et R² sont FANA; et a = 1.

58. Procédé selon la revendication 35, dans lequel R¹ est [FANA-ADN]; R² est [ADN-FANA]; et a = 1.

59. Procédé selon la revendication 35, dans lequel R¹ est [FANA-ADN]; R² est FANA; et a = 1.

60. Procédé selon la revendication 35, dans lequel R¹ est FANA; R² est [ADN-FANA]; et a = 1.

61. Procédé selon la revendication 35, dans lequel R¹ est [ARN-ADN]; R² est [ADN-ARN]; et a = 1.

62. Procédé selon la revendication 35, dans lequel R¹ est [ARN-ADN]; R² est ARN; et a = 1.

63. Procédé selon la revendication 35, dans lequel R¹ est ARN; R² est [ADN-ARN]; et a = 1.

64. Procédé selon la revendication 35, dans lequel R¹ est S-[(2'O-alkyl)ARN-ADN]; R² est S-[ADN-(2'O-alkyl)ARN]; et a = 1.

65. Procédé selon la revendication 35, dans lequel R¹ est S-[(2'O-alkyl)ARN-ADN]; R² est S-[(2'O-alkyl)ARN]; et a = 1.

66. Procédé selon la revendication 35, dans lequel R¹ est S-[(2'O-alkyl)ARN]; R² est S-[ADN-(2'O-alkyl)ARN]; et a = 1.

67. Procédé selon la revendication 35, dans lequel R¹ est S-[(2'O-alcoxyalkyl)ARN-ADN]; R² est S-[ADN-(2'O-alcoxyalkyl)ARN]; et a = 1.

68. Procédé selon la revendication 35, dans lequel R¹ est S-[(2'O-alcoxyalkyl)ARN-ADN]; R² est S-[(2'O-alcoxyalkyl)ARN]; et a = 1.

69. Procédé selon la revendication 35, dans lequel R¹ est S-[(2'O-alcoxyalkyl)ARN]; R² est S-[ADN-(2'O-alcoxyalkyl)ARN]; et a = 1.

70. Procédé selon la revendication 54, dans lequel R¹ est FANA; R² est PS-FANA; a = 1; et ledit oligonucléotide a la structure IIb dans laquelle Y¹, Y², Z¹ et Z² sont oxygène et n = 4.

71. Procédé selon la revendication 54, dans lequel R¹ est PS-FANA; R² est FANA; a = 1; et ledit oligonucléotide a la structure IIb dans laquelle Y¹, Y², Z² sont oxygène, et Z¹ est soufre et n = 4.

72. Procédé selon la revendication 54, dans lequel R¹ est PS-FANA; R² est FANA; a = 1; et ledit oligonucléotide a la structure IIc.

73. Procédé selon la revendication 54, dans lequel R¹ est FANA; R² est PS-FANA; a = 1; et ledit oligonucléotide a la structure IIc.

74. Procédé selon la revendication 35, dans lequel a = 2 et R¹ et R² sont constitués chacun indépendamment d'au moins 3 nucléotides.

75. Procédé selon la revendication 74, dans lequel R¹ et R² sont constitués chacun indépendamment de 3-8 nucléotides.

76. Procédé selon la revendication 35, dans lequel a = 3 et R¹ et R² sont constitués chacun indépendamment d'au moins 2 nucléotides.

77. Procédé selon la revendication 76, dans lequel R¹ et R² sont constitués chacun indépendamment de 2-6 nucléotides.

78. Procédé selon la revendication 35, dans lequel ledit oligonucléotide est antisens pour un ARN cible.


### SEQUENCE LISTING

<110> MCGILL UNIVERSITY
<120> ACYCLIC LINKER-CONTAINING OLIGONUCLEOTIDES AND USES THEREOF
<130> 85827-61
<150> US 60/330 719
   <151> 2001-10-29
<160> 31
<170> Patentin version 3.1
<210> 1
   <211> 18
   <212> DNA
   <213> Artificial
<220>
   <223> Oligonucleotide
<400> 1
   tttttttttt tttttttt 18
<210> 2
   <211> 17
   <212> DNA
   <213> Artificial
<220>
   <223> oligonucleotide
<220>
   <221> misc_feature
   <222> (9)..(10)
   <223> Residues 9 and 10 are joined by a butanediol linker
<400> 2
   tttttttttt ttttttt 17
<210> 3
   <211> 18
   <212> DNA
   <213> Artificial
<220>
   <223> oligonucleotide
<400> 3
   ttatattttt tctttccc 18
<210> 4
   <211> 17
   <212> DNA
   <213> Artificial
<220>
   <223> oligonucleotide
<220>
   <221> misc_feature
   <222> (9)..(10)
   <223> Residues 9 and 10 are joined by a butanediol linker
<400> 4
   ttatattttt ctttccc 17
<210> 5
   <211> 18
   <212> DNA
   <213> Artificial
<220>
   <223> oligonucleotide
<400> 5
   ttatattttc tctttccc 18
<210> 6
   <211> 18
   <212> DNA
   <213> Artificial
<220>
   <223> oligonucleotide
<220>
   <221> misc_feature
   <222> (9)..(10)
   <223> Residues 9 and 10 are joined by a butanediol linker
<400> 6
   ttatattttt tctttccc 18
<210> 7
   <211> 18
   <212> DNA
   <213> Artificial
<220>
   <223> oligonucleotide
<220>
   <221> misc_feature
   <222> (1)..(18)
   <223> Residues 1 to 18 are 2'-deoxy-2'-fluoroarabinothymidine
<400> 7
   nnnnnnnnnn nnnnnnnn 18
<210> 8
   <211> 17
   <212> DNA
   <213> Artificial
<220>
   <223> oligonucleotide
<220>
   <221> misc_feature
   <222> (1) .. (17)
   <223> Residues 1 to 17 are 2'-deoxy-2'-fluoroarabinothymidine
<220>
   <221> misc_feature
   <222> (4)..(5)
   <223> Residues 4 and 5 are joined by a butanediol linker
<400> 8
   nnnnnnnnnn nnnnnnn 17
<210> 9
   <211> 17
   <212> DNA
   <213> Artificial
<220>
   <223> oligonucleotide
<220>
   <221> misc_feature
   <222> (1)..(17)
   <223> Residues 1 to 17 are 2'-deoxy-2'-fluoroarabinothymidine
<220>
   <221> misc_feature
   <222> (9)..(10)
   <223> Residues 9 and 10 are joined by a butanediol linker
<400> 9
   nnnnnnnnnn nnnnnnn 17
<210> 10
   <211> 17
   <212> DNA
   <213> Artificial
<220>
   <223> oligonucleotide
<220>
   <221> misc_feature
   <222> (1)..(17)
   <223> Residues 1 to 17 are 2'-deoxy-2'-fluoroarabinothymidine
<220>
   <221> misc_feature
   <222> (12)..(13)
   <223> Residues 12 and 13 are joined by a butanediol linker
<400> 10
   nnnnnnnnnn nnnnnnn 17
<210> 11
   <211> 17
   <212> DNA
   <213> Artificial
<220>
   <223> Oligonucleotide
<220>
   <221> misc_feature
   <222> (1)..(17)
   <223> Residues 1 to 17 are 2'-deoxy-2'-fluoroarabinothymidine
<220>
   <221> misc_feature
   <222> (9)..(10)
   <223> Residues 9 and 10 are joined by a secouridine linker
<400> 11
   nnnnnnnnnn nnnnnnn 17
<210> 12
   <211> 16
   <212> DNA
   <213> Artificial
<220>
   <223> oligonucleotide
<220>
   <221> misc_feature
   <222> (1)..(16)
   <223> Residues 1 to 16 are 2'-deoxy-2'-fluoroarabinothymidine
<220>
   <221> misc_feature
   <222> (8)..(9)
   <223> Residues 8 and 9 are joined by a linker of 2 secouridine units
<400> 12
   nnnnnnnnnn nnnnnn 16
<210> 13
   <211> 18
   <212> DNA
   <213> Artificial
<220>
   <223> oligonucleotide
<220>
   <221> misc_feature
   <222> (1)..(18)
   <223> Residues 1, 2, 4, 6-11 and 13-15 are 2'-deoxy-2'-fluoroarabinothymidine; residues 3 and 5 are 2'-deoxy-2'-fluoroarabinoadenosine; residues 12 and 16-18 are 2'-deoxy-2'-fluoroarabinocytidine
<400> 13
   nnnnnnnnnn nnnnnnnn 18
<210> 14
   <211> 17
   <212> DNA
   <213> Artificial
<220>
   <223> oligonucleotide
<220>
   <221> misc_feature
   <222> (1)..(17)
   <223> Residues 1, 2, 4, 6-9, 11 and 13-15 are 2'-deoxy-2'-fluoroarabinothymidine; residues 3 and 5 are 2'-deoxy-2'-fluoroarabinoadenosine; residues 11 and 15-17 are 2'-deoxy-2'-fluoroarabinacyxidine
<220>
   <221> misc_feature
   <222> (9)..(10)
   <223> Residues 9 and 10 are joined by a butanediol linker
<400> 14
   nnnnnnnnnn nnnnnnn 17
<210> 15
   <211> 18
   <212> DNA
   <213> Artificial
<220>
   <223> oligonucleotide
<220>
   <221> misc_feature
   <222> (1)..(18)
   <223> Residues 1, 2, 4, 6-9, 11 and 13-15 are 2'-deoxy-2'-fluoroarabinothymidine; residues 3 and 5 are 2'-deoxy-2'-fluoroarabinoadenosine; residues 10, 12 and 16-18 are 2'-deoxy-2'-fluoroarabinocytidine
<400> 15
   nnnnnnnnnn nnnnnnnn 18
<210> 16
   <211> 18
   <212> DNA
   <213> Artificial
<220>
   <223> oligonucleotide
<220>
   <221> misc_feature
   <222> (1)..(9)
   <223> Residues 1, 2, 4 and 6-9 are 2'-deoxy-2'-fluoroarabinothymidine; residues 3 and 5 are 2'-deoxy-2'-fluoroarabinoadenosine
<220>
   <221> misc_feature
   <222> (11)..(18)
   <223> Residues 11 and 13-15 are 2'-deoxy-2'-fluoroarabinothymidine; residues 12 at 16-18 are 2'-deoxy-2'-fluoroarabinocytidine
<400> 16
   nnnnnnnnnt nnnnnnnn 18
<210> 17
   <211> 1.8
   <212> DNA
   <213> Artificial
<220>
   <223> oligonucleotide
<220>
   <221> misc_feature
   <222> (1)..(18)
   <223> Residues 1, 2, 4, 6-11 and 13-15 are 2'-deoxy-2'-fluoroarabinothymidine; residues 3 and 5 are 2'-deoxy-2'-fluoroarabinoadenosine; residues 12 and 16-18 are 2'-deoxy-2'-fluoraarabinocytidine
<220>
   <221> misc_feature
   <222> (9)..(10)
   <223> Residues 9 and 10 are joined by a butanediol linker
<400> 17
   nnnnnnnnnn nnnnnnnn 18
<210> 18
   <211> 18
   <212> DNA
   <213> Artificial
<220>
   <223> Oligonucleotide
<400> 18 1 8
   attccgtcat cgctcctc
<210> 19
   <211> 17
   <212> DNA
   <213> Artificial
<220>
   <223> oligonucleotide
<220>
   <221> misc_feature
   <222> (9)..(10)
   <223> Residues 9 and 10 are joined by a butanediol linker
<400> 19
   attccgtcac gctcctc 17
<210> 20
   <211> 18
   <212> DNA
   <213> Artificial
<220>
   <223> oligonucleotide
   <400> 20
   attccgtcac cgctcctc 18
<210> 21
   <211> 18
   <212> DNA
   <213> Artificial
<220>
   <223> Oligonucleotide
<220>
   <221> misc_feature
   <222> (1)..(18)
   <223> Residues 2, 3, 7, 10, 14 and 17 are 2'-deoxy-2'-fluoroarabinothymidine; residues 1 and 9 are 2'-deoxy-2'-f1uoroarabinoadenosine; residues 3, 4, 8, 11, 13, 15 16 and 18 are 2'-deoxy-2'-fluoroarabinocytidine; residues 6 and 12 are 2'-deoxy-2'-fluoroarabinoguanosine
<400> 21
   nnnnnnnnnn nnnnnnnn 18
<210> 22
   <211> 17
   <212> DNA
   <213> Artificial
<220>
   <223> Oligonucleotide
<220>
   <221> misc_feature
   <222> (1)..(17)
   <223> Residues 2, 3, 7, 13 and 16 are 2'-deoxy-2'-fluoroarabinothymidine; residues 1 and 9 are 2'-deoxy-2'-fluoroarabinaadenosine; residues 3, 4, 8, 10, 12, 14, 15 and 17 are 2'-deoxy-2'-fluoroarabinocytidine: residues 6 and 11 are 2'-deoxy-2'-fluoroarabinoguanosine
<220>
   <221> misc_feature
   <222> (9) .. (10)
   <223> Residues 9 and 10 are joined by a butanediol linker
<400> 22
   nnnnnnnnnn nnnnnnn 17
<210> 23
   <211> 20
   <212> DNA
   <213> Artificial
<220>
   <223> Oligonucleotide
<220>
   <221> misc_feature
   <222> (1)..(20)
   <223> Residues 1-20 are linked by phosphorothioate linkages
<400> 23
   tattccgtca tcgctcctca 20
<210> 24
   <211> 19
   <212> DNA
   <213> Artificial
<220>
   <223> oligonucleotide
<220>
   <221> misc_feature
   <222> (12)..(13)
   <223> Residues 12 and 13 are joined by a butanediol linker
<220>
   <221> misc_feature
   <222> (1)..(19)
   <223> Residues 1-19 are linked by phosphorothioate linkages
<400> 24
   tattccgtca tcctcctca 19
<210> 25
   <211> 20
   <212> DNA
   <213> Artificial
<220>
   <223> oligonucleotide
<220>
   <221> misc_feature
   <222> (1)..(20)
   <223> Residues 1, 3, 4, 8, 11, 15 and 18 are 2'-deoxy-2'-fluoroarabinothymidine; residues 2, 10 and 20 are 2'-deoxy-2'-fluoroarabinoadenosine; residues 5, 6, 9, 12, 14, 16, 17 and 19 are 2'-deoxy-2'-fluoroarabinocytidine; residues 7 and 13 are 2'-deoxy-2'-fluoroarabinoguanosine
<220>
   <221> misc_feature
   <222> (1)..(20)
   <223> Residues 1-20 are linked by phosphorothioate linkages
<400> 25
   nnnnnnnnnn nnnnnnnnnn 20
<210> 26
   <211> 19
   <212> DNA
   <213> Artificial
<220>
   <223> oligonucleotide
<220>
   <221> misc_feature
   <222> (1)..(19)
   <223> Residues 1, 3, 4, 8, 11, 14 and 17 are 2'-deoxy-2'-fluoroarabinothymidine; residues 2, 10 and 19 are 2'-deoxy-2'-fluoroarabinoadenosine; residues 5, 6, 9, 12, 13, 15, 16 and 18 are 2'-deoxy-2'-fluoroarabinocytidine; residue 7 is 2'-deoxy-2'-fluoroarabinoguanosine
<220>
   <221> misc_feature
   <222> (12)..(13)
   <223> Residues 12 and 13 are joined by a butanediol linker
<220>
   <221> misc_feature
   <222> (1)..(19)
   <223> Residues 1-19 are linked by phosphorothioate linkages
<400> 26
   nnnnnnnnnn nnnnnnnnn 19
<210> 27
   <211> 18
   <212> RNA
   <213> Artificial
<220>
   <223> Target RNA oligonucleotide
<400> 27
   aaaaaaaaaa aaaaaaaa 18
<210> 28
   <211> 18
   <212> RNA
   <213> Homo sapiens
<400> 28
   gggaaagaaa aaauauaa 18
<210> 29
   <211> 40
   <212> RNA
   <213> Homo sapiens
<220>
   <221> misc_binding
   <222> (12) .. (29)
   <223> AON binding region 1
<220>
   <221> misc_binding
   <222> (11)..(30)
   <223> AON binding region 2
<400> 29
   cgcaggcccc ugaggagcga ugacggaaua uaagcuggug 40
<210> 30
   <211> 36
   <212> DNA
   <213> Artificial
<220>
   <223> oligonucleotide
<400> 30
   agctatctcg agatgagctg gcttctgttc ctggcc 36
<210> 31
   <211> 36
   <212> DNA
   <213> Artificial
<220>
   <223> Oligonucleotide
<400> 31
   ggccgcaagc tttcagtctt ccgattgttt agctcc 36